# EUROPEAN PATENT SPECIFICATION

(11) **EP 4 618 872 B1**
(45) Date of publication and mention of the grant of the patent: **01.04.2026**
(21) Application number: 23813842.4
(22) Date of filing: 17.11.2023
(51) Int. Cl.: A61K 48/00, C12N 15/86, A61M 25/10

(54) **METHODS OF DELIVERING A VIRAL VECTOR TO A KIDNEY**
VERFAHREN ZUR VERABREICHUNG EINES VIRALEN VEKTORS IN EINE NIERE
MÉTHODES D'ADMINISTRATION D'UN VECTEUR VIRAL DANS UN REIN

(30) Priority: 18.11.2022 GB 202217332
(43) Date of publication of application: 24.09.2025
(60) Divisional application: 25224936.2
(73) Proprietor: The University of Bristol, Bristol BS8 1QU (GB); Purespring Therapeutics Limited, London WC1B 3SR (GB)
(72) Inventor: SALEEM-UDDIN, Moin, Bristol Beacon House Queens Road Bristol BS8 1QU (GB); WELSH, Gavin, Bristol Beacon House Queens Road Bristol BS8 1QU (GB); GRIFFITH, Alan William, London WC1B 3SR (GB)
(74) Representative: Stratagem IPM Limited
(86) International application number: PCT/GB2023/053027
(87) International publication number: WO 2024/105415

(56) References cited:
- WO-A1-2021/176220
- WO-A1-2022/003357
- WO-A1-2022/175546
- WO-A1-2022/189811
- US-A1- 2002 086 846
- US-A1- 2003 170 897
- DAHLQVIST ULRIKA ET AL: "Direct Drug Delivery to Kidney via the Renal Artery", JOURNAL OF VISUALIZED EXPERIMENTS, vol. 170, 17 April 2021 (2021-04-17), United States, XP093215290, ISSN: 1940-087X, DOI: 10.3791/61932
- ZAW THIN MAY ET AL: "Stem cell delivery to kidney via minimally invasive ultrasound-guided renal artery injection in mice", SCIENTIFIC REPORTS, vol. 10, no. 1, 5 May 2020 (2020-05-05), US, XP093215289, ISSN: 2045-2322, DOI: 10.1038/s41598-020-64417-2

## Description

### FIELD OF THE INVENTION

The present invention relates to a method of delivering a viral vector to a kidney. The present invention also relates to a viral vector for use in therapy, wherein the viral vector is delivered by said method.

### BACKGROUND TO THE INVENTION

There are many diseases which affect kidney function by attacking the glomerulus. The glomerulus filters approximately 180 litres of plasma each day, and the healthy glomerular filtration barrier has an astonishing ability to retain about 99.9% of large proteins including albumin over our lifetimes without clogging. The glomerular filtration barrier (GFB) comprises 3 main layers: the glomerular endothelial cell, the glomerular basement membrane (GBM) and the podocyte.

The GBM is made of a highly crosslinked macromolecular meshwork of type IV collagen, proteoglycans, and laminin. Genetic forms of glomerular disease can be caused by genetic defects in these molecular structures. For example, Alport syndrome is caused by pathogenic variants in the COL4A3, COL4A4 and COL4A5 genes, which result in abnormalities of the collagen IV α345 network of basement membranes. Alport syndrome affects approximately 1 in 5,000-10,000 of all individuals in continental Europe and the USA. The condition usually presents during childhood and is associated with a spectrum of phenotypes that include a progressive loss of kidney function, and can also include hearing loss and eye abnormalities. Other GBM-associated diseases include Pierson syndrome and Nail-patella syndrome (Chiang, C.K. and Inagi, R., 2010. Nature Reviews Nephrology, 6(9), p.539).

The podocyte has also been implicated as a key cell in the progression of glomerular disease. Podocytes are mesodermally derived cells that are highly specialized and found only in the renal glomerulus. They exhibit unique characteristics such as foot processes and slit diaphragms, which are critical for glomerular filtration. Podocyte-associated genetic glomerular diseases include Nephrotic Syndrome, Frasier syndrome and Denys-Drash syndrome, Schimke immuno-osseous dysplasia, and Epstein and Fechtner syndrome. (Chiang, C.K. and Inagi, R., 2010. Nature Reviews Nephrology, 6(9), p.539).

Accordingly, glomerular cells, such as podocytes, represent a potential target for gene therapy approaches.

In order to maximise gene therapy potential, optimal methods to delivery viral vectors to glomerular cells, such as podocytes, are required. However, the kidney is a difficult target for conventional intravenous delivery, since the permselectivity of the glomerulus will prevent entry of most molecular therapies from the blood into the kidney. Moreover, while various direct kidney injection methods have been tested with viral vector, such as adeno-associated viral (AAV) vectors, the efficiency of gene delivery is inconsistent and typically still too small to effectively treat disease (see e.g. Rubin, J.D. and Barry, M.A., 2020. Molecular diagnosis & therapy, 24(4), pp.375-396).

WO 2022/175546 discloses a method of perfusing a kidney of a patient comprising introducing percutaneously a perfusion catheter in the renal artery of the kidney for loco-regional perfusing of drugs, such as a therapeutic polynucleotide sequence present in a viral vector (such as AAV). The method comprises a step of positioning a recovery catheter in the renal vein of the kidney, which is necessary to form a closed perfusion circuit through the kidney.

Thus, there is a demand for improved methods to deliver viral vectors to glomerular cells, such as podocytes.

### SUMMARY OF THE INVENTION

The present inventors have developed an improved method of delivering viral vectors to the kidney, in particular glomerular cells, such as podocytes, via direct renal artery injection. The method can be performed in a minimally invasive manner and with improved safety compared to prior art direct kidney injection methods.

Further, the present inventors have surprisingly shown that direct renal artery injection of a viral vector can result in a marked increase in transgene expression and viral copy number in the kidney, in particular glomeruli and podocytes, when compared to intravenous injection. Moreover, the direct renal artery injection of a viral vector can result in transgene expression that is localised to the kidney with minimal expression in other tissues (e.g. the liver).

The present invention provides a method of delivering a viral vector to a kidney, the method comprising inserting a catheter into the renal artery and delivering the viral vector into the renal artery via the catheter.

The method is a minimally invasive procedure. Suitably, the method does not comprise a step of occluding the renal vein. The method does not comprise a step of inserting a catheter into the renal vein . Suitably, the method does not comprise a step of inserting a catheter directly into the aorta. Suitably, the method does not comprise forming a closed circuit through the kidney. The method does not comprise a step of clamping the renal artery, the renal vein, or the aorta. Suitably, the total kidney ischemia time is about 60 minutes or less, about 50 minutes or less, about 40 minutes or less, about 30 minutes or less, about 10 minutes to about 30 minutes, about 15 to about 25 minutes, or about 5 minutes.

The catheter may be inserted into the renal artery by any suitable method. In preferred embodiments, the catheter is inserted into the renal artery via a percutaneous route. Suitably, the percutaneous route is via the carotid artery or via the femoral artery. Suitably, insertion of the catheter via the percutaneous route is facilitated with a sheath. Suitably, the catheter is inserted into the renal artery via guidewire.

The viral vector may be delivered into the renal artery by any suitable method. Suitably, the viral vector is injected or infused into the renal artery. In some embodiments, the viral vector is infused into the renal artery under no flow conditions using an infusion pump. Suitably, the viral vector is delivered into the renal artery over about 1 minute to about 30 minutes. In some embodiments, the viral vector is delivered into the renal artery over about 1 minute to about 5 minutes, optionally about 2 minutes or about 4 minutes. In some embodiments, the viral vector is delivered into the renal artery over about 15 minutes to about 30 minutes or about 15 minutes to about 20 minutes, optionally about 17 minutes.

In some embodiments, the catheter is an occlusion balloon catheter. In some embodiments, the method comprises a step of inflating a balloon to occlude the renal artery. Suitably, the renal artery is occluded for from about 1 minute to about 25 minutes. In some embodiments, the renal artery is occluded for from about 2 minutes to about 10 minutes, optionally about 5 minutes. In some embodiments, the renal artery is occluded for from about 15 minutes to about 25 minutes, or from about 15 minutes to about 20 minutes, optionally about 20 minutes.

The present invention provides a viral vector for use in a method of treating a human subject that has or is at risk of a kidney disease, the method comprising:
(a) inserting a catheter into the renal artery of the kidney;
(b) optionally inflating a balloon to occlude the renal artery; and
(c) injecting or infusing the viral vector into the renal artery via the catheter,
wherein the method is a minimally invasive procedure, wherein the method does not comprise a step of clamping the renal artery of the kidney, the renal vein of the kidney, or the aorta, and wherein the method does not comprise a step of inserting a catheter into the renal vein of the kidney.

In one embodiment, the present invention provides a method of delivering a viral vector to a subject's kidney, the method comprising: inserting a catheter into the renal artery of the kidney and injecting or infusing the viral vector into the renal artery via the catheter, wherein the method does not comprise a step of occluding the renal artery of the kidney or the renal vein of the kidney, and wherein the method does not comprise a step of clamping the aorta.

The method of the present invention may result in delivery of the viral vector to the kidney. For example, the method may result in delivery of the viral vector to the kidney cortex and/or kidney medulla, in particular the kidney cortex. The method may result in delivery of the viral vector to the kidney glomeruli. The method may result in delivery of the viral vector to the kidney podocytes. The method may result in kidney-specific delivery of the viral vector.

The subject is a human subject. The human subject may be an adult, an adolescent, or a child. The subject has or is at risk of a kidney disease. The subject may have or may be at risk of a glomerular disease. The subject may have or may be at risk of a genetic glomerular disease, optionally wherein the subject has or is at risk of a podocyte-associated genetic glomerular disease.

The viral vector may be delivered in any suitable dose. Suitably, the viral vector is delivered in a dose of from about 1x10⁶ vg/kg to about 1x10¹⁴ vg/kg, or from about 1x10⁶ vg/kg to about 1x10¹³ vg/kg. In some embodiments, the viral vector is delivered in a dose of from about 1x10⁹ vg/kg to about 1x10¹² vg/kg. In some embodiments, the viral vector is delivered in a dose of from about 3x10⁹ vg/kg to about 3x10¹¹ vg/kg. Suitably, the viral vector is delivered in a dose of from about 1x10⁶ vg to about 1x10¹⁵ vg, or from about 1x10⁶ vg to about 5x10¹⁴ vg. In some embodiments, the viral vector is delivered in a dose of from about 1x10¹¹ vg to about 1x10¹⁴ vg. In some embodiments, the viral vector is delivered in a dose of from about 2x10¹¹ vg to about 2x10¹³ vg. In some embodiments, the viral vector is delivered in a dose of from about 5x10¹¹ vg to about 2x10¹³ vg. In some embodiments, the viral vector is delivered in a dose of from about 1x10¹² vg to about 2x10¹³ vg. In some embodiments, the viral vector is delivered in a dose of about 1x10¹³ vg.

The viral vector may be any suitable viral vector. Suitably, the viral vector is capable of transducing kidney cells, optionally wherein the vector is capable of specifically transducing kidney cells. Suitably, the viral vector is capable of transducing glomerular cells, optionally wherein the vector is capable of specifically transducing glomerular cells. Suitably, the viral vector is capable of transducing podocytes, optionally wherein the vector is capable of specifically transducing glomerular podocytes. Suitably the viral vector is selected from an adeno-associated virus (AAV) vector, a lentiviral vector, a retroviral vector, an adenoviral vector, a herpes simplex viral vector, an alphaviral vector, a flaviviral vector, a rhabdoviral vector, a measles viral vector, a Newcastle disease viral vector, a poxviral vector, and a picornaviral vector.

In preferred embodiments, the viral vector is an adeno-associated virus (AAV) vector particle. In some embodiments, the viral vector is the form of an AAV vector particle encapsidated by LK03, AAV3B, or AAV9 capsid proteins. In some embodiments, the viral vector is the form of an AAV vector particle encapsidated by LK03 capsid proteins.

The viral vector may comprise any suitable protein-coding sequence. Suitably, the protein-coding sequence encodes a therapeutic protein, preferably wherein the protein-coding sequence encodes a polypeptide associated with a genetic glomerular disease, optionally a polypeptide involved in podocyte-associated genetic glomerular disease. In some embodiments, the protein-coding sequence encodes a COL4A3, COL4A4, COL4A5, NPHS2, CFH, CFL, FHL-1, C1INH, C4BP, MASP2, C3, C5aR1, C5, C5a, CD55, CD35, CD46, CD59, vitronectin, clusterin, ADCK4, ALG1, ARHGAP24, ARGHDIA, CD151, CD2AP, COQ2, COQ6, DGKE, E2F3, EMP2, KANK2, LAGE3, LMNA, LMX1B, MAF B, NUP85, NUP93, NXF5, OSGEP, PAX2, PDSS2, PMM2, PODXL, SCARB2, SGPL1, Smad7, TP53RK, TPRKB, VDR, WDR73, WT1, ZMPSTE24, APOL1, NPHS1, TRPC6, NUP107, NUP133, NUP160, ACTN4, INF2, ANKFY1, ANLN, CRB2, ITGA3, KANK1, KANK4, MAGl2, **MYO1E,** OCRL, PTPRO, SMARCAL1, SYNPO, TBC1D8B, XPO5, TNS2, NLRP3, or VEGFC polypeptide. In some embodiments, the protein-coding sequence encodes NPHS2 or a fragment and/or variant thereof; a COL4A3, COL4A4 or COL4A5 polypeptide, or a fragment or derivative thereof; or CFI, CFH or FHL-1, or a fragment and/or variant thereof. In some embodiments, the protein-coding sequence does not encode a gene-editing agent. In some embodiments, the protein-coding sequence does not encode a nuclease. In some embodiments, the protein-coding sequence does not encode a Cas9.

The protein-coding sequence may be operably linked to any promoter. In some embodiments, the protein-coding sequence is operably linked to a kidney-specific promoter. In some embodiments, the protein-coding sequence is operably linked to a podocyte-specific promoter. In some embodiments, the protein-coding sequence is operably linked to a NPHS1 promoter or a NPHS2 promoter. In some embodiments, the protein-coding sequence is operably linked to a minimal NPHS1 promoter. In some embodiments, the protein-coding sequence is operably linked to a constitutive promoter. In some embodiments, the protein-coding sequence is operably linked to a CMV promoter.

The protein-coding sequence may be operably linked to one or more further regulatory elements. Suitably, the protein-coding sequence is operably linked to a post-transcriptional regulatory element and/or a polyadenylation sequence. In some embodiments, the protein-coding sequence is operably linked to a Woodchuck hepatitis post-transcriptional regulatory element (WPRE). In some embodiments, the protein-coding sequence is operably linked a polyadenylation signal, for example a bovine growth hormone polyadenylation signal.

The viral vector may be in the form of a viral vector formulation. The viral vector formulation may comprise the viral vector in any suitable amount and may be formulated in any suitable manner. Suitably, the viral vector formulation comprises the viral vector in an amount of from about 1x10⁷ vg/ml to about 1x10¹⁴ vg/ml, or from about 1x10⁷ vg/ml to about 5x10¹³ vg/ml. In some embodiments, the viral vector formulation comprises the viral vector in an amount of from about 1x10¹⁰ vg/ml to about 1x10¹³ vg/ml. In some embodiments, the viral vector formulation comprises the viral vector in an amount of from about 1x10¹⁰ vg/ml to about 1x10¹² vg/ml. Suitably, the viral vector formulation comprises an isotonic buffer, such as phosphate buffered saline (PBS) buffer or plasmalyte. In some embodiments, the viral vector formulation comprises about 0.001% poloxamer 188. Suitably, the viral vector formulation has a volume of from about 5 ml to about 50 ml, from about 5 ml to about 25 ml, or from about 10 ml to about 25 ml.

The viral vector may be delivered to a single kidney or both kidneys of the subject. In some embodiments, the method of the present invention is carried out once to deliver the viral vector to a single kidney of the subject. In some embodiments, the method of the present invention is carried out twice to deliver the viral vector to both kidneys of the subject.

### DESCRIPTION OF DRAWINGS

Figure 1 - A schematic representation of an AAV vector encoding podocin under control of a hNPHS1 promoter
   ITR: inverted terminal repeat; hNPHS1 promoter: human full-length nephrin promoter; HA: haemagglutinin; WPRE: woodchuck post-transcriptional regulatory element; bGH: bovine growth hormone polyA signal.
**Figure 2** **- Transcriptomic Analysis to Assess Transduction Efficiency**
   Pigs 3-6 were the dRAi treated pigs. Pig 1 and Pig 2 were controls. AAV: adeno-associated virus; bGH: bovine growth hormone; LKC: left kidney cortex; LKM: left kidney medulla; RKC: right kidney cortex; RKM: right kidney medulla.
**Figure 3** **- Colocalization Studies of Nephrin and HA-tagged Podocin (LKC & RKC)**
   **(A)** Single channel images showing nephrin (left panel) and HA-tagged podocin (middle panel), and corresponding composite image (right panel) showing colocalization of nephrin and HA-tagged podocin. **(B)** Magnified panel from indicated region demonstrated the expression of HA-podocin in podocytes as seen by colocalization with nephrin, the podocyte marker. HA: haemagglutinin; LKC: left kidney cortex; RKC: right kidney cortex.

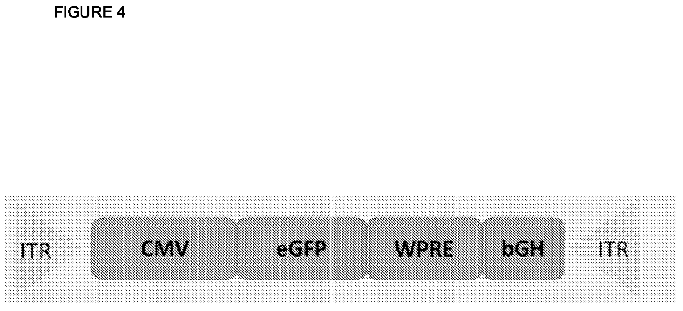

**Figure 4** **- A schematic representation of an AAV vector encoding eGFP under control of a CMV promoter**
   CMV: cytomegalovirus; bGH: bovine growth hormone; eGFP: enhanced green fluorescence protein; ITR: inverted terminal repeat; WPRE: woodchuck post-transcriptional regulatory element.

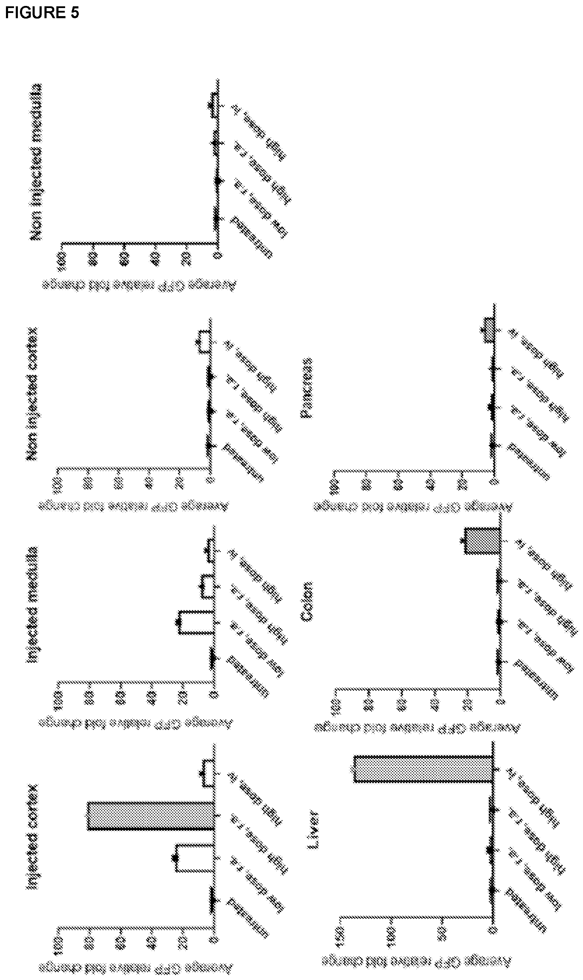

**Figure 5** **- Transcriptomic Analysis of mRNA GFP Expression (rAAV Injected Kidney Versus the Non injected Kidney) and Biodistribution (Colon, Liver, Pancreas)**
   GFP: green fluorescent protein; IV: intravenous; r.a.: direct renal artery injection.

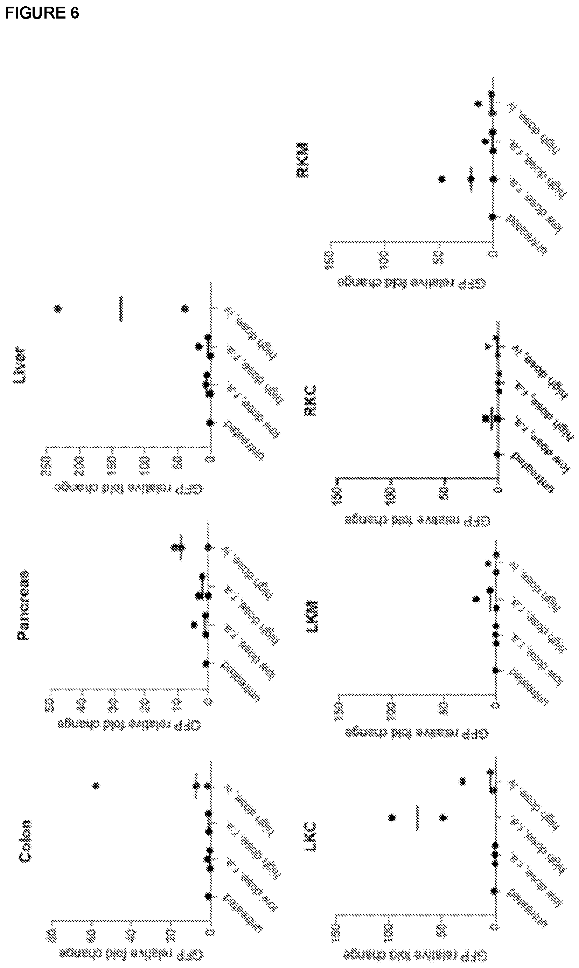

**Figure 6** **- Transcriptomic Analysis of mRNA GFP Expression in Pig Kidneys and Biodistribution (Colon, Liver, Pancreas)**
   GFP: green fluorescent protein; IV: intravenous; LKC: left kidney cortex; LKM: left kidney medulla; r.a.: direct Renal Artery injection; RKC: right kidney cortex; RKM: right kidney medulla.

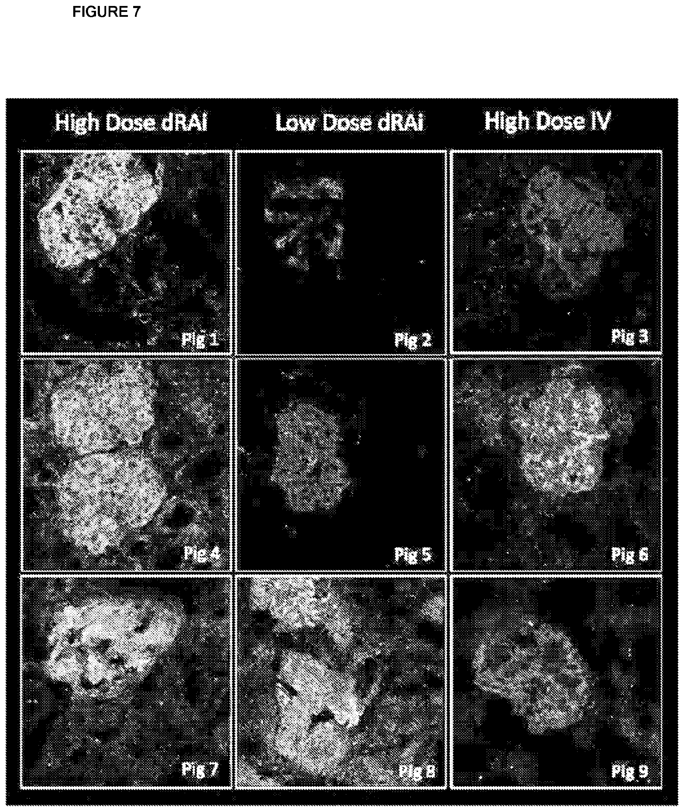

**Figure 7** **- Double Immunofluorescence of Porcine Left Kidney Cortex (Anti-GFP)**
   Composite images showing colocalization of GFP (green fluorescence protein) and nephrin in kidney cortex (63x magnification). dRAi: direct renal artery injection; IV: intravenous.

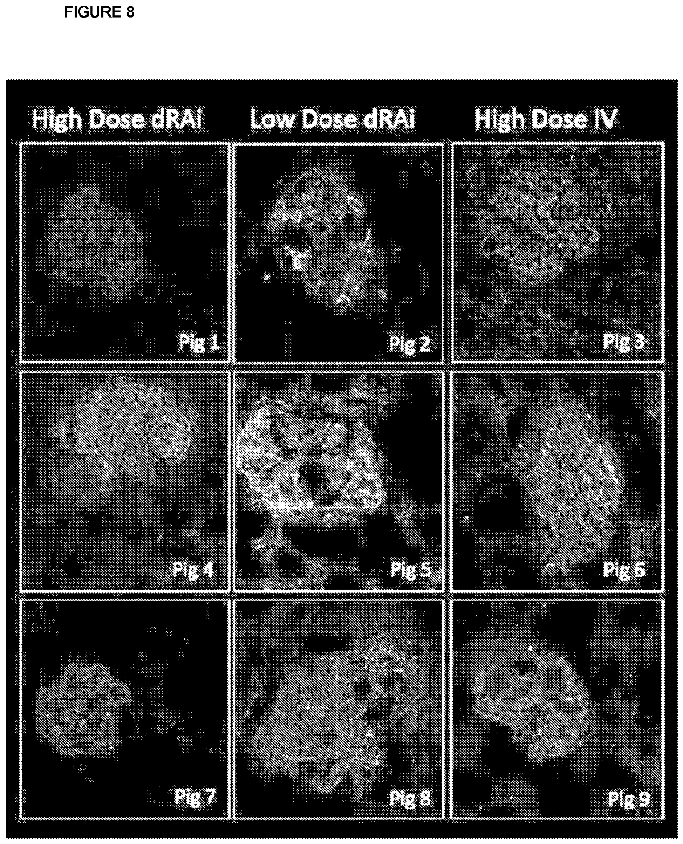

**Figure 8** **- Double Immunofluorescence of Porcine Right Kidney Cortex (Anti-GFP and Anti-nephrin)**
   Composite images showing colocalization of GFP (green fluorescence protein) and nephrin in kidney cortex (63x magnification). dRAi: direct renal artery injection; IV: intravenous.

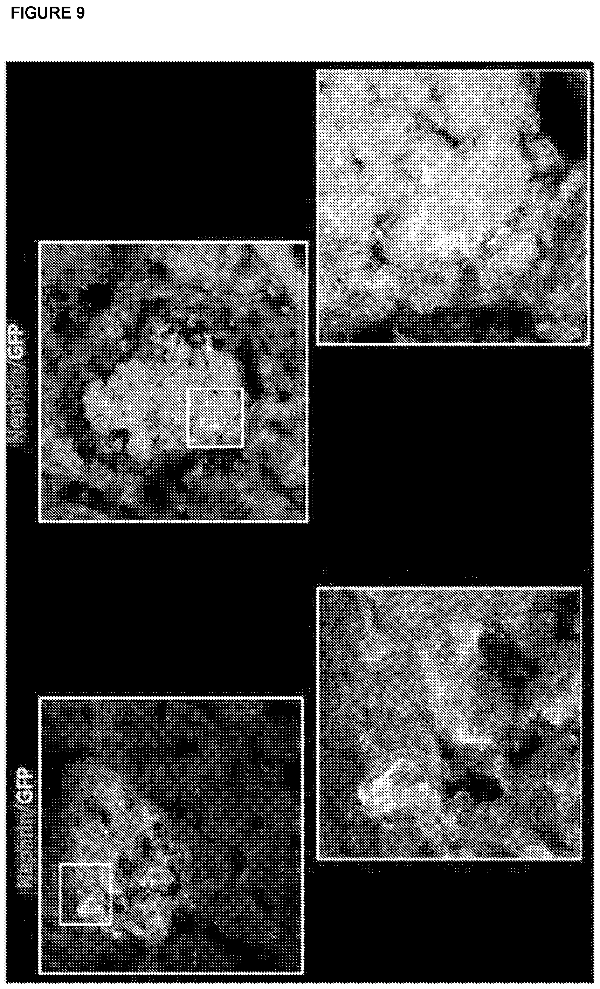

**Figure 9** **- High Magnification of Expression Within the Glomerulus Ultrastructure**
   Composite images showing colocalization of GFP (green fluorescence protein), nephrin, and DAPI (4',6-diamidino-2-phenylindole) within the glomerulus ultrastructure.

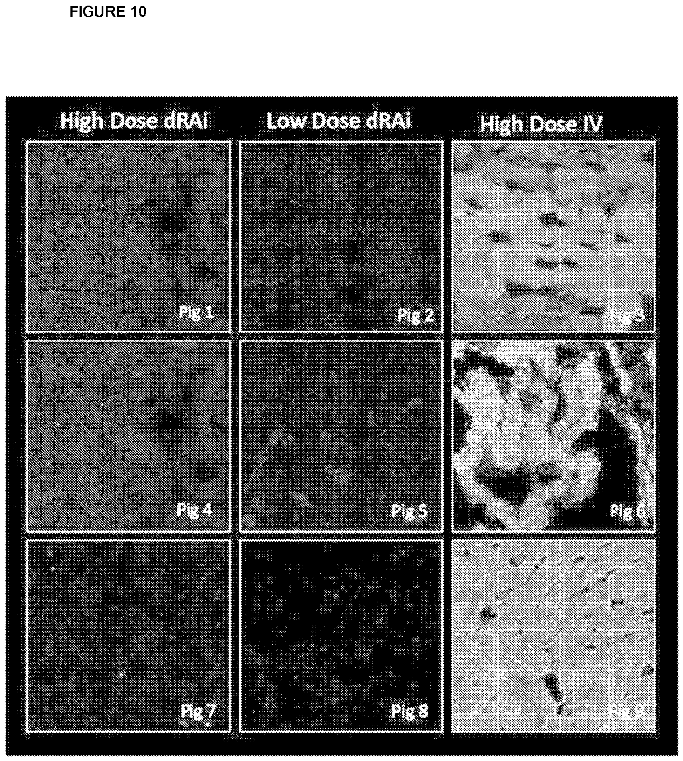

**Figure 10** **- Double Immunofluorescence of Porcine Liver (Anti-GFP)**
   Composite images showing colocalization of GFP (green fluorescence protein) and DAPI (4',6-diamidino-2-phenylindole) in liver tissue. As all images for the high and low dose dRAi treatment groups were negative for GFP, a representative sample for each animal in was taken at random. dRAi: direct renal artery injection; IV: intravenous.

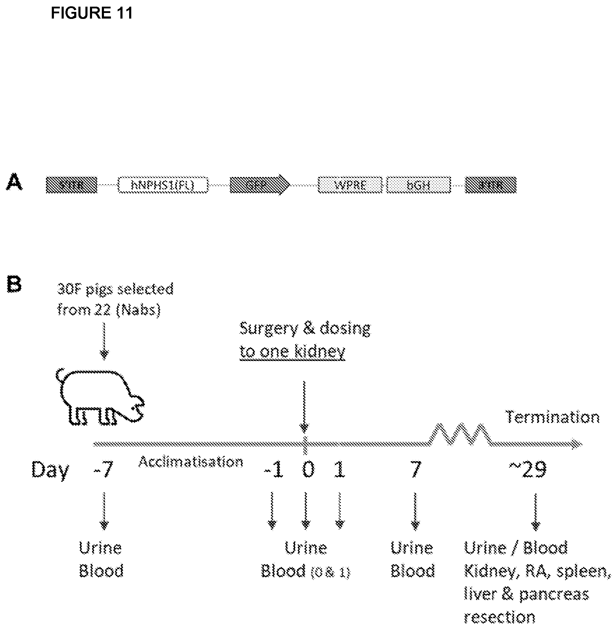

**Figure 11** **- Schematic representation of an AAV vector and study design**
   **(A)** A schematic representation of an AAV vector encoding GFP under control of a hNPHS1 (full-length) promoter. ITR: inverted terminal repeat; hNPHS1(FL) promoter: human full-length nephrin promoter; WPRE: woodchuck post-transcriptional regulatory element; bGH: bovine growth hormone polyA signal. **(B)** Schematic representation of study design.

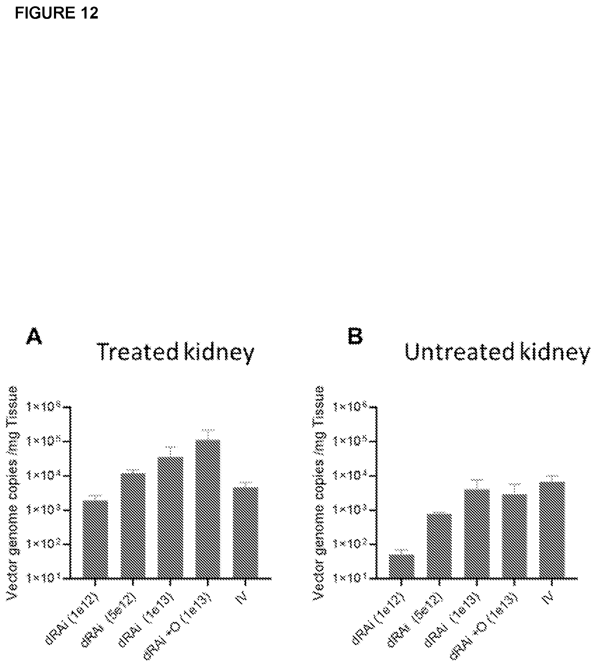

**Figure 12** **- Biodistribution by qPCR of tissue samples**
   AAV genomes detected per milligram (mg) of tissue following direct renal artery injection with (dRAi+O) or without (dRAi) renal artery occlusion, or IV injection. **(A)** Treated kidney; and (B) untreated kidney.

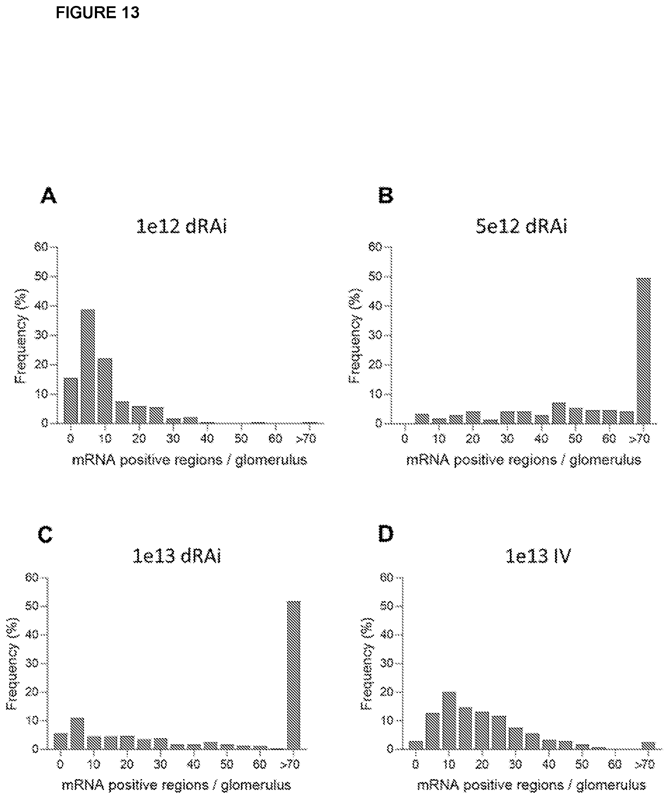

**Figure 13** **- RNAscope in-situ hybridisation (dRAi versus IV)**
   Paraffin embedded formalin fixed tissues were assessed by RNAscope in-situ hybridisation for the localised presence of AAV mRNA in kidney cortex samples following direct renal artery injection without renal artery occlusion (dRAi) or IV injection. Pigs received **(A)** 1x10¹² vg by dRAi; **(B)** 5x10¹² vg by dRAi; (C) 1x10¹³ vg by dRAi; or **(D)** 1x10¹³ vg by IV.

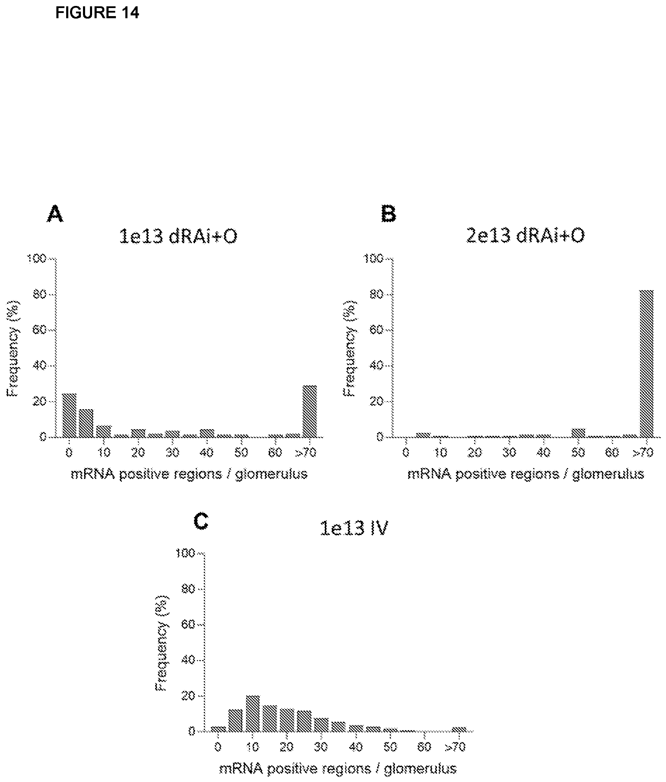

**Figure 14** **- RNAscope in-situ hybridisation (dRAi+O versus IV)**
   Paraffin embedded formalin fixed tissues were assessed by RNAscope in-situ hybridisation for the localised presence of AAV mRNA in kidney cortex samples following direct renal artery injection with renal artery occlusion (dRAi+O) or IV injection. Pigs received **(A)** 1x10¹³ vg by dRAi+O; **(B)** 2x10¹³ vg by dRAi+O; or (C) 1x10¹³ vg by IV.

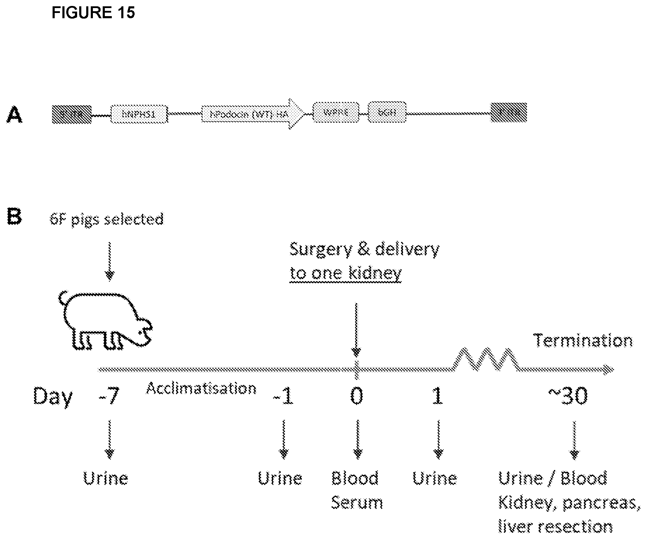

**Figure 15** **- Schematic representation of an AAV vector and study design**
   **(A)** A schematic representation of an AAV vector encoding podocin under control of a hNPHS1 promoter. ITR: inverted terminal repeat; hNPHS1: human full-length nephrin promoter; hPodocin (WT)-HA: wild-type human podocin transgene with haemagglutinin tag; WPRE: woodchuck post-transcriptional regulatory element; bGH: bovine growth hormone polyA signal. **(B)** Schematic representation of study design.

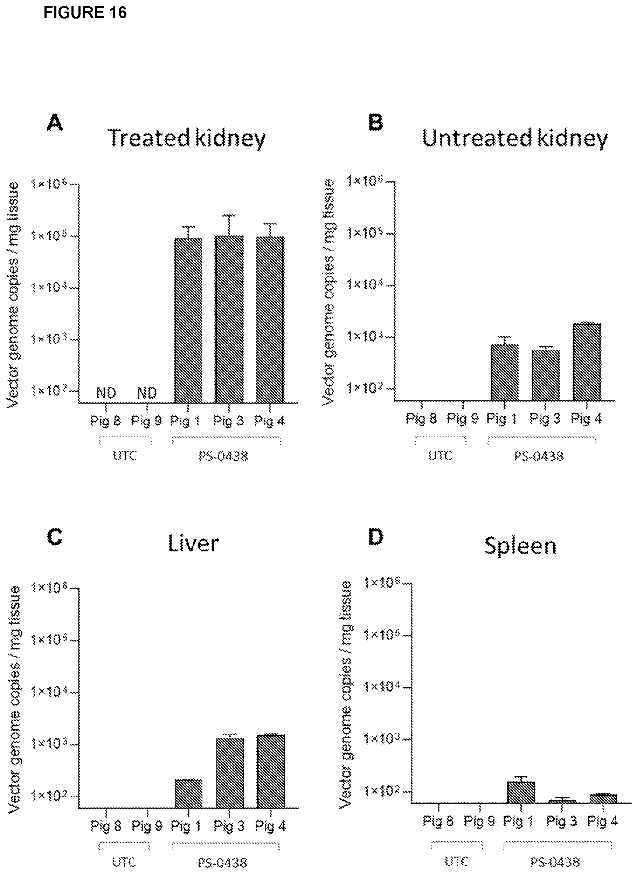

**Figure 16** **- Biodistribution by qPCR of tissue samples**
   AAV genomes detected per milligram (mg) of tissue for untreated control pigs (UTC) and pigs treated with PS0438. **(A)** Treated kidney; **(B)** untreated kidney; **(C)** liver; and **(D)** spleen.

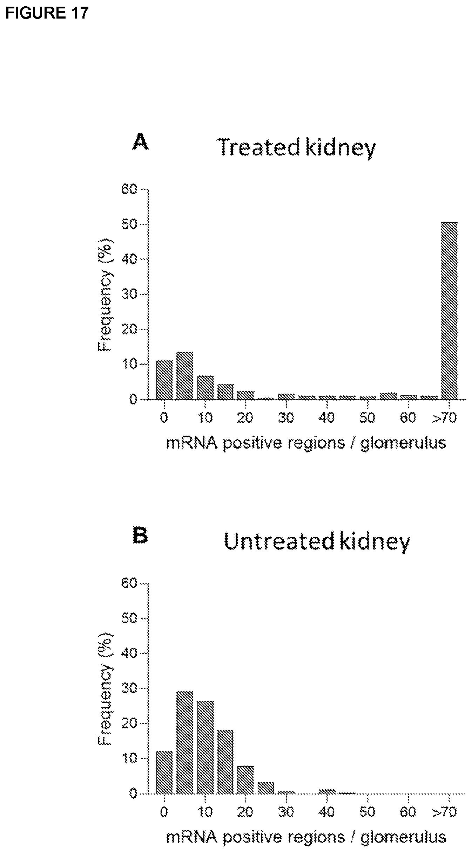

**Figure 17** **- RNAscope in-situ hybridisation (dRAi+O)**
   Paraffin embedded formalin fixed tissues were assessed by RNAscope in-situ hybridisation for the localised presence of AAV mRNA in kidney cortex samples following direct renal artery injection with renal artery occlusion (dRAi+O). **(A)** Treated kidney and **(B)** untreated kidney.

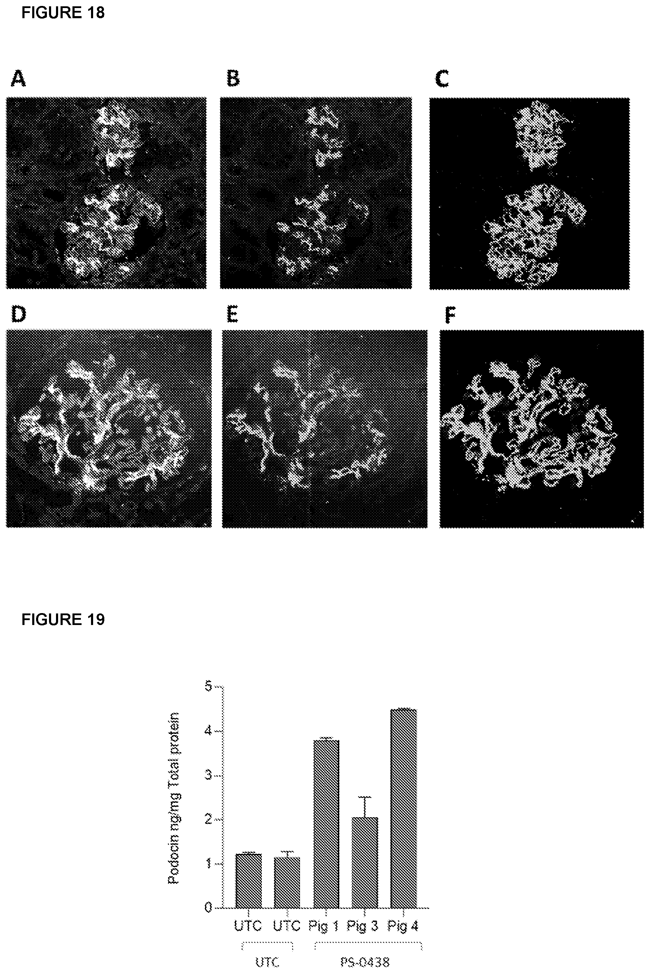

**Figure 18** **- Immunofluorescence of HA-tagged podocin and nephrin**
   Immunofluorescent (IF) analysis was performed on sections obtained from OCT blocks of kidney cortex. Sections were stained with DAPI and antibodies targeting WT-1, HA, and nephrin. **(A)** Composite image of two glomeruli and corresponding single channel images showing **(B)** HA-tagged podocin (green) and **(C)** nephrin. **(D)** Composite image of one glomerulus and corresponding single channel images showing **(E)** HA-tagged podocin (green) and **(F)** nephrin.
**Figure 19** **- Podocin ELISA of kidney cortex**
   Podocin protein detected in kidney cortex samples by ELISA in nanograms per milligram of total protein for untreated control pigs (UTC) and pigs treated with PS0438.

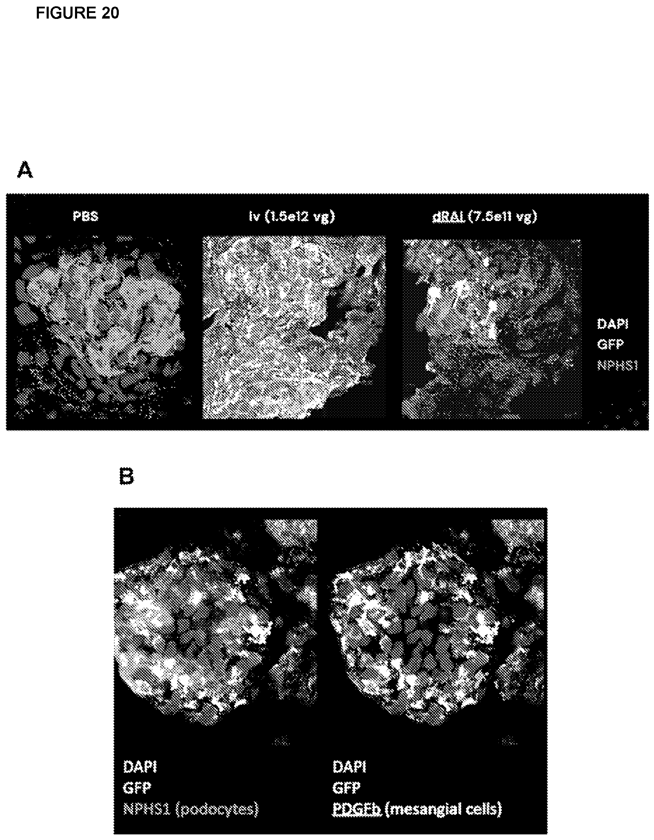

**Figure 20** **- Double Immunofluorescence of glomeruli**
   **(A)** Composite images are shown for glomeruli stained with DAPI and antibodies targeting nephrin. GFP expression is absent in the negative control (PBS, left panel) and higher following direct renal artery injection (dRAi, right panel) compared to IV injection (iv, middle panel). **(B)** Composite images are shown for glomeruli stained with DAPI and antibodies targeting nephrin (left panel) or PDGFb (right panel). GFP expression is co-localised with nephrin (NPHS1) and PDGFb, confirming localisation in podocytes and mesangial cells, respectively.

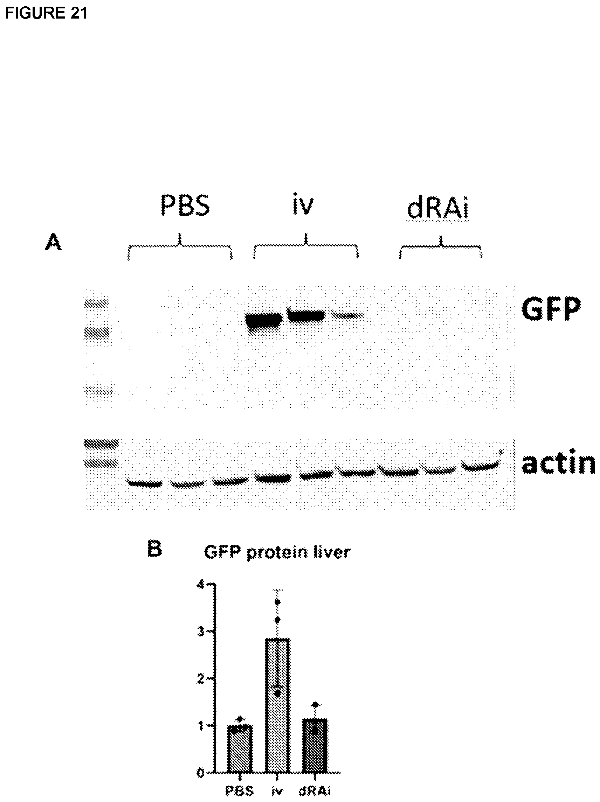

**Figure 21** **- Western blot analysis**
   **(A)** Western blot analysis and **(B)** densitometry of mouse liver samples demonstrates higher GFP expression by IV administration of the AAV vector in comparison to administration of the AAV vector by direct renal artery injection (dRAi).

### DETAILED DESCRIPTION

Various preferred features and embodiments of the present invention will now be described by way of non-limiting examples. This disclosure is not limited by the exemplary methods and materials disclosed herein, and any methods and materials similar or equivalent to those described herein can be used in the practice or testing of embodiments of this disclosure. The skilled person will understand that they can combine all features of the invention disclosed herein without departing from the scope of the invention as disclosed.

The invention is defined by the appended claims.

It must be noted that as used herein and in the appended claims, the singular forms "a", "an", and "the" include plural referents unless the context clearly dictates otherwise.

The terms "comprising", "comprises" and "comprised of" as used herein are synonymous with "including", "includes", "containing", or "contains", and are inclusive or open-ended and do not exclude additional, non-recited members, elements or steps. The terms "comprising", "comprises" and "comprised of" also include the term "consisting of".

Numeric ranges are inclusive of the numbers defining the range. As used herein the term "about" means approximately, in the region of, roughly, or around. When the term "about" is used in conjunction with a numerical value or range, it modifies that value or range by extending the boundaries above and below the numerical value(s) set forth. In general, the terms "about" and "approximately" may be used herein to modify a numerical value(s) above and below the stated value(s) by 10%.

Unless otherwise indicated, any nucleic acid sequences are written left to right in 5' to 3' orientation; amino acid sequences are written left to right in amino to carboxy orientation, respectively.

The publications discussed herein are provided solely for their disclosure prior to the filing date of the present application. Nothing herein is to be construed as an admission that such publications constitute prior art to the claims appended hereto.

### Glomerulus and podocyte gene therapy

The glomerulus is the filtration unit of the kidneys. Approximately 180 litres of plasma are filtered each day, and the healthy glomerular filtration barrier has an astonishing ability to retain about 99.9% of large proteins including albumin over our lifetimes without clogging. The afferent arteriole enters into the glomerular capillary bed, where filtration occurs, and blood leaves the glomerulus via the efferent arteriole. The glomerular filtration barrier (GFB) comprises 3 main layers: the glomerular endothelial cell, the glomerular basement membrane (GBM) and the podocyte.

The podocyte, the third layer of the GFB, plays a key role in the maintenance of the GFB. The podocyte is a highly-specialised cell, comprising of a cell body, major processes, secondary processes and foot processes that interdigitate with foot processes of adjacent podocytes to form the slit diaphragm. Podocytes form an effective and dynamic sieve, and this is predominantly thought to be due to the integrity of the slit diaphragm.

Gene therapy targeting the glomerulus is challenging. For example, although lentivirus might be of utility in transducing the tubules, it has thus far not shown any in vivo transduction of the glomerulus. Moreover, initial attempts to deliver adenovirus via either the renal artery or retrograde delivery via the ureter seemed to mainly result in tubular or interstitial transduction. Initial studies on the rodent kidney using AAV2 demonstrated mostly transduction of the tubules and no expression in the glomerulus.

### Method of delivery

The present invention provides a method of delivering a viral vector to a kidney via direct renal artery injection. The method may comprise the steps of inserting a catheter into the renal artery and injecting or infusing the viral vector into the renal artery via the catheter. In the context of the present invention, the term "delivering" may be used interchangeably with the term "administering". The method is a minimally invasive procedure, i.e. the method is a minimally invasive method. As used herein, a "minimally invasive procedure" or "minimally invasive surgeries" may refer to surgical techniques that limit the size of incisions needed, thereby reducing wound healing time, associated pain, and risk of infection (see e.g. Jaffray, B., 2005.Archives of disease in childhood, 90(5), pp.537-542). In contrast, incisions made during "open surgery" can sometimes leave large wounds that may be painful and take a long time to heal. Many medical procedures are called minimally invasive including percutaneous surgery. In preferred embodiments, the method does not comprise open surgery.

In some embodiments, the method does not comprise inserting more than one catheter. The method does not comprise a step of inserting a catheter into the renal vein. In some embodiments, the method does not comprise a step of inserting a catheter directly into the aorta (e.g. into the infrarenal aorta).

The method does not comprise any clamping steps (e.g. clamping of renal artery, renal vein, or aorta). The method does not comprise a step of clamping the renal artery. The method does not comprise a step of clamping the renal vein. The method does not comprise a step of clamping the aorta (e.g. the suprarenal aorta).

In some embodiments, the method has a total kidney ischemia time of about 1 hour or less. As used herein, "kidney ischemia time" may refer to the time from the cutting off or reduced blood supply to the kidney to the time when normal blood supply to the kidney is restored. A reduced kidney ischemia may result in reduced kidney damage or reduced risk or kidney damage.

In some embodiments, the total kidney ischemia time is about 60 minutes or less, about 50 minutes or less, about 40 minutes or less, about 30 minutes or less, about 25 minutes or less, about 20 minutes or less, about 15 minutes or less, about 10 minutes or less, about 5 minutes or less, about 4 minutes or less, about 3 minutes or less, about 2 minutes or less, or about 1 minute or less. In some embodiments, the total kidney ischemia time is about 0 minutes.

In some embodiments, the total kidney ischemia time is from about 10 minutes to about 30 minutes, from about 15 to about 25 minutes, or about 20 minutes.

In some embodiments, the total kidney ischemia time is about 5 minutes.

### Step (a): insertion of the catheter

In the method of the present invention, a catheter is inserted into the renal artery.

As used herein, a "catheter" may refer to a thin tube made from medical grade materials that can be inserted in the body to perform a surgical procedure. In most uses, a catheter is a thin, flexible tube (a soft catheter) though catheters are available in varying levels of stiffness depending on the application. Suitably, the catheter is a percutaneous catheter. In some embodiments, the catheter is an occlusion balloon catheter.

As used herein, insertion "into the renal artery" means that following insertion of the catheter the end of the catheter is located within the renal artery. The present method is distinguished from methods in which the catheter is located beneath the renal artery. The renal artery normally arises at a 90° angle off of the left interior side of the abdominal aorta, immediately below the superior mesenteric artery, and may have a radius of approximately 0.25 cm. Before reaching the hilus of the kidney, each renal artery divides into four or five branches. The term "renal artery" may refer to any of these branches.

The catheter may be inserted into the renal artery by any suitable route. In preferred embodiments, the catheter is inserted into the renal artery via a percutaneous route. Such a route is compatible with a minimally invasive procedure. The percutaneous route may be any suitable route, such as via the carotid artery or via the femoral artery. In some embodiments, the catheter is inserted into the renal artery via the carotid artery. In some embodiments, the catheter is inserted into the renal artery via the (common) femoral artery. Standard procedures will be known to one of skill in the art to insert a catheter via a percutaneous route. For example, the carotid artery or (common) femoral artery may be catheterised, a sheath may be introduced, and a guidewire may be introduced to facilitate introduction of the catheter into the renal artery.

In some embodiments, the method comprises a step of catheterising the carotid artery or (common) femoral artery. In some embodiments, the insertion of the catheter via the percutaneous route is facilitated with a sheath. In some embodiments, the method comprises a step of introducing a sheath into the carotid artery or (common) femoral artery. As used herein, a "sheath" may refer to a short hollow tube that may be introduced into a vessel to facilitate catheterisation. The sheath may remain in place until after completion of the procedure and removal of the catheter. In some embodiments, the catheter is inserted into the renal artery via a guidewire. In some embodiments, the method comprises a step of introducing a guidewire into the renal artery. As used herein, a "guidewire" may refer to a thin wire used to guide the placement of a catheter within a vessel.

In some embodiments, the step (a) of inserting a catheter into the renal artery comprises:
(a1) catheterising the carotid artery or (common) femoral artery;
(a2) inserting a sheath in the carotid artery or (common) femoral artery;
(a3) inserting a guidewire into the renal artery; and
(a4) inserting a catheter into the renal artery.

### Step (b): occlusion of vessels

The method may optionally include a step of occluding the renal artery. This may increase the residence time of the viral vector in the kidney, thereby increasing viral transduction.

As used herein, "occlusion" of a vessel may refer to a complete blocking of blood flow through the vessel. The occlusion may be checked by any suitable method. For example, by injecting or infusing a contrast agent and imaging flow by an angiogram. The occlusion may be by any suitable method, preferably via inflating a balloon. Such a method is compatible with a minimally invasive procedure.

In some embodiments, the method comprises the steps of:
(a) inserting a balloon catheter into the renal artery;
(b) inflating the balloon to occlude the renal artery; and
(c) injecting or infusing the viral vector into the renal artery via the catheter.

In some embodiments, the renal artery is occluded for about 1 minute or more, about 2 minutes or more, about 3 minutes or more, about 4 minutes or more, about 5 minutes or more, about 6 minutes or more, about 7 minutes or more, about 8 minutes or more, about 9 minutes or more, about 10 minutes or more, about 11 minutes or more, about 12 minutes or more, about 13 minutes or more, about 14 minutes or more, about 15 minutes or more, about 16 minutes or more, about 17 minutes or more, about 18 minutes or more, about 19 minutes or more, or about 20 minutes or more.

In some embodiments, the renal artery is occluded for about 30 minutes or less, about 25 minutes or less, about 20 minutes or less, about 19 minutes or less, about 18 minutes or less, about 17 minutes or less, about 16 minutes or less, about 15 minutes or less, about 14 minutes or less, about 13 minutes or less, about 12 minutes or less, about 11 minutes or less, about 10 minutes or less, about 9 minutes or less, about 8 minutes or less, about 7 minutes or less, about 6 minutes or less, about 5 minutes or less, about 4 minutes or less, about 3 minutes or less, about 2 minutes or less, about 1 minute or less.

In some embodiments, the renal artery is occluded for from about 1 minute to about 30 minutes or from about 1 minute to about 25 minutes. In some embodiments, the renal artery is occluded for about 1 minute, about 2 minutes, about 3 minutes, about 4 minutes, about 5 minutes, about 6 minutes, about 7 minutes, about 8 minutes, about 9 minutes, about 10 minutes, about 11 minutes, about 12 minutes, about 13 minutes, about 14 minutes, about 15 minutes, about 16 minutes, about 17 minutes, about 18 minutes, about 19 minutes, about 20 minutes, about 21 minutes, about 22 minutes, about 23 minutes, about 24 minutes, about 25 minutes, about 26 minutes, about 27 minutes, about 28 minutes, about 29 minutes, or about 30 minutes.

In some embodiments, the renal artery is occluded for from about 2 minutes to about 10 minutes, from about 3 minutes to about 8 minutes, from about 4 minutes to about 6 minutes, or about 5 minutes.

In some embodiments, the renal artery is occluded for from about 10 minutes to about 30 minutes, from about 15 minutes to about 25 minutes, from about 15 minutes to about 20 minutes, or about 20 minutes.

In other embodiments, the method does not comprise a step of occluding the renal artery.

In preferred embodiments, the method does not comprise a step of occluding the renal vein. Occlusion of the renal vein may increase the risk of renal vein thrombosis. In subjects with kidney disease, the kidneys may be at an increased risk of thrombosis.

In preferred embodiments, the method does not comprise a step of occluding the aorta.

In some embodiments, the method does not comprise a step of occluding the renal vein or the aorta.

In some embodiments, the method does not comprise a step of occluding the renal artery, the renal vein, or the aorta.

In some embodiments, the method does not comprise occluding any of the subject's blood vessels.

### Step (c): injection or infusion of viral vector

In the method of the present invention, the viral vector is delivered into the renal artery via the catheter. The viral vector may be delivered by any suitable method, for example by injection or infusion.

Suitably, the viral vector may be delivered into the renal artery over about 1 minute to about 30 minutes. In some embodiments, the viral vector is delivered into the renal artery over about 1 minute to about 25 minutes. In some embodiments, the viral vector is delivered into the renal artery over about 1 minute, about 2 minutes, about 3 minutes, about 4 minutes, about 5 minutes, about 6 minutes, about 7 minutes, about 8 minutes, about 9 minutes, about 10 minutes, about 11 minutes, about 12 minutes, about 13 minutes, about 14 minutes, about 15 minutes, about 16 minutes, about 17 minutes, about 18 minutes, about 19 minutes, about 20 minutes, about 21 minutes, about 22 minutes, about 23 minutes, about 24 minutes, about 25 minutes, about 26 minutes, about 27 minutes, about 28 minutes, about 29 minutes, or about 30 minutes. Suitably, the viral vector is delivered at approximately a constant rate.

In some embodiments, the viral vector is injected into the renal artery via the catheter. As used herein, "injection" may refer to delivery under pressure or flow (e.g. by forcing a liquid into the body by means of a syringe). In some embodiments, the viral vector is injected into the renal artery without using an infusion pump. In some embodiments, the viral vector is injected into the renal artery over about 5 minutes to about 30 minutes, over about 10 minutes to about 25 minutes, over about 15 minutes to about 20 minutes, over about 16 minutes to about 18 minutes, or over about 17 minutes.

In some embodiments, the viral vector is infused into the renal artery via the catheter. As used herein, "infusion" may refer to delivery under atmospheric pressure or no flow (e.g. by using an infusion pump). In some embodiments, the viral vector is infused into the renal artery under no flow conditions using an infusion pump. In some embodiments, the viral vector is infused into the renal artery over about 5 minutes to about 30 minutes, over about 10 minutes to about 25 minutes, over about 15 minutes to about 20 minutes, or over about 17 minutes. In some embodiments, the viral vector is infused into the renal artery over about 1 minute to about 5 minutes, about 1 minute to about 4 minutes, about 1 minute to about 3 minutes, or about 2 minutes. In some embodiments, the viral vector is infused into the renal artery over about 1 minute to about 10 minutes, about 2 minutes to about 8 minutes, about 3 minutes to about 5 minutes, or about 4 minutes.

### Other method steps

The method of the present invention may include any other suitable steps, for example flushing the catheter, withdrawing the catheter, withdrawing the sheath, and/or closing any opening.

In some embodiments, the method further comprises a step of flushing the catheter. Such a step may ensure that viral vector remains within the catheter. The catheter may be flushed with any suitable solution, such as an isotonic solution. In some embodiments, the catheter is flushed with saline (e.g. heparinised saline) or phosphate buffered saline (PBS). In some embodiments, the catheter is flushed with heparinised saline.

In some embodiments, the method comprises a step of flushing the catheter before injecting or infusing the viral vector. In some embodiments, the method comprises a step of flushing the catheter after injecting or infusing the viral vector. In some embodiments, the method comprises a step of flushing the catheter before and after injecting or infusing the viral vector.

In some embodiments, the method further comprises a step of deflating the balloon. In some embodiments, the method further comprises a step of withdrawing the catheter. In some embodiments, the method further comprises a step of withdrawing the sheath. Suitably, the catheter and sheath are removed slowly to avoid damaging vessels. In some embodiments, the method further comprises closing the opening.

In some embodiments, the method comprises the steps of:
(a) inserting a balloon catheter into the renal artery;
(b) optionally inflating the balloon to occlude the renal artery;
(c) injecting or infusing the viral vector into the renal artery via the catheter; and
(d) flushing the catheter, optionally deflating the balloon, and withdrawing the catheter.

In some embodiments, the method does not comprise forming a closed circuit through the kidney. As used herein, a "closed circuit" through the kidney may isolate the viral vector formulation from the subject's systemic circulation. In some embodiments, less than about 20% v/v of the viral vector formulation circulated through a closed circuit leaks outside of the closed circuit. For example, a closed circuit through the kidney may comprise a catheter inserted into the renal artery and a catheter inserted into the renal vein. A pump may drive fluid flow through into the kidney via the renal artery catheter and out of the kidney via the renal vein catheter.

In some embodiments, the method does not comprise use of a membrane oxygenation device. In some embodiments, the viral vector formulation does not pass through a membrane oxygenation device. In some embodiments, a closed circuit further comprises a membrane oxygenation device.

In some embodiments, the method does not comprise a step of perfusing the kidney with saline prior to injecting or infusing the viral vector into the renal artery.

### Kidney-specific delivery

The method of the present invention results in delivery of the viral vector to the kidney.

As used herein, "delivery" of the viral vector may refer to transduction with the viral vector, which may subsequently result in expression of an RNA and/or a protein encoded by the viral vector. Delivery of the viral vector to specific organs, tissues or cell-types may be determined by any suitable method, including transcriptomic analysis and/or immunofluorescence.

The method of the present invention results in transduction of the kidney with the viral vector, and subsequent expression of an RNA encoded by the viral vector in the kidney and/or expression of a protein encoded by the viral vector in the kidney. Transduction of specific organs, tissues or cell-types may be determined by viral load or viral copy number, which may be determined by any suitable method (see e.g. Dobnik, D., et al., 2019. Frontiers in microbiology, 10, p.1570). Expression of RNA in specific organs, tissues or cell-types may be determined by transcriptomics. For example, RNA may be extracted from tissues, converted to cDNA, and quantified by qPCR. Expression of protein in specific organs, tissues or cell-types may be determined by immunofluorescence. For example, by labelling the protein with a fluorescent marker and quantifying by fluorescence microscopy.

The method may result in delivery of the viral vector to the kidney cortex and/or kidney medulla. In preferred embodiments, the method results in delivery of the viral vector to the kidney cortex. In more preferred embodiments, the method results in delivery of the viral vector to the kidney glomeruli. In even more preferred embodiments, the method results in delivery of the viral vector to the kidney podocytes.

The method of the present invention may result in kidney-specific delivery of the viral vector. As used herein, "kidney-specific delivery" may mean that the viral vector predominantly transduces the kidney, which may subsequently result in expression of an RNA and/or a protein encoded by the viral vector predominantly in the kidney.

Suitably, the method results in delivery of the viral vector to the kidney, but does not result in significant delivery of the viral vector to other tissues. In some embodiments, the other tissues are selected from one or more of liver, colon and pancreas. In some embodiments, the method results in delivery of the viral vector to the kidney, but does not result in significant delivery of the viral vector to the liver.

The viral vector may be delivered to a single kidney or both kidneys of the subject. In some embodiments, the method delivers the viral vector to a single kidney of the subject. In some embodiments, the method is repeated to deliver the viral vector to both kidneys of the subject.

In one embodiment, the method comprises: (1) delivering the viral vector to the subject's right kidney, by a method according to the present invention; and/or (2) delivering the viral vector to the subject's left kidney, by a method according to the present invention.

In one embodiment, the method comprises: (1) delivering the viral vector to the subject's right kidney, by a method according to the present invention; and (2) delivering the viral vector to the subject's left kidney, by a method according to the present invention.

Steps (1) and (2) can be in any order and can be carried out over any duration. In some embodiments, steps (1) and (2) are carried out within a single day. In some embodiments, steps (1) and (2) are carried out as part of the same procedure. In some embodiments, steps (2) directly follows step (1) or step (1) directly follows step (2).

The method of the present invention may be carried out one or more times for a subject. The method may be repeated over any suitable period. Suitably, the method of the present invention is carried out once on a kidney (i.e. the viral vector is administered as a single dose). In some embodiments, the method of the present invention is carried out once to deliver the viral vector to a single kidney of the subject. In some embodiments, the method is carried out twice to deliver the viral vector to both kidneys of the subject.

### Subject

The subject is a human. The subject may be an adult, an adolescent, or a child.

The subject has or is at risk of a kidney disease. For example, the subject may have or may be at risk of a glomerular disease. For example, the subject may have or may be at risk of a podocyte-associated glomerular disease. For example, the subject may have or may be at risk of a GBM-associated glomerular disease.

In some embodiments, the subject has or is at risk of a genetic glomerular disease, i.e. a glomerular disease which is inherited. Genetic glomerular diseases include podocyte-associated genetic glomerular diseases, such as nephrotic syndrome, and GBM-associated glomerular diseases, such as Alport Syndrome.

In some embodiments, the subject has or is at risk of a podocyte-associated genetic glomerular disease. Podocyte-associated genetic glomerular diseases include Congenital nephrotic syndrome of the Finnish type, Congenital nephrotic syndrome type 2, Familial nephrotic syndrome type 3, Frasier syndrome and Denys-Drash syndrome, Schimke immuno-osseous dysplasia, Nephrotic syndrome caused by mutations in CD2AP, Nephrotic syndrome caused by mutations in actinin-4, Nephrotic syndrome caused by mutations in TRPC6, and Epstein and Fechtner syndrome. Suitably, the glomerular disease is nephrotic syndrome.

In some embodiments, the subject has or is at risk of a GBM-associated genetic glomerular disease. GBM-associated genetic glomerular diseases include X-linked Alport syndrome, Autosomal recessive Alport syndrome, Autosomal dominant Alport syndrome, Thin basement membrane diseases, Pierson syndrome, and Nail-patella syndrome. Suitably, the glomerular disease is Alport syndrome (AS). AS is also known as familial nephritis, hereditary nephritis, thin basement membrane disease and thin basement membrane nephropathy.

In some embodiments, the subject has or is at risk of a complement-mediated kidney disease. As used herein a "complement-mediated kidney disease" is a disease of the kidney which is caused by dysregulation of the complement system. The complement system can cause kidney injury in a variety of different diseases. Suitably, the complement-mediated kidney disease is caused by excessive activation of the complement system. Exemplary complement-mediated kidney diseases include IgA nephropathy, C3 glomerulopathy, atypical hemolytic uremic syndrome (aHUS), stx-associated HUS, lupus nephritis, cryoglobulinemia, anti-GBM disease, ANCA-associated vasculitis, bacterial endocarditis, post-infectious glomerulonephritis, antibody-mediated rejection of renal transplant, membranous nephropathy, membranoproliferative glomerulonephritis I, or membranoproliferative glomerulonephritis III.

In some embodiments, the subject has or is at risk of diabetic nephropathy. Diabetic nephropathy, also known as diabetic kidney disease, is the chronic loss of kidney function occurring in those with diabetes mellitus.

In some embodiments, the subject has or is at risk of Fabry disease. Fabry disease is an inherited disorder that results from the accumulation of globotriaosylceramide within lysosomes. Kidney complications are common and serious effects of the disease.

### Viral vector dosage

The viral vector may be delivered in any suitable dosage (e.g. measured in vector genomes (vg) or vg per kg). The dosage may be determined by such factors as the condition of the subject, age of the subject, weight of the subject, and the type and severity of the subject's disease, and appropriate dosages may be determined by a physician. The viral vector may be formulated accordingly.

The method of the present invention can allow a lower dose of viral vector to be used in comparison to other delivery methods (e.g. systemic administration such as intravenous administration). Consequently, the method of the present invention is associated with less risk of an immune response and fewer off-target effects, such as lower expression in the liver or no liver expression of a protein encoded by the viral vector compared to systematic administration such as intravenous administration.

Suitably, the viral vector is delivered in a dose of about 1x10⁶ vg/kg or more, about 1x10⁷ vg/kg or more, about 1x10⁶ vg/kg or more, about 1x10⁹ vg/kg or more, about 1x10¹⁰ vg/kg or more, about 1x10¹¹ vg/kg or more, or about 1x10¹² vg/kg or more. Suitably, the viral vector is delivered in a dose of about 1x10¹⁴ vg/kg or less or about 1x10¹³ vg/kg or less. Suitably, the viral vector is delivered in a dose of from about 1x10⁶ vg/kg to about 1x10¹⁴ vg/kg. Suitably, the viral vector is delivered in a dose of from about 1x10⁶ vg/kg to about 1x10¹³ vg/kg. Suitably, the viral vector is delivered in a dose of about 1x10⁶ vg/kg to about 10x10⁶ vg/kg, about 1x10⁷ vg/kg to about 10x10⁷ vg/kg, about 1x10⁶ vg/kg to about 10x10⁸ vg/kg, about 1x10⁹ vg/kg to about 10x10⁹ vg/kg, about 1x10¹⁰ vg/kg to about 10x10¹⁰ vg/kg, about 1x10¹¹ vg/kg to about 10x10¹¹ vg/kg, or about 1x10¹² vg/kg to about 10x10¹² vg/kg.

In some embodiments, the viral vector is delivered in a dose of from about 1x10⁹ vg/kg to about 1x10¹² vg/kg. In some embodiments, the viral vector is delivered in a dose of from about 3x10⁹ vg/kg to about 3x10¹¹ vg/kg.

Suitably, the viral vector is delivered in a dose of about 1x10⁶ vg or more, about 1x10⁹ vg or more, about 1x10¹⁰ vg or more, about 1x10¹¹ vg or more, about 1x10¹² vg or more, about 1x10¹³ vg or more, or about 1x10¹⁴ vg or more. Suitably, the viral vector is delivered in a dose of about 1x10¹⁵ vg or less or about 1x10¹⁴ vg or less. Suitably, the viral vector is delivered in a dose of from about 1x10⁶ vg to about 1x10¹⁵ vg. Suitably, the viral vector is delivered in a dose of from about 1x10⁶ vg to about 5x10¹⁴ vg. Suitably, the viral vector is delivered in a dose of from about 1x10⁶ vg to about 1x10¹⁴ vg, from about 1x10⁹ vg to about 1x10¹⁴ vg, from about 1x10¹⁰ vg to about 1x10¹⁴ vg, from about 1x10¹¹ vg to about 1x10¹⁴ vg, or from about 1x10¹² vg to about 1x10¹⁴ vg. Suitably, the viral vector is delivered in a dose of about 1x10⁶ vg to about 10x10⁸ vg, about 1x10⁹ vg to about 10x10⁹ vg, about 1x10¹⁰ vg to about 10x10¹⁰ vg, about 1x10¹¹ vg to about 10x10¹¹ vg, about 1x10¹² vg to about 10x10¹² vg, about 1x10¹³ vg to about 10x10¹³ vg, or about 1x10¹⁴ vg to about 10x10¹⁴ vg.

In some embodiments, the viral vector is delivered in a dose of from about 1x10¹¹ vg to about 1x10¹⁴ vg. In some embodiments, the viral vector is delivered in a dose of about 1x10¹¹ vg, about 2x10¹¹ vg, about 3x10¹¹ vg, about 4x10¹¹ vg, about 5x10¹¹ vg, about 6x10¹¹ vg, about 7x10¹¹ vg, about 8x10¹¹ vg, about 9x10¹¹ vg, about 1x10¹² vg, about 2x10¹² vg, about 3x10¹² vg, about 4x10¹² vg, about 5x10¹² vg, about 6x10¹² vg, about 7x10¹² vg, about 8x10¹² vg, about 9x10¹² vg, about 1x10¹³ vg, about 2x10¹³ vg, about 3x10¹³ vg, about 4x10¹³ vg, about 5x10¹³ vg, about 6x10¹³ vg, about 7x10¹³ vg, about 8x10¹³ vg, about 9x10¹³ vg, or about 1x10¹⁴ vg.

In some embodiments, the viral vector is delivered in a dose of from about 2x10¹¹ vg to about 2x10¹³ vg, for example based on a 70 kg subject.

In some embodiments, the viral vector is delivered in a dose of from about 5x10¹¹ vg to about 5x10¹³ vg. In some embodiments, the viral vector is delivered in a dose of from about 1x10¹² vg to about 5x10¹³ vg. In some embodiments, the viral vector is delivered in a dose of from about 5x10¹² vg to about 5x10¹³ vg.

In some embodiments, the viral vector is delivered in a dose of from about 5x10¹¹ vg to about 2x10¹³ vg. In some embodiments, the viral vector is delivered in a dose of from about 1x10¹² vg to about 2x10¹³ vg. In some embodiments, the viral vector is delivered in a dose of from about 5x10¹² vg to about 2x10¹³ vg.

In some embodiments, the viral vector is delivered in a dose of from about 5x10¹¹ vg to about 1x10¹³ vg. In some embodiments, the viral vector is delivered in a dose of from about 1x10¹² vg to about 1x10¹³ vg. In some embodiments, the viral vector is delivered in a dose of from about 5x10¹² vg to about 1x10¹³ vg.

In some embodiments, the viral vector is delivered in a dose of about 1x10¹³ vg.

### Viral vector formulation

The viral vector may be delivered into the renal artery in the form of a viral vector formulation.

As used herein, a "viral vector formulation" may refer to a composition that comprises or consists of a therapeutically effective amount of a viral vector. It preferably includes a pharmaceutically acceptable carrier, diluent or excipient (including combinations thereof). By "pharmaceutically acceptable" is included that the formulation is sterile and pyrogen free. The carrier, diluent, and/or excipient must be "acceptable" in the sense of being compatible with the viral vector and not deleterious to the recipients thereof. Typically, the carriers, diluents, and excipients will be saline or infusion media which will be sterile and pyrogen free, however, other acceptable carriers, diluents, and excipients may be used.

The viral vector may be delivered into the renal artery in the form of a sterile aqueous solution which may contain other substances, for example, enough salts or glucose to make the solution isotonic with blood. The aqueous solution may be suitably buffered (preferably to a pH of from 3 to 9). The preparation of suitable parenteral formulations under sterile conditions is readily accomplished by standard pharmaceutical techniques well-known to those skilled in the art.

Suitably, the viral vector formulation comprises an isotonic buffer (e.g. at about pH 7.4). In some embodiments, the viral vector formulation comprises phosphate buffered saline (PBS) buffer (e.g. pH 7.4). Optionally the PBS is supplemented with about 200 mM NaCl. In some embodiments, the viral vector formulation comprises plasmalyte. In some embodiments, the viral vector formulation comprises about 0.001% poloxamer 188 (also known as Pluronic F-68).

The viral vector formulation may comprise the viral vector in any suitable amount. Suitably, the viral vector formulation comprises the viral vector in an amount of about 1x10⁷ vg/ml or more, about 1x10⁶ vg/ml or more, about 1x10⁹ vg/ml or more, about 1x10¹⁰ vg/ml or more, about 1x10¹¹ vg/ml or more, about 1x10¹² vg/ml or more, or about 1x10¹³ vg/ml or more. Suitably, the viral vector formulation comprises the viral vector in an amount of about 1x10¹⁴ vg/ml or less or about 1x10¹³ vg/ml or less. Suitably, the viral vector formulation comprises the viral vector in an amount of from about 1x10⁷ vg/ml to about 1x10¹⁴ vg/ml. Suitably, the viral vector formulation comprises the viral vector in an amount of from about 1x10⁷ vg/ml to about 5x10¹³ vg/ml. Suitably, the viral vector formulation comprises the viral vector in an amount of about 1x10⁷ vg/ml to about 10x10⁷ vg/ml, about 1x10⁶ vg/ml to about 10x10⁸ vg/ml, about 1x10⁹ vg/ml to about 10x10⁹ vg/ml, about 1x10¹⁰ vg/ml to about 10x10¹⁰ vg/ml, about 1x10¹¹ vg/ml to about 10x10¹¹ vg/ml, about 1x10¹² vg/ml to about 10x10¹² vg/ml, or about 1x10¹³ vg/ml to about 10x10¹³ vg/ml.

In some embodiments, the viral vector formulation comprises the viral vector in an amount of from about 1x10¹⁰ vg/ml to about 1x10¹³ vg/ml. In some embodiments, the viral vector formulation comprises the viral vector in an amount of from about 1x10¹⁰ vg/ml to about 1x10¹² vg/ml.

The viral vector formulation may be any suitable volume. Suitably, the viral vector formulation has a volume of from about 5 ml to about 50 ml, from about 5 ml to about 25 ml, or from about 10 ml to about 25 ml. In some embodiments, the viral vector formulation has a volume of about 1 ml, about 2 ml, about 3 ml, about 4 ml, about 5 ml, about 6 ml, about 7 ml, about 8 ml, about 9 ml, about 10 ml, about 11 ml, about 12 ml, about 13 ml, about 14 ml, about 15 ml, about 16 ml, about 17 ml, about 18 ml, about 19 ml, about 20 ml, about 21 ml, about 22 ml, about 23 ml, about 24 ml, about 25 ml, about 26 ml, about 27 ml, about 28 ml, about 29 ml, or about 30 ml.

The viral vector formulation may further comprise one or more other therapeutic agents

### Viral vector

The viral vector of the invention is preferably an adeno-associated viral (AAV) vector, although it is contemplated that other viral vectors may be used. Other suitable viral vectors may include a lentiviral vector, a retroviral vector, an adenoviral vector, a herpes simplex viral vector, an alphaviral vector, a flaviviral vector, a rhabdoviral vector, a measles viral vector, a Newcastle disease viral vector, a poxviral vector, and a picornaviral vector.

The viral vector of the present invention may be in the form of a viral vector particle. Methods of preparing and modifying viral vectors and viral vector particles, such as those derived from AAV, are well known in the art. Suitable methods are described in Ayuso, E., et al., 2010. Current gene therapy, 10(6), pp.423-436, Merten, O.W., et al., 2016. Molecular Therapy-Methods & Clinical Development, 3, p.16017; and Nadeau, I. and Kamen, A., 2003. Biotechnology advances, 20(7-8), pp.475-489.

The viral vector of the present invention is preferably capable of transducing kidney cells. Suitably, the viral vector of the present invention is capable of specifically transducing kidney cells. The viral vector of the present invention is preferably capable of transducing glomerular cells. Suitably, the viral vector of the present invention is capable of specifically transducing glomerular cells. The viral vector of the present invention is preferably capable of transducing podocytes. Preferably, the viral vector of the present invention is capable of specifically transducing podocytes.

### Adeno-associated virus (AAV) vector

The viral vector of the present invention is preferably an adeno-associated viral (AAV) vector particle.

### AAV genome

The AAV vector particle may comprise an AAV genome or a fragment or derivative thereof.

An AAV genome is a polynucleotide sequence, which may encode functions needed for production of an AAV particle. These functions include those operating in the replication and packaging cycle of AAV in a host cell, including encapsidation of the AAV genome into an AAV particle. Naturally occurring AAVs are replication-deficient and rely on the provision of helper functions in trans for completion of a replication and packaging cycle. Accordingly, the AAV genome of the AAV vector of the invention is typically replication-deficient.

The AAV genome may be in single-stranded form (ssAAV), either positive or negative-sense, or alternatively in double-stranded form (dsAAV). The use of a double-stranded form allows bypass of the DNA replication step in the target cell and so can accelerate transgene expression. The maximum packaging capacity of the single-stranded form is larger than the double-stranded form. Suitably, the AAV genome is in single-stranded form.

AAVs occurring in nature may be classified according to various biological systems. The AAV genome may be from any naturally derived serotype, isolate or clade of AAV.

AAV may be referred to in terms of their serotype. A serotype corresponds to a variant subspecies of AAV which, owing to its profile of expression of capsid surface antigens, has a distinctive reactivity which can be used to distinguish it from other variant subspecies. Typically, an AAV vector particle having a particular AAV serotype does not efficiently cross-react with neutralising antibodies specific for any other AAV serotype. AAV serotypes include AAV1, AAV2, AAV3, AAV4, AAV5, AAV6, AAV7, AAV8, AAV9, AAV10 and AAV11. In some embodiments, the AAV vector of the invention may be an AAV3B, LK03, AAV9, or AAV8 serotype. In some embodiments, the AAV vector of the invention may be an AAV3B, LK03, or AAV9 serotype.

AAV may also be referred to in terms of clades or clones. This refers to the phylogenetic relationship of naturally derived AAVs, and typically to a phylogenetic group of AAVs which can be traced back to a common ancestor, and includes all descendants thereof. Additionally, AAVs may be referred to in terms of a specific isolate, i.e. a genetic isolate of a specific AAV found in nature. The term genetic isolate describes a population of AAVs which has undergone limited genetic mixing with other naturally occurring AAVs, thereby defining a recognisably distinct population at a genetic level.

Typically, the AAV genome of a naturally derived serotype, isolate or clade of AAV comprises at least one inverted terminal repeat sequence (ITR). An ITR sequence acts in cis to provide a functional origin of replication and allows for integration and excision of the vector from the genome of a cell. ITRs may be the only sequences required in cis next to the therapeutic gene.

The AAV genome may also comprise packaging genes, such as rep and/or cap genes which encode packaging functions for an AAV particle. A promoter may be operably linked to each of the packaging genes. Specific examples of such promoters include the p5, p19 and p40 promoters. For example, the p5 and p19 promoters are generally used to express the rep gene, while the p40 promoter is generally used to express the cap gene. The rep gene encodes one or more of the proteins Rep78, Rep68, Rep52 and Rep40 or variants thereof. The cap gene encodes one or more capsid proteins such as VP1, VP2 and VP3 or variants thereof. These proteins make up the capsid of an AAV particle, which determines the AAV serotype. VP1, VP2, and VP3 may be produced by alternate mRNA splicing (Trempe, J.P. and Carter, B.J., 1988. Journal of virology, 62(9), pp.3356-3363). Thus, VP1, VP2 and VP3 may have identical sequences, but wherein VP2 is truncated at the N-terminus relative to VP1, and VP3 is truncated at the N-terminus relative to VP2.

The AAV genome may be the full genome of a naturally occurring AAV. For example, a vector comprising a full AAV genome may be used to prepare an AAV vector.

Preferably, the AAV genome is derivatised for the purpose of administration to patients. Such derivatisation is standard in the art and the invention encompasses the use of any known derivative of an AAV genome, and derivatives which could be generated by applying techniques known in the art. The AAV genome may be a derivative of any naturally occurring AAV. Suitably, the AAV genome is a derivative of AAV1, AAV2, AAV3, AAV4, AAV5, AAV6, AAV7, AAV8, AAV9, AAV10, or AAV11. Suitably, the AAV genome is a derivative of AAV2.

Derivatives of an AAV genome include any truncated or modified forms of an AAV genome which allow for expression of a transgene from an AAV vector of the invention *in vivo.* Typically, it is possible to truncate the AAV genome significantly to include minimal viral sequence yet retain the above function. This is preferred for safety reasons to reduce the risk of recombination of the vector with wild-type virus, and also to avoid triggering a cellular immune response by the presence of viral gene proteins in the target cell.

Typically, a derivative will include at least one inverted terminal repeat sequence (ITR), preferably more than one ITR, such as two ITRs or more. One or more of the ITRs may be derived from AAV genomes having different serotypes, or may be a chimeric or mutant ITR.

A preferred mutant ITR is one having a deletion of a trs (terminal resolution site). This deletion allows for continued replication of the genome to generate a single-stranded genome which contains both coding and complementary sequences, i.e. a self-complementary AAV (scAAV) genome. This allows for bypass of DNA replication in the target cell, and so enables accelerated transgene expression. However, the maximum packaging capacity of a scAAV is reduced. Suitably, the AAV genome is not a scAAV genome.

The AAV genome may comprise one or more ITR sequences from any naturally derived serotype, isolate or clade of AAV or a variant thereof. The AAV genome may comprise at least one, such as two, AAV1, AAV2, AAV3, AAV4, AAV5, AAV6, AAV7, AAV8, AAV9, AAV10, or AAV11 ITRs, or variants thereof. Suitably, the AAV genome may comprise at least one, such as two, AAV2 ITRs.

The inclusion of one or more ITRs is preferred to aid concatamer formation of the AAV vector in the nucleus of a host cell, for example following the conversion of single-stranded vector DNA into double-stranded DNA by the action of host cell DNA polymerases. The formation of such episomal concatamers protects the AAV vector during the life of the host cell, thereby allowing for prolonged expression of the transgene in vivo.

Suitably, ITR elements will be the only sequences retained from the native AAV genome in the derivative. A derivative will preferably not include the rep and/or cap genes of the native genome and any other sequences of the native genome. This is preferred for the reasons described above, and also to reduce the possibility of integration of the vector into the host cell genome. Additionally, reducing the size of the AAV genome allows for increased flexibility in incorporating other sequence elements (such as regulatory elements) within the vector in addition to the transgene.

The following portions could therefore be removed in a derivative of the invention: one inverted terminal repeat (ITR) sequence, the replication (rep) and capsid (cap) genes. However, derivatives may additionally include one or more rep and/or cap genes or other viral sequences of an AAV genome. Naturally occurring AAV integrates with a high frequency at a specific site on human chromosome 19, and shows a negligible frequency of random integration, such that retention of an integrative capacity in the AAV vector may be tolerated in a therapeutic setting.

The invention additionally encompasses the provision of sequences of an AAV genome in a different order and configuration to that of a native AAV genome. The invention also encompasses the replacement of one or more AAV sequences or genes with sequences from another virus or with chimeric genes composed of sequences from more than one virus. Such chimeric genes may be composed of sequences from two or more related viral proteins of different viral species.

### AAV serotype and capsid proteins

The AAV vector particle may be encapsidated by capsid proteins. The serotype may facilitate the transduction of glomerular cells (e.g. podocytes), for example specific transduction of glomerular cells (e.g. podocytes).

The AAV vector particle may be a kidney-specific vector particle. Preferably, the AAV vector particle is a glomerular-specific (e.g. podocyte-specific) vector particle. The AAV vector particle may be encapsidated by a glomerular-specific (e.g. podocyte-specific) capsid. The AAV vector particle may comprise a glomerular-specific (e.g. podocyte-specific) capsid protein.

Suitably, the AAV vector particles may be transcapsidated forms wherein an AAV genome or derivative having an ITR of one serotype is packaged in the capsid of a different serotype. The AAV vector particle also includes mosaic forms wherein a mixture of unmodified capsid proteins from two or more different serotypes makes up the viral capsid. The AAV vector particle also includes chemically modified forms bearing ligands adsorbed to the capsid surface. For example, such ligands may include antibodies for targeting a particular cell surface receptor.

Where a derivative comprises capsid proteins i.e. VP1, VP2 and/or VP3, the derivative may be a chimeric, shuffled or capsid-modified derivative of one or more naturally occurring AAVs. In particular, the invention encompasses the provision of capsid protein sequences from different serotypes, clades, clones, or isolates of AAV within the same vector (i.e. a pseudotyped vector). The AAV vector may be in the form of a pseudotyped AAV vector particle.

Chimeric, shuffled or capsid-modified derivatives will be typically selected to provide one or more desired functionalities for the AAV vector. Thus, these derivatives may display increased efficiency of gene delivery, decreased immunogenicity (humoral or cellular), an altered tropism range and/or improved targeting of podocytes compared to an AAV vector comprising a naturally occurring AAV genome. Increased efficiency of gene delivery may be effected by improved receptor or co-receptor binding at the cell surface, improved internalisation, improved trafficking within the cell and into the nucleus, improved uncoating of the viral particle and improved conversion of a single-stranded genome to double-stranded form. Increased efficiency may also relate to an altered tropism range or targeting of podocytes, such that the vector dose is not diluted by administration to tissues where it is not needed.

Chimeric capsid proteins include those generated by recombination between two or more capsid coding sequences of naturally occurring AAV serotypes. This may be performed for example by a marker rescue approach in which non-infectious capsid sequences of one serotype are co-transfected with capsid sequences of a different serotype, and directed selection is used to select for capsid sequences having desired properties. The capsid sequences of the different serotypes can be altered by homologous recombination within the cell to produce novel chimeric capsid proteins.

Chimeric capsid proteins also include those generated by engineering of capsid protein sequences to transfer specific capsid protein domains, surface loops or specific amino acid residues between two or more capsid proteins, for example between two or more capsid proteins of different serotypes.

Shuffled or chimeric capsid proteins may also be generated by DNA shuffling or by error-prone PCR. Hybrid AAV capsid genes can be created by randomly fragmenting the sequences of related AAV genes e.g. those encoding capsid proteins of multiple different serotypes and then subsequently reassembling the fragments in a self-priming polymerase reaction, which may also cause crossovers in regions of sequence homology. A library of hybrid AAV genes created in this way by shuffling the capsid genes of several serotypes can be screened to identify viral clones having a desired functionality. Similarly, error prone PCR may be used to randomly mutate AAV capsid genes to create a diverse library of variants which may then be selected for a desired property.

The sequences of the capsid genes may also be genetically modified to introduce specific deletions, substitutions or insertions with respect to the native wild-type sequence. In particular, capsid genes may be modified by the insertion of a sequence of an unrelated protein or peptide within an open reading frame of a capsid coding sequence, or at the N- and/or C-terminus of a capsid coding sequence. The unrelated protein or peptide may advantageously be one which acts as a ligand for a particular cell type, thereby conferring improved binding to a target cell or improving the specificity of targeting of the vector to a particular cell population. The unrelated protein may also be one which assists purification of the viral particle as part of the production process, i.e. an epitope or affinity tag. The site of insertion will typically be selected so as not to interfere with other functions of the viral particle e.g. internalisation, trafficking of the viral particle.

The capsid protein may be an artificial or mutant capsid protein. The term "artificial capsid" as used herein means that the capsid particle comprises an amino acid sequence which does not occur in nature or which comprises an amino acid sequence which has been engineered (e.g. modified) from a naturally occurring capsid amino acid sequence. In other words, the artificial capsid protein comprises a mutation or a variation in the amino acid sequence compared to the sequence of the parent capsid from which it is derived where the artificial capsid amino acid sequence and the parent capsid amino acid sequences are aligned.

The capsid protein may comprise a mutation or modification relative to the wild type capsid protein which improves the ability to transduce podocytes relative to an unmodified or wild type viral particle. Improved ability to transduce podocytes may be measured for example by measuring the expression of a transgene, e.g. GFP, carried by the AAV vector particle, wherein expression of the transgene in podocytes correlates with the ability of the AAV vector particle to transduce podocytes.

The AAV vector particle may be an AAV3B, LK03, AAV9, or AAV8 vector particle. The present inventors have shown that AAV vector particles with AAV3B, LK03, AAV9 and AAV8 serotypes can transduce podocytes. Preferably, the AAV vector particle is an AAV3B vector particle or an LK03 vector particle. More preferably, the AAV vector particle is an LK03 vector particle.

The AAV vector particle may comprise an AAV3B, LK03, AAV9, or AAV8 capsid protein. Preferably, the AAV vector particle comprises an AAV3B capsid protein or an LK03 capsid protein. More preferably, the AAV vector particle comprises an LK03 capsid protein.

The AAV vector particle may comprise AAV3B, LK03, AAV9, or AAV8 capsid proteins VP1, VP2 and VP3. Preferably, the AAV vector particle comprises AAV3B or LK03 capsid proteins VP1, VP2 and VP3. More preferably, the AAV vector particle comprises LK03 capsid proteins VP1, VP2 and VP3.

The AAV vector particle may comprise one or more AAV2 ITR sequences and AAV3B capsid proteins, LK03 capsid proteins, AAV9 capsid proteins, or AAV8 capsid proteins. Preferably, the AAV vector particle comprises one or more AAV2 ITR sequences and AAV3B or LK03 capsid proteins. More preferably, the AAV vector particle comprises one or more AAV2 ITR sequences and LK03 capsid proteins.

The AAV vector particle may have an AAV2 genome and AAV3B capsid proteins (AAV2/3B), an AAV2 genome and LK03 capsid proteins, an AAV2 genome and AAV9 capsid proteins (AAV2/9), or an AAV2 genome and AAV8 capsid proteins (AAV2/8). Preferably, the AAV vector particle comprises an AAV2 genome and AAV3B or LK03 capsid proteins. More preferably, the AAV vector particle comprises an AAV2 genome and LK03 capsid proteins.

The nomenclature AAVX/Y may denote a pseudotyped AAV, for example where the ITR sequences are from AAVX and flank a cassette harbouring a payload which is encapsidated into serotype AAVY (i.e. with AAVY capsid proteins).

### AAV3B serotype

The AAV vector particle may comprise an AAV3B capsid protein. Suitably, the AAV vector particle may be encapsidated by AAV3B capsid proteins.

Two distinct AAV3 isolates (AAV3A and AAV3B) have been cloned. In comparison with vectors based on other AAV serotypes, it is thought that AAV3 vectors inefficiently transduce most cell types. However, AAV3B may efficiently transduce podocytes. AA3B has been described in Rutledge, E.A., et al., 1998. Journal of virology, 72(1), pp.309-319.

The AAV vector particle may comprise an AAV3B VP1 capsid protein, an AAV3B VP2 capsid protein, and/or an AAV3B VP3 capsid protein. Suitably, the AAV vector particle may be encapsidated by AAV3B VP1 capsid proteins, AAV3B VP2 capsid proteins, and/or AAV3B VP3 capsid proteins. Suitably, the AAV vector particle may be encapsidated by AAV3B VP1, VP2, and VP3 capsid proteins.

Suitably, the AAV3B VP1 capsid protein may comprise or consist of the amino acid sequence shown as SEQ ID NO: 1, or a variant which is at least 90% identical to SEQ ID NO: 1.

MAADGYLPDWLEDNLSEGIREWWALKPGVPQPKANQQHQDNRRGLVLPGYKYLGPGNGLDKGEPVNEA
DAAALEHDKAYDQQLKAGDNPYLKYNHADAEFQERLQEDTSFGGNLGRAVFQAKKRILEPLGLVEEAA
KTAPGKKRPVDQSPQEPDSSSGVGKSGKQPARKRLNFGQTGDSESVPDPQPLGEPPAAPTSLGSNTMA
SGGGAPMADNNEGADGVGNSSGNWHCDSQWLGDRVITTSTRTWALPTYNNHLYKQISSQSGASNDNHY
FGYSTPWGYFDFNRFHCHFSPRDWQRLINNNWGFRPKKLSFKLFNIQVKEVTQNDGTTTIANNLTSTV
QVFTDSEYQLPYVLGSAHQGCLPPFPADVFMVPQYGYLTLNNGSQAVGRSSFYCLEYFPSQMLRTGNN
FQFSYTFEDVPFHSSYAHSQSLDRLMNPLIDQYLYYLNRTQGTTSGTTNQSRLLFSQAGPQSMSLQAR
NWLPGPCYRQQRLSKTANDNNNSNFPWTAASKYHLNGRDSLVNPGPAMASHKDDEEKFFPMHGNLIFG
KEGTTASNAELDNVMITDEEEIRTTNPVATEQYGTVANNLQSSNTAPTTRTVNDQGALPGMVWQDRDV
YLQGPIWAKIPHTDGHFHPSPLMGGFGLKHPPPQIMIKNTPVPANPPTTFSPAKFASFITQYSTGQVS
VEIEWELQKENSKRWNPEIQYTSNYNKSVNVDFTVDTNGVYSEPRPIGTRYLTRN

### Example AAV3B VP1 capsid protein (SEQ ID NO: 1)

Suitably, the variant may be at least 95%, at least 96%, at least 97%, at least 98% or at least 99% identical to SEQ ID NO: 1.

Suitably, the AAV3B VP2 and VP3 capsid proteins may be N-terminal truncations of SEQ ID NO: 1, or N-terminal truncations of a variant which is at least 90% identical, at least 95%, at least 96%, at least 97%, at least 98% or at least 99% identical to SEQ ID NO: 1.

### LK03 serotype

The AAV vector particle may comprise an LK03 capsid protein. Suitably, the AAV vector particle may be encapsidated by LK03 capsid proteins.

The AAV-LK03 cap sequence consists of fragments from seven different wild-type serotypes (AAV1, 2, 3B, 4, 6, 8, 9) and is described in Lisowski, L., et al., 2014. Nature, 506(7488), pp.382-386. The present inventors have demonstrated that AAV-LK03 vectors can achieve high transduction of close to 100% in human podocytes *in vitro.*

The AAV vector particle may comprise an LK03 VP1 capsid protein, an LK03 VP2 capsid protein, and/or an LK03 VP3 capsid protein. Suitably, the AAV vector particle may be encapsidated by LK03 VP1 capsid proteins, LK03 VP2 capsid proteins, and/or LK03 VP3 capsid proteins. Suitably, the AAV vector particle may be encapsidated by LK03 VP1, VP2, and VP3 capsid proteins.

Suitably, the LK03 VP1 capsid protein may comprise or consist of the amino acid sequence shown as SEQ ID NO: 2, or a variant which is at least 90% identical to SEQ ID NO: 2.

MAADGYLPDWLEDNLSEGIREWWALQPGAPKPKANQQHQDNARGLVLPGYKYLGPGNGLDKGEPVNAA
DAAALEHDKAYDQQLKAGDNPYLKYNHADAEFQERLKEDTSFGGNLGRAVFQAKKRLLEPLGLVEEAA
KTAPGKKRPVDQSPQEPDSSSGVGKSGKQPARKRLNFGQTGDSESVPDPQPLGEPPAAPTSLGSNTMA
SGGGAPMADNNEGADGVGNSSGNWHCDSQWLGDRVITTSTRTWALPTYNNHLYKQISSQSGASNDNHY
FGYSTPWGYFDFNRFHCHFSPRDWQRLINNNWGFRPKKLSFKLFNIQVKEVTQNDGTTTIANNLTSTV
QVFTDSEYQLPYVLGSAHQGCLPPFPADVFMVPQYGYLTLNNGSQAVGRSSFYCLEYFPSQMLRTGNN
FQFSYTFEDVPFHSSYAHSQSLDRLMNPLIDQYLYYLNRTQGTTSGTTNQSRLLFSQAGPQSMSLQAR
NWLPGPCYRQQRLSKTANDNNNSNFPWTAASKYHLNGRDSLVNPGPAMASHKDDEEKFFPMHGNLIFG
KEGTTASNAELDNVMITDEEEIRTTNPVATEQYGTVANNLQSSNTAPTTRTVNDQGALPGMVWQDRDV
YLQGPIWAKIPHTDGHFHPSPLMGGFGLKHPPPQIMIKNTPVPANPPTTFSPAKFASFITQYSTGQVS
VEIEWELQKENSKRWNPEIQYTSNYNKSVNVDFTVDTNGVYSEPRPIGTRYLTRPL

### Example LK03 VP1 capsid protein (SEQ ID NO: 2)

Suitably, the variant may be at least 95%, at least 96%, at least 97%, at least 98% or at least 99% identical to SEQ ID NO: 2.

Suitably, the LK03 VP2 and VP3 capsid proteins may be N-terminal truncations of SEQ ID NO: 2, or N-terminal truncations of a variant which is at least 90% identical, at least 95%, at least 96%, at least 97%, at least 98% or at least 99% identical to SEQ ID NO: 2.

### AAV9 serotype

The AAV vector particle may comprise an AAV9 capsid protein. Suitably, the AAV vector particle may be encapsidated by AAV9 capsid proteins.

The present inventors have demonstrated that AAV9 vectors can achieve high transduction in human podocytes *in vitro.*

The AAV vector particle may comprise an AAV9 VP1 capsid protein, an AAV9 VP2 capsid protein, and/or an AAV9 VP3 capsid protein. Suitably, the AAV vector particle may be encapsidated by AAV9 VP1 capsid proteins, AAV9 VP2 capsid proteins, and/or AAV9 VP3 capsid proteins. Suitably, the AAV vector particle may be encapsidated by AAV9 VP1, VP2, and VP3 capsid proteins.

Suitably, the AAV9 VP1 capsid protein may comprise or consist of the amino acid sequence shown as SEQ ID NO: 3, or a variant which is at least 90% identical to SEQ ID NO: 3.

MAADGYLPDWLEDNLSEGIREWWALKPGAPQPKANQQHQDNARGLVLPGYKYLGPGNGLDKGEPVNAA
DAAALEHDKAYDQQLKAGDNPYLKYNHADAEFQERLKEDTSFGGNLGRAVFQAKKRLLEPLGLVEEAA
KTAPGKKRPVEQSPQEPDSSAGIGKSGAQPAKKRLNFGQTGDTESVPDPQPIGEPPAAPSGVGSLTMA
SGGGAPVADNNEGADGVGSSSGNWHCDSQWLGDRVITTSTRTWALPTYNNHLYKQISNSTSGGSSNDN
AYFGYSTPWGYFDFNRFHCHFSPRDWQRLINNNWGFRPKRLNFKLFNIQVKEVTDNNGVKTIANNLTS
TVQVFTDSDYQLPYVLGSAHEGCLPPFPADVFMIPQYGYLTLNDGSQAVGRSSFYCLEYFPSQMLRTG
NNFQFSYEFENVPFHSSYAHSQSLDRLMNPLIDQYLYYLSKTINGSGQNQQTLKFSVAGPSNMAVQGR
NYIPGPSYRQQRVSTTVTQNNNSEFAWPGASSWALNGRNSLMNPGPAMASHKEGEDRFFPLSGSLIFG
KQGTGRDNVDADKVMITNEEEIKTTNPVATESYGQVATNHQSAQAQAQTGWVQNQGILPGMVWQDRDV
YLQGPIWAKIPHTDGNFHPSPLMGGFGMKHPPPQILIKNTPVPADPPTAFNKDKLNSFITQYSTGQVS
VEIEWELQKENSKRWNPEIQYTSNYYKSNNVEFAVNTEGVYSEPRPIGTRYLTRNL

### Exemplary AAV9 VP1 capsid protein (SEQ ID NO: 3)

Suitably, the variant may be at least 95%, at least 96%, at least 97%, at least 98% or at least 99% identical to SEQ ID NO: 3.

Suitably, the AAV9 VP2 and VP3 capsid proteins may be N-terminal truncations of SEQ ID NO: 3, or N-terminal truncations of a variant which is at least 90% identical, at least 95%, at least 96%, at least 97%, at least 98% or at least 99% identical to SEQ ID NO: 3.

### Other viral vectors

### Retroviral and lentiviral vectors

The viral vector of the present invention may be a retroviral vector or a lentiviral vector.

A retroviral vector may be derived from or may be derivable from any suitable retrovirus. A large number of different retroviruses have been identified. Examples include murine leukaemia virus (MLV), human T-cell leukaemia virus (HTLV), mouse mammary tumour virus (MMTV), Rous sarcoma virus (RSV), Fujinami sarcoma virus (FuSV), Moloney murine leukaemia virus (Mo-MLV), FBR murine osteosarcoma virus (FBR MSV), Moloney murine sarcoma virus (Mo-MSV), Abelson murine leukaemia virus (A-MLV), avian myelocytomatosis virus-29 (MC29) and avian erythroblastosis virus (AEV).

Retroviruses may be broadly divided into two categories, "simple" and "complex". Retroviruses may be even further divided into seven groups. Five of these groups represent retroviruses with oncogenic potential. The remaining two groups are the lentiviruses and the spumaviruses.

The basic structure of retrovirus and lentivirus genomes share many common features such as a 5' LTR and a 3' LTR. Between or within these are located a packaging signal to enable the genome to be packaged, a primer binding site, integration sites to enable integration into a host cell genome, and gag, pol and env genes encoding the packaging components - these are polypeptides required for the assembly of viral particles. Lentiviruses have additional features, such as rev and RRE sequences in HIV, which enable the efficient export of RNA transcripts of the integrated provirus from the nucleus to the cytoplasm of an infected target cell.

In the provirus, these genes are flanked at both ends by regions called long terminal repeats (LTRs). The LTRs are responsible for proviral integration and transcription. LTRs also serve as enhancer-promoter sequences and can control the expression of the viral genes.

The LTRs themselves are identical sequences that can be divided into three elements: U3, R and U5. U3 is derived from the sequence unique to the 3' end of the RNA. R is derived from a sequence repeated at both ends of the RNA. U5 is derived from the sequence unique to the 5' end of the RNA. The sizes of the three elements can vary considerably among different retroviruses.

In a defective retroviral vector genome gag, pol and env may be absent or not functional.

In a typical retroviral vector, at least part of one or more protein coding regions essential for replication may be removed from the virus. This makes the viral vector replication-defective. Portions of the viral genome may also be replaced by a library encoding candidate modulating moieties operably linked to a regulatory control region and a reporter moiety in the vector genome in order to generate a vector comprising candidate modulating moieties which is capable of transducing a target host cell and/or integrating its genome into a host genome.

Lentivirus vectors are part of the larger group of retroviral vectors. In brief, lentiviruses can be divided into primate and non-primate groups. Examples of primate lentiviruses include but are not limited to human immunodeficiency virus (HIV), the causative agent of human acquired immunodeficiency syndrome (AIDS); and simian immunodeficiency virus (SIV). Examples of non-primate lentiviruses include the prototype "slow virus" visna/maedi virus (VMV), as well as the related caprine arthritis-encephalitis virus (CAEV), equine infectious anaemia virus (EIAV), and the more recently described feline immunodeficiency virus (FIV) and bovine immunodeficiency virus (BIV).

The lentivirus family differs from retroviruses in that lentiviruses have the capability to infect both dividing and non-dividing cells. In contrast, other retroviruses, such as MLV, are unable to infect non-dividing or slowly dividing cells such as those that make up, for example, muscle, brain, lung and liver tissue.

A lentiviral vector, as used herein, is a vector which comprises at least one component part derivable from a lentivirus. Preferably, that component part is involved in the biological mechanisms by which the vector infects cells, expresses genes or is replicated.

The lentiviral vector may be a "primate" vector. The lentiviral vector may be a "non-primate" vector (i.e. derived from a virus which does not primarily infect primates, especially humans). Examples of non-primate lentiviruses may be any member of the family of lentiviridae which does not naturally infect a primate.

As examples of lentivirus-based vectors, HIV-1- and HIV-2-based vectors are described below.

The HIV-1 vector contains cis-acting elements that are also found in simple retroviruses. It has been shown that sequences that extend into the gag open reading frame are important for packaging of HIV-1. Therefore, HIV-1 vectors often contain the relevant portion of gag in which the translational initiation codon has been mutated. In addition, most HIV-1 vectors also contain a portion of the env gene that includes the RRE. Rev binds to RRE, which permits the transport of full-length or singly spliced mRNAs from the nucleus to the cytoplasm. In the absence of Rev and/or RRE, full-length HIV-1 RNAs accumulate in the nucleus. Alternatively, a constitutive transport element from certain simple retroviruses such as Mason-Pfizer monkey virus can be used to relieve the requirement for Rev and RRE. Efficient transcription from the HIV-1 LTR promoter requires the viral protein Tat.

Most HIV-2-based vectors are structurally very similar to HIV-1 vectors. Similar to HIV-1-based vectors, HIV-2 vectors also require RRE for efficient transport of the full-length or singly spliced viral RNAs.

Preferably, the viral vector used in the present invention has a minimal viral genome.

By "minimal viral genome" it is to be understood that the viral vector has been manipulated so as to remove the non-essential elements and to retain the essential elements in order to provide the required functionality to infect, transduce and deliver a nucleotide sequence of interest to a target host cell. Further details of this strategy can be found in WO 1998/017815.

Preferably, the plasmid vector used to produce the viral genome within a host cell/packaging cell will have sufficient lentiviral genetic information to allow packaging of an RNA genome, in the presence of packaging components, into a viral particle which is capable of infecting a target cell, but is incapable of independent replication to produce infectious viral particles within the final target cell. Preferably, the vector lacks a functional gag-pol and/or env gene and/or other genes essential for replication.

However, the plasmid vector used to produce the viral genome within a host cell/packaging cell will also include transcriptional regulatory control sequences operably linked to the lentiviral genome to direct transcription of the genome in a host cell/packaging cell. These regulatory sequences may be the natural sequences associated with the transcribed viral sequence (i.e. the 5' U3 region), or they may be a heterologous promoter, such as another viral promoter (e.g. the CMV promoter).

The vectors may be self-inactivating (SIN) vectors in which the viral enhancer and promoter sequences have been deleted. SIN vectors can be generated and transduce non-dividing cells in vivo with an efficacy similar to that of wild-type vectors. The transcriptional inactivation of the long terminal repeat (LTR) in the SIN provirus should prevent mobilisation by replication-competent virus. This should also enable the regulated expression of genes from internal promoters by eliminating any cis-acting effects of the LTR.

The vectors may be integration-defective. Integration defective lentiviral vectors (IDLVs) can be produced, for example, either by packaging the vector with catalytically inactive integrase (such as an HIV integrase bearing the D64V mutation in the catalytic site) or by modifying or deleting essential att sequences from the vector LTR, or by a combination of the above.

### Adenoviral vectors

The viral vector of the present invention may be an adenoviral vector.

The adenovirus is a double-stranded, linear DNA virus that does not go through an RNA intermediate. There are over 50 different human serotypes of adenovirus divided into 6 subgroups based on the genetic sequence homology. The natural targets of adenovirus are the respiratory and gastrointestinal epithelia, generally giving rise to only mild symptoms. Serotypes 2 and 5 (with 95% sequence homology) are most commonly used in adenoviral vector systems and are normally associated with upper respiratory tract infections in the young.

Adenoviruses have been used as vectors for gene therapy and for expression of heterologous genes. The large (36 kb) genome can accommodate up to 8 kb of foreign insert DNA and is able to replicate efficiently in complementing cell lines to produce very high titres of up to 10¹². Adenovirus is thus one of the best systems to study the expression of genes in primary non-replicative cells.

The expression of viral or foreign genes from the adenovirus genome does not require a replicating cell. Adenoviral vectors enter cells by receptor mediated endocytosis. Once inside the cell, adenovirus vectors rarely integrate into the host chromosome. Instead, they function episomally (independently from the host genome) as a linear genome in the host nucleus. Hence, the use of recombinant adenovirus alleviates the problems associated with random integration into the host genome.

### Herpes simplex viral vector

The viral vector of the present invention may be a herpes simplex viral vector.

Herpes simplex virus (HSV) is a neurotropic DNA virus with favorable properties as a gene delivery vector. HSV is highly infectious, so HSV vectors are efficient vehicles for the delivery of exogenous genetic material to cells. Viral replication is readily disrupted by null mutations in immediate early genes that in vitro can be complemented in trans enabling straightforward production of high-titre pure preparations of non-pathogenic vector. The genome is large (152 Kb) and many of the viral genes are dispensable for replication in vitro, allowing their replacement with large or multiple transgenes. Latent infection with wild-type virus results in episomal viral persistence in sensory neuronal nuclei for the duration of the host lifetime. The vectors are non-pathogenic, unable to reactivate and persist long-term. The latency active promoter complex can be exploited in vector design to achieve long-term stable transgene expression in the nervous system. HSV vectors transduce a broad range of tissues because of the wide expression pattern of the cellular receptors recognized by the virus. Increasing understanding of the processes involved in cellular entry has allowed targeting the tropism of HSV vectors.

### Other viral vectors

Other suitable viral vectors include those described in Lundstrom, K., 2018. Diseases, 6(2), p.42.

The viral vector of the present invention may be an alphaviral vector. The viral vector of the present invention may be a flaviviral vector.

Self-amplifying ssRNA viruses comprise of alphaviruses (e.g. Semliki Forest virus, Sindbis virus, Venezuelan equine encephalitis virus, and M1) and flaviviruses (e.g. Kunjin virus, West Nile virus, and Dengue virus) possessing a genome of positive polarity. Alphaviruses have been mainly applied in preclinical gene therapy studies for cancer treatment. Alphavirus vectors can be delivered in the form of naked RNA, layered plasmid DNA vectors and recombinant replication-deficient or -proficient particles.

The viral vector of the present invention may be a rhabdoviral vector. The viral vector of the present invention may be a measles viral vector.

Rhabdoviruses (e.g. rabies and vesicular stomatitis virus) and measles viruses carry negative strand genomes. Among rhabdoviruses, recombinant vesicular stomatitis virus (VSV) has been applied for preclinical gene therapy studies. Measles viruses (e.g. MV-Edm) have found a number of gene therapy applications.

The viral vector of the present invention may be a Newcastle disease viral vector.

The ssRNA paramyxovirus Newcastle disease virus (NDV) replicates specifically in tumour cells and has therefore been frequently applied for cancer gene therapy.

The viral vector of the present invention may be a poxviral vector.

The characteristic feature of poxviruses is their dsDNA genome, which can generously accommodate more than 30 kb of foreign DNA. Poxviruses have found several applications as gene therapy vectors. For instance, vaccinia virus vectors have demonstrated potential for treatment of cancer. Vaccinia virus is large enveloped poxvirus that has an approximately 190 kb linear, double-stranded DNA genome. Vaccinia virus can accommodate up to approximately 25 kb of foreign DNA, which also makes it useful for the delivery of large genes. A number of attenuated vaccinia virus strains are known in the art that are suitable for gene therapy applications, for example the MVA and NYVAC strains.

The viral vector of the present invention may be a picornaviral vector.

Picornoviruses are non-enveloped ssRNA viruses. Coxsackieviruses belonging to Picornaviridae, have been applied as oncolytic vectors.

### Protein-coding sequence

The viral vector may comprise a protein-coding sequence.

The protein-coding sequence may encode any polypeptide of interest, such as a therapeutic protein. For example, the protein-coding sequence can encode any polypeptide associated with a glomerular disease. The protein-coding sequence may encode a polypeptide involved in a GBM-associated genetic glomerular disease, such as Alport Syndrome. The protein-coding sequence may encode a polypeptide involved in podocyte-associated genetic glomerular disease.

Suitably, the protein-coding sequence may encode a COL4A3, COL4A4, COL4A5, NPHS2, CFH, CFL, FHL-1, C1INH, C4BP, MASP2, C3, C5aR1, C5, C5a, CD55, CD35, CD46, CD59, vitronectin, clusterin, ADCK4, ALG1, ARHGAP24, ARGHDIA, CD151, CD2AP, COQ2, COQ6, DGKE, E2F3, EMP2, KANK2, LAGE3, LMNA, LMX1B, MAF B, NUP85, NUP93, NXF5, OSGEP, PAX2, PDSS2, PMM2, PODXL, SCARB2, SGPL1, Smad7, TP53RK, TPRKB, VDR, WDR73, WT1, ZMPSTE24, APOL1, NPHS1, TRPC6, NUP107, NUP133, NUP160, ACTN4, INF2, ANKFY1, ANLN, CRB2, ITGA3, KANK1, KANK4, MAGI2, MYO1E, OCRL, PTPRO, SMARCAL1, SYNPO, TBC1D8B, XPO5, TNS2, NLRP3, or VEGFC polypeptide.

Suitably, the protein-coding sequence encodes a polypeptide which has a length of 1450 amino acids or more, 1500 amino acids or more, 1550 amino acids or more, 1600 amino acids or more, or 1650 amino acids or more.

For a protein-coding polynucleotide, it will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides of the invention to reflect the codon usage of any particular host organism in which the polypeptides of the invention are to be expressed.

The protein-coding sequence may be codon-optimised. Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available. Codon usage tables are known in the art for mammalian cells (e.g. humans), as well as for a variety of other organisms.

The protein coding nucleotide sequences disclosed herein may comprise or lack stop codons at their 3' end. Thus, the present disclosure encompasses the SEQ ID NOs disclosed herein with the stop codons present or absent.

### COL4A3, COL4A4 and COL4A5 polypeptides

The protein-coding sequence may encode a COL4A3, COL4A4 or COL4A5 polypeptide, or a fragment or derivative thereof.

COL4A3, COL4A4 and COL4A5 proteins are approximately 170-185 kDa homologous polypeptides containing collagenous Gly-X-Y repeat sequences frequently interrupted by non-collagenous sequences and forming a triple helix repeat. Each polypeptide also contains a large globular non-collagenous domain at the carboxyl-terminal end.

Alport syndrome (AS) is caused by pathogenic variants in the COL4A3, COL4A4 and COL4A5 genes, which result in abnormalities of the collagen IV α345 network of basement membranes.

The COL4A3, COL4A4 or COL4A5 polypeptide or a fragment or derivative thereof may be capable of forming a collagen IV α345 network.

Approximately 200-300 amino acids may be removed from each of the COL4A3, COL4A4 and COL4A5 polypeptides to produce a truncated transgene suitable for a mini-gene approach. The amino acids may be removed from the triple helix repeat. Preferably the amino acids are not removed from the non-collagenous region.

In some embodiments the COL4A3, COL4A4 and COL4A5 polypeptides are full-length polypeptides.

Preferably, the COL4A3, COL4A4 or COL4A5 polypeptide is human. An example human COL4A3 is the COL4A3 having the UniProtKB accession number Q01955. An example human COL4A4 is the COL4A3 having the UniProtKB accession number P53420. An example human COL4A5 is the COL4A5 having the UniProtKB accession number P29400. Suitably, the COL4A3 peptide may comprise or consist of the polypeptide sequence shown as SEQ ID NO: 4, or a variant which is at least 70% identical to SEQ ID NO: 4. Suitably, the variant may be at least 75%, at least 80%, at least 85%, at least 90%, at least 95%, at least 96%, at least 97%, at least 98%, at least 99% identical to SEQ ID NO: 4.

Suitably, the COL4A4 peptide may comprise or consist of the polypeptide sequence shown as SEQ ID NO: 5, or a variant which is at least 70% identical to SEQ ID NO: 5. Suitably, the variant may be at least 75%, at least 80%, at least 85%, at least 90%, at least 95%, at least 96%, at least 97%, at least 98%, at least 99% identical to SEQ ID NO: 5.

Suitably, the COL4A5 peptide may comprise or consist of the polypeptide sequence shown as SEQ ID NO: 6, or a variant which is at least 70% identical to SEQ ID NO: 6. Suitably, the variant may be at least 75%, at least 80%, at least 85%, at least 90%, at least 95%, at least 96%, at least 97%, at least 98%, at least 99% identical to SEQ ID NO: 6.
Example COL4A3 amino acid sequence - Uniprot reference Q01955 (SEQ ID NO: 4)
Example COL4A4 amino acid sequence - Uniprot reference P53420 (SEQ ID NO: 5)
Example COL4A5 amino acid sequence - Uniprot reference P29400 (SEQ ID NO: 6)

The protein-coding sequence may comprise or consist of a COL4A3, COL4A4 or COL4A5 transgene.

An example transgene encoding COL4A3 is provided in NM_000091.5. An example transgene encoding COL4A4 is provided in NM_000092.5. An example transgene encoding COL4A5 is provided in NM_000495.5.

Suitably, the COL4A3 transgene may comprise or consist of the polynucleotide sequence shown as SEQ ID NO: 7, or a variant which is at least 70% identical to SEQ ID NO: 7. Suitably, the variant may be at least 75%, at least 80%, at least 85%, at least 90%, at least 95%, at least 96%, at least 97%, at least 98%, at least 99% identical to SEQ ID NO: 7.

Suitably, the COL4A4 transgene may comprise or consist of the polynucleotide sequence shown as SEQ ID NO: 8, or a variant which is at least 70% identical to SEQ ID NO: 8. Suitably, the variant may be at least 75%, at least 80%, at least 85%, at least 90%, at least 95%, at least 96%, at least 97%, at least 98%, at least 99% identical to SEQ ID NO: 8.

Suitably, the COL4A5 transgene may comprise or consist of the polynucleotide sequence shown as SEQ ID NO: 9, or a variant which is at least 70% identical to SEQ ID NO: 9. Suitably, the variant may be at least 75%, at least 80%, at least 85%, at least 90%, at least 95%, at least 96%, at least 97%, at least 98%, at least 99% identical to SEQ ID NO: 9. 
atgagcgcccggaccgcccccaggccgcaggtgctcctgctgccgctcctgctggtgctcctggcggc
ggcgcccgcagccagcaaggttgtgtctgtaaagacaaaggccagtgcttctgtgacgggccaaag
ggagaagggggagaaggctttcctggacccccggttctcctggccagaaaggattcacaggtcct
gaaggcttgcctggaccgcagggacccaagggctttccaggacttccaggactcacgggttccaaagg
tgtaaggggaataagtggattgccaggattttctggttctcctggacttccaggcaccccaggcaata
ccgggccttacggacttgtcggtgtaccaggatgcagtggttctaagggtgagcaggggtttccagga
ctcccagggacactgggctacccaggatcccgggtgctgctggtttgaaaggacaaaggtgctcc
tgctaaagaagaagatatagaacttgatgcaaaggcgaccccgggttgccaggggctccaggacccc
agggtttgccaggccctccaggttttcctggcctgttggcccacctggtcctccgggattctttggc
tttccaggagccatgggacctagaggacctaagggtcacatgggtgaaagagtgataggacataaagg
agagcggggtgtgaaagggttaacaggacccccggaccaccaggaacagttattgtgaccctaactg
gcccagataacagaacggacctcaaggggaaaaggagacaaggagcaatgggcgagcctggacct
cctggaccctcaggactgcctggagaatcatatggatctgaaaagggtgctcctggagaccctggcct
gcagggaaaacccggaaaagatggtgttcctggcttccctggaagtgagggagtcaagggcaacaggg
gtttccctgggttaatgggtgaagatggcattaaggacagaaaggggacattggccctccaggattt
cgtggtccaacagaatattatgacacataccaggaaaaggagatgaaggcactccaggcccaccagg
gcccagaggagctcgtggcccacaaggtcccagtggtcccccggagttcctggaagtcctggatcat
caaggcctggcctcagaggagcccctggatggccaggcctgaaaggaagtaaagggaacgaggccgc
ccaggaaaggatgccatggggactcctgggtccccaggttgtgctggttcaccaggtcttccaggatc
accgggacctccaggaccgccaggtgacatcgtttttcgcaagggtccacctggagatcacggactgc
caggctatctagggtctccaggaatcccaggagttgatgggcccaaaggagaaccaggcctcctgtgt
acacagtgcccttatatcccaggcctcccggtctcccaggattgccaggttacatggtgtaaaagg
aatcccaggaagacaaggcgcagctggcttgaaaggaagcccagggtccccaggaaatacaggtcttc
caggatttccaggtttcccaggtgcccagggtgacccaggacttaaaggagaaaaaggtgaaacactt
cagcctgaggggcaagtgggtgtcccaggtgacccggggctcagaggccaacctgggagaaaggctt
ggatggaattcctggaactccgggagtgaaaggattaccaggacctaaaggcgaactggctctgagtg
gtgagaaaggggaccaaggtcctccaggggatcctggctcccctgggtccccaggacctgcaggacca
gctggaccacctggctacggaccccaaggagaacctggtctccagggcacgcaaggagttcctggagc
cccggaccacccggagaagccggccctaggggagagctcagtgtttcaacaccagttccaggcccac
 
caggacctccagggccccctggccatcctggcccccaaggtccacctggtatccctggatccctgggg
aaatgtggagatcctggtcttccaggcctgatggtgaaccaggaattccaggaattggatttcctgg
gcctcctggacctaagggagaccaaggttttccaggtacaaaggatcactgggttgtcctggaaaaa
tgggagagcctgggttacctggaaagccaggcctcccaggagccaagggagaaccagcagtagccatg
cctggaggaccaggaacaccaggttttccaggagaaagaggcaattctggggaacatggagaaattgg
actccctggacttccaggtctccctggaactccaggaaatgaaggcttgatggaccacgaggagatc
cagggcagcctggaccacctggagaacaaggacccccaggaaggtgcatagagggtcccaggggagcc
caaggacttccaggcttaaatggattgaaaggcaacaaggcagaagaggtaaaacgggccaaaggg
agacccaggaattccaggcttggatagatcaggatttcctggagaaactggatcaccaggaattccag
gtcatcaaggtgaaatgggaccactgggtcaaagaggatatccaggaaatccgggaattttaggcca
ccaggtgaagatggagtgattgggatgatgggctttcctggagccattggccctccaggccccctgg
gaacccaggcacaccagggcagagggggagccctggaattccaggagtaaaggccagagaggaaccc
caggagccaaggggggaacaaggagataaaggaaatcccgggccttcagagatatcccacgtaataggg
gacaaaggagaaccaggtctcaaaggattcgcaggaaatccaggtgagaaaggaaacagaggcgttcc
agggatgccaggtttaaaggcctcaaaggactacccggaccagcaggaccaccaggccccagaggag
atttgggcagcactgggaatcctggagaaccaggactgcgtggtataccaggaagcatgggaacatg
ggcatgccaggttctaaaggaaaaagggaactttgggattcccaggtcgagcaggaagaccaggcct
cccaggtattcatggtctccagggagataaggagagccaggttattcagaaggtacaaggccaggac
caccgggaccaacgggggatccaggactgccgggtgatatggaaagaaaggagaaatggggcaacct
ggcccacctggacatttgggcctgctggacctgagggagcccctggaagtcctggaagtcctggcct
cccaggaaagccaggtcctcatggtgatttgggttttaaaggaatcaaaggcctcctgggccctccag
gaatcagaggccctccaggtcttccaggatttccaggatctcctggaccaatgggtataagaggtgac
caaggacgtgatggaattcctggtccagccggagaaaaggagaaacgggtttattgaggcccctcc
aggcccaagagggaaccctggtgctcaaggagccaaaggagacaggggagccccaggttttcctggcc
tcccgggcagaaaagggccatgggagatgctggacctcgaggacccacaggcatagaaggattccca
gggccaccaggtctgcccggtgcaattatccctggccagacaggaaatcgtggtccaccaggctcaag
aggaagcccaggtgcgcctggtccccctggacctccagggagtcatgtaataggcataaaggagaca
aagggtctatggccaccctggcccaaaggtccacctggaactgcaggagacatgggaccaccaggt
cgtctgggagcaccaggtactccaggtcttccaggacccagaggtgatcctggattccaggggtttcc
aggcgtgaaaggagaaaagggtaatcctggatttctaggatccattggacctccaggaccaattgggc
caaaaggaccacctggtgtacgtggagaccctggcacacttaagattatctcccttccaggaagccca
gggccacctggcacacctggagaaccagggatgcagggagaacctgggccaccaggccacctggaaa
cctaggaccctgtggccaagaggtaagccaggcaaggatggaaaaccaggaactcctggaccagctg
gagaaaaaggcaacaaaggttctaaaggagagccaggaccagctggatcagatggattgccaggtttg
aaaggaaaacgtggagacagtggatcacctgcaacctggacaacgagaggctttgtcttcacccgaca
cagtcaaaccacagcaattccttcatgtccagaggggacagtgccactctacagtgggttttctttc
tttttgtacaaggaaatcaacgagcccacggacaagaccttggaactcttggcagctgcctgcagcga
tttaccacaatgccattcttattctgcaatgtcaatgatgtatgtaattttgcatctcgaaatgatta
ttcatactggctgtcaacaccagctctgatgccaatgaacatggctcccattactggcagagcccttg
agccttatataagcagatgcactgtttgtgaaggtcctgcgatcgccatagccgttcacagccaaacc
actgacattcctccatgtcctcacggctggatttctctctggaaaggattttcattcatcatgttcac
aagtgcaggttctgagggcaccgggcaagcactggcctcccctggctcctgcctggaagaattccgag
ccagcccatttctagaatgtcatggaagaggaacgtgcaactactattcaaattcctacagtttctgg
ctggcttcattaaacccagaaagaatgttcagaaagcctattccatcaactgtgaaagctggggaatt
agaaaaaataataagtcgctgtcaggtgtgcatgaagaaaagacactga
Example COL4A3 transgene sequence (SEQ ID NO: 7) 
atgtggtctctgcacatagtactaatgaggtgctccttcagattgaccaagtccttggccacaggtcc
ctggtcacttatactcattctcttttctgtacaatatgtatatggagtggaaagaatacattggtc
cttgtggaggaagagattgctctgtttgccactgtgttcctgaaaaggggtctcggggtccaccagga
ccaccagggccacagggtccaattggacccctgggagccccaggacccattgggctttcaggagagaa
aggaatgagaggggaccgcggccctcctggagcagcaggggacaaaggagataaggtccaactggtg
ttcctggatttccaggtttagatggcatacctgggcacccagggcctcctggacccagaggcaaacct
ggtatgagtggccacaatggctcaagaggtgacccagggtttccaggaggaagaggagctcttggccc
aggaggcccccctaggccatcctggggaaaggagaaaaggaaattcagtgttcattttaggtgccg
 
ttaaaggtattcagggagacagaggggacccaggactgcctggcttaccaggatcttggggtgcagga
ggaccggcaggtcccacaggatatcctggagagccagggttagtgggacctccgggccaaccagggcg
tccaggtttgaagggaaatcccggtgtgggagtaaagggcaaatggagacccgggtgaggttggtc
agcaaggttctcctggacccaccctgttggtagagccacctgacttttgtctctataaaggagaaaag
ggtataaaaggaattcctggaatggttggactgccaggaccaccaggacgcaaggagaatctggtat
tggggcaaaaggagaaaaaggtattcctggatttccagggcctcgggggatcctggttcctatggat
ctccaggttttccaggattaaagggagaactaggactggttggagatcctgggctatttggattaatt
ggcccaaaggggggatcctggaaatcgaggcacccaggaccaccaggtgttttggtgactccacctct
tccactcaaaggcccaccaggggacccagggttccctggccgctatggagaaacaggggatgttggac
cacctggtccccccaggtctcttgggcagaccaggggaagcctgtgcaggcatgataggacccccctggg
ccacaaggatttcctggtcttcctgggcttccaggagaagctggtattcctggagacctgattctgc
tccaggaaaaccagggaagccaggatcacctggcttgcctggagcaccaggcctgcaggcctcccag
gatcaagtgtgatatactgtagtgttgggaaccccggaccacaaggaataaaggcaaagttggtccc
ccaggaggaagaggcccaaaaggagaaaaaggaaatgaaggactctgtgcctgtgagcctggacccat
gggccccccctggccctccaggacttcctgggaggcaggggagtaaggagacttggggctccctggct
ggcttggaacaaaaggtgacccaggacctcctggtgctgaaggacctccaggctaccaggaaagcat
ggtgcctctggaccacctggcaacaaaggggcgaaggtgacatggttgtatcaagagttaaagggca
caaaggagaaagaggtcctgatgggcccccaggatttccaggcagccaggatcacatggtcggatg
gacatgctggagaaaaggggatccaggacctccaggggatcatgaagatgcgaccccaggtggtaaa
ggatttcctggacctctgggcccccaggcaaagcaggacctgtggggcccccaggactgggatttcc
tggtccaccaggagagcgaggccacccaggagttccaggccacccaggtgtgaggggccctgatggct
tgaagggtcagaaaggtgacacaatttcttgcaacgtaacctaccctgggaggcatggccctccaggt
tttgatggacctccaggtccgaagggatttccaggtccccaaggtgcccctgggctgagtggttcaga
tgggcataaaggcagacctggcacaccaggaacagcggaaataccaggtccacctggttttcgtggtg
acatgggagatccgggttttggaggtgaaaaggggtcctcccctgttgggcccccaggccctcccggc
tcaccaggagtgaatggtcagaaaggaatcccgggagaccctgcatttggtcacctggacccccggg
aaagaggggtctttcaggagtgccaggataaaaggacccagaggtgatccgggatgtccagggggctg
aagggccagctggcattcctggattcctaggtctcaaaggtcccaaaggcagagaggacatgctggg
tttccaggtgtcccaggtccacctggccattcctgtgaaagaggtgctccaggataccaggcaacc
gggactccctgggtatccaggtagcccaggtgctccaggtgggaaaggacagccgggagatgtggggc
ctcccgggccagctggaatgaaaggcctccccggactcccaggacggcctggggcacatggtcccca
ggcctcccaggaatcccaggtccctttggagatgatggctacctggtcctccaggtccaaaggacc
ccgggggctgcctggtttcccaggttttcccggagaaagaggaaagcctggtgcagaggatgtcctg
gcgcaaagggagaacctggagagaaggcatgtctggccttcctggagaccggggactgagaggggcc
aaaggagccataggacctcccggagatgaaggagaaatggctatcatttcacaaaaggaacacctgg
ggaacctggacctcctggagatgatggattcccaggagaaagaggtgataaaggaactcccggatgc
aagggagaagaggagagccgggaagatacggaccacctggatttcacagaggggaacctggtgagaaa
ggtcagccaggcctcctggacccccaggccctccaggctcaactggtctaagaggttcattggttt
tccaggacttccaggtgaccaggtgagccaggttctccaggtcccctggattttcaggaattgatg
gagcaagaggacctaaaggaaacaaaggtgaccctgccagtcactttggtccacctggtccaaagggt
gagccaggtagccctggatgtccagggcattttggagcatccggagagcaggcttgcctggtattca
agggcccagaggatcacctggaaggccagggccacctggctcctctggaccaccagggtgcccaggtg
atcacgggatgcctggctgaggggacagccaggagaaatggagaccctggccaagaggcctccag
ggggatccagggataccaggtcctccgggaataaaaggtccctccggatcacctggcctgaacggctt
gcatggattgaaaggtcagaaaggaactaaaggtgcttcaggtttgcatgatgtggggccacctggtc
cagtgggaatacctgggctaaaggggagagaggagaccctgggagcccaggaatctctcctccaggt
cctcgtggaaagaaaggtcccccaggacccccagggagttcaggaccacctggtcctgcaggtgccac
aggaagagctcctaaggacattcctgacccgggtccacctggagatcagggacctcctggtcctgatg
gcccaagaggagcacctgggcctccaggcctccctgggagtgttgaccttctgagaggggagccaggt
gactgtggtctaccagggccaccaggtccccctggcccaccaggccctccaggatacaaaggctttcc
aggatgtgatggaaaagatggccagaaaggaccagtgggattcccggaccgcagggaccacatggat
ttcctgggccacctggagagaagggtttacctggacctccagggagaaaaggcccactggtcttccg
ggtcccagaggtgaaccggggccacctgcagatgtggatgactgtccccgaatcccaggccttcctgg
ggcgccaggcatgagaggaccagaaggagccatggggctccctggaatgagaggcccctcaggaccag
ggtgcaaaggagagcctggctggatggcaggagggtgtggatggcgtccctgggtctcctggcct
cccggacgtaaaggtgacacaggagaagacggctaccctggaggaccagggcctcctggtcccattgg
 
ggatcctgggcccaaagggtttggccctggatacctcggtggcttcctcctggttctccacagtcaga
cggaccaggagcccacctgccccctgggcatgcccaggctctggactgggtatagtctgttatacctg
gaagggcaagagaaagctcacaatcaagaccttggtctggcagggtcttgccttcccgtatttagcac
gctgccctttgcctactgcaacatccaccaggtgtgccactatgcccagagaaacgacagatcctact
ggctggccagcgctgcgcccctccccatgatgccactctctgaagaggcgatccgcccctatgtcagc
cgctgtgcggtatgcgaggccccggcccaggcggtggcggtgcacagccaggaccagtccatcccccc
atgtccgcagacctggaggagcctctggatcggtattcattcctgatgcacacaggagctggggacc
aaggaggagggcaggcccttatgtcacctggcagctgcctggaagatttcagagcagcaccattcctt
gaatgccagggccggcagggaacttgccactttttcgcaaataagtatagcttctggctcacaacggt
gaaagcagacttgcagttttcctctgctccagcaccagacaccttaaaagaaagccaggcccaacgcc
agaaaatcagccggtgccaggtctgcgtgaagtatagctag
Example COL4A4 transgene sequence (SEQ ID NO: 8) 
atgaaactgcgtggagtcagcctggctgccggcttgttcttactggccctgagtctttgggggcagcc
tgcagaggctgcggcttgctatgggtgttctccaggatcaaagtgtgactgcagtggcataaagggg
aaaagggagagagagggtttccaggtttggaaggacacccaggattgcctggatttccaggtccagaa
gggcctccggggcctcggggacaaaaggtgatgatggaattccagggccaccaggaccaaaaggaat
cagaggtcctcctggacttcctggatttccaggacaccaggtcttcctggaatgccaggccacgatg
ggccccaggacctcaaggtattcccggatgcaatggaaccaaggagaacgtggatttccaggcagt
cccggttttcctggtttacagggtcctccaggacccctgggatcccaggtatgaagggtgaaccagg
tagtataattatgtcatcactgccaggaccaaaggtaatccaggatatccaggtcctcctggaatac
aaggcctacctggtcccactggtataccagggccaattggtccccaggaccaccaggtttgatgggc
cctcctggtccaccaggacttccaggacctaaggggaatatggcttaaatttccaggacccaaagg
tgaaaaaggtgagcaaggtcttcagggcccacctgggccacctggcagatcagtgaacagaaaagac
caattgatgtagagtttcagaaaggagatcagggacttcctggtgaccgagggcctcctggacctcca
gggatacgtggtcctccaggtccccaggtggtgagaaaggtgagaagggtgagcaaggagagccagg
caaaagaggtaaaccaggcaaagatggagaaaatggccaaccaggaattcctggtttgcctggtgatc
ctggttaccctggtgaacccggaaggatggtgaaaaggccaaaaaggtgacactggcccacctgga
cctcctggacttgtaattcctagacctgggactggtataactataggagaaaaaggaaacattgggtt
gcctgggttgcctggagaaaaaggagagcgaggatttcctggaatacagggtccacctggccttcctg
gacctccaggggctgcagttatgggtcctcctggccctcctggatttcctggagaaaggggtcagaaa
ggtgatgaaggaccacctggaatttccattcctggacctcctggacttgacggacagcctggggctcc
tgggcttccagggcctcctggccctgctggccctcacattcctcctagtgatgagatatgtgaaccag
gccctccaggcccccaggatctccaggtgataaaggactccaaggagaacaaggagtgaaaggtgac
aaaggtgacacttgcttcaactgcattggaactggtatttcaggcctccaggtcaacctggtttgcc
aggtctcccaggtcctccaggatctcttggtttccctggacagaaaggggaaaaaggacaagctggtg
caactggtcccaaaggattaccaggcattccaggagctccaggtgctccaggctttcctggatctaaa
ggtgaacctggtgatatcctcactttccaggaatgaagggtgacaaaggagagttgggttcccctgg
agctccaggcttcctggtttacctggcactcctggacaggatggattgccagggcttcctggcccga
aaggagagcctggtggaattacttttaagggtgaaagaggtcccctgggaacccaggtttaccaggc
ctcccagggaatataggcctatgggtcccctggtttcggccctccaggcccagtaggtgaaaaagg
catacaaggtgtggcaggaaatccaggccagccaggaataccaggtcctaaagggatccaggtcaga
ctataacccagccggggaagcctggcttgcctggtaacccaggcagagatggtgatgtaggtcttcca
ggtgaccctggacttccaggcaaccaggcttgccaggatacctggtagcaaaggagaaccaggtat
ccctggaattgggcttcctggaccacctggtcccaaaggctttcctggaattccaggacctccaggag
cacctggacacctggaagaattggtctagaaggccctcctggccacccggctttccaggaccaaag
ggtgaaccaggatttgcattacctggccacctggccaccaggacttccaggtttcaaaggagcact
tggtccaaaaggtgatcgtggtttcccaggacctccgggtcctccaggacgcactggcttagatggc
tccctggaccaaaaggtgatgttggaccaaatggacaacctggaccaatgggacctcctggctgcca
ggaataggtgttcaggaccaccaggaccaccagggattcctggccaataggtcaacctggtttaca
tggaataccaggagagaaggggatccaggacctcctggacttgatgttccaggacccccaggtgaaa
gaggcagtccagggatccccggagcacctggtcctataggacctccaggatcaccaggcttccagga
aaagcaggtgcctctggatttccaggtaccaaaggtgaaatgggtatgatgggacctccaggcccacc
aggacctttgggaattcctggcaggagtggtgtacctggtcttaaaggtgatgatggcttgcagggtc
agccaggacttcctggccctacaggagaaaaaggtagtaaaggagagcctggccttccaggccctcct
 
ggaccaatggatccaaatcttctgggctcaaaggagagaaggggaacctggcttaccaggtatacc
tggagtttcagggccaaaaggttatcagggtttgcctggagacccagggcaacctggactgagtggac
aacctggattaccaggaccaccaggtcccaaaggtaaccctggtctccctggacagccaggtcttata
ggacctcctggacttaaaggaaccatcggtgatatgggtttccaggcctcaggtgtggaaggcc
tcctggaccttctggagttcctggacaacctggctccccaggattacctggacagaaaggcgacaaag
gtgatcctggtatttcaagcattggtcttccaggtcttcctggtccaaaggtgagcctggtctgcct
ggatacccagggaaccctggtatcaaaggttctgtgggagatcctggtttgcccggattaccaggaac
ccctggagcaaaggacaaccaggccttcctggattcccaggaaccccaggccctcctggaccaaaag
gtattagtggccctcctgggaaccccggccttccaggagaacctggtcctgtaggtggtggaggtcat
cctgggcaaccaggcctccaggcgaaaaggcaaacccggtcaagatggtattcctggaccagctgg
acagaagggtgaaccaggtcaaccaggctttggaaacccaggacccccctggacttccaggactttctg
gccaaaagggtgatggaggattacctgggattccaggaaatcctggccttccaggtccaaagggcgaa
ccaggctttcacggtttccctggtgtgcaggtcccccaggccctcctggttctccgggtccagctct
ggaaggacctaaaggcaaccctgggccccaaggtcctcctgggagaccaggtctaccaggtccagaag
gtcctccaggtctccctggaaatggaggtattaaaggagagaagggaaatccaggccaacctgggcta
cctggcttgcctggtttgaaaggagatcaaggaccaccaggactccagggtaatcctggccggccggg
tctcaatggaatgaaaggagatcctggtctccctggtgttccaggattcccaggcatgaaaggaccca
gtggagtacctggatcagctggccctgaggggaaccggacttattggtcctccaggtcctcctgga
ttacctggtccttcaggacagagtatcataattaaaggagatgctggtcctccaggaatccctggcca
gcctgggctaaagggtctaccaggaccccaaggacctcaaggcttaccaggtccaactggccctccag
gagatcctggacgcaatggactccctggctttgatggtgcaggagggcgcaaaggagacccaggtctg
ccaggacagccaggtacccgtggtttggatggtcccccctggtccagatggattgcaaggtcccccagg
tccccctggaacctcctctgttgcacatggatttcttattacacgccacagccagacaacggatgcac
cacaatgcccacaggaacacttcaggtctatgaaggcttttctctcctgtatgtacaaggaaataaa
agagcccacggtcaagacttggggacggctggcagctgccttcgtcgctttagtaccatgcctttcat
gttctgcaacatcaataatgtttgcaactttgcttcaagaaatgactattcttactggctctctaccc
cagagcccatgccaatgagcatgcaacccctaaaggccagagcatccagccattcattagtcgatgt
gcagtatgtgaagctccagctgtggtgatcgcagttcacagtcagacgatccagattccccattgtcc
tcaggatgggattctctgtggattggttattccttcatgatgcatacaagtgcagggcagaaggct
caggtcaagccctagcctcccctggttcctgcttggaagagtttcgttcagctcccttcatcgaatgt
catgggagggtacctgtaactactatgccaactcctacagcttttggctggcaactgtagatgtgtc
agacatgttcagtaaacctcagtcagaaacgctgaaagcaggagacttgaggacacgaattagccgat
gtcaagtgtgcatgaagaggacataa
Example COL4A5 transgene sequence (SEQ ID NO: 9)

The COL4A3, COL4A4 or COL4A5 transgene may comprise an intron or intronic sequences, which can be used to improve gene expression. The COL4A3, COL4A4 or COL4A5 transgene may comprise a protein tag, such as a hemagglutinin (HA) tag. HA can be used as an epitope tag and has been shown not to interfere with bioactivity or biodistribution of proteins to which it has been added. The protein tag can facilitate detection, isolation, and purification of the transgene. Other suitable protein tags may include Myc tags, polyhistidine tags and flag tags.

### Nephrotic syndrome (NS)-associated transgene

The protein-coding sequence may comprise or consist of a NS-associated transgene.

Nephrotic syndrome (NS) is a chronic kidney disease characterized by significant proteinuria, hypoalbuminemia, oedema and hyperlipidemia. The NS-associated transgene may be a gene associated with a monogenic form of NS and expressed in podocytes.

Suitable NS-associated transgenes include *NPHS2, ADCK4, ALG1, ARHGAP24, ARGHDIA,* CD151, CD2AP, COQ2, COQ6, *DGKE, E2F3, EMP2, KANK2, LAGE3, LMNA, LMX1B, MAFB, NUP85, NUP93, NXF5, OSGEP, PAX2, PDSS2, PMM2, PODXL, SCARB2, SGPL 1, Smad7, TP53RK, TPRKB, VDR, WDR73, WT1, ZMPSTE24, APOL1, NPHS1, TRPC6, NUP107, NUP133, NUP160, ACTN4, INF2, ANKFY1, ANLN, CRB2, ITGA3, KANK1, KANK4, MAGI2, MYO1E, OCRL, PTPRO, SMARCAL1, SYNPO, TBC1D88, XPO5, TNS2* and *NLRP3.*

### NPHS2

The protein-coding sequence may encode NPHS2, or a fragment and/or variant thereof.

"NPHS2" is the abbreviated name of the polypeptide encoded by the *NPHS2* gene and is also known as podocin. NPHS2 is a 42kDa hairpin like membrane-associated podocyte-specific protein that is a key component of the protein complex at the slit diaphragm; the cell-cell junction between adjacent podocyte foot processes. It localises to lipid rafts and interacts with other important slit diaphragm proteins like nephrin, CD2AP and TRPC6. It is essential in the maintenance of the slit diaphragm, and consequently the integrity of the glomerular filtration barrier.

A fragment and/or variant of NPHS2 may retain NPHS2 activity or function. For example, a fragment and/or variant of podocin may regulate glomerular permeability. Suitably, a fragment and/or variant of NPHS2 may have the same or similar activity or function to NPHS2, e.g. may have at least 40%, at least 50%, at least 60%, at least 70%, at least 80%, at least 90%, at least 95%, or at least 100% of the activity or function of NPHS2.

A person skilled in the art would be able to generate fragments and/or variants using conservative substitutions, based on the known structural and functional features of NPHS2 (see e.g. Tabassum, A., et al., 2014. Interdisciplinary Sciences: Computational Life Sciences, 6(1), pp.32-39), and/or based on known variants (see e.g. NCBI Gene ID: 7827 and NCBI HomoloGene: 22826). Suitably, a fragment and/or variant of NPHS2 comprises a transmembrane domain, with two cytoplasmic domains at the N- and C-terminus.

The *NPHS2* gene is conserved in chimpanzee, Rhesus monkey, dog, cow, mouse, and rat. The NPHS2 may be a human NPHS2. Suitably, the NPHS2 may comprise or consist of a polypeptide sequence of UniProtKB accession Q9NP85, or a fragment and/or variant thereof.

In some embodiments, the NPHS2 comprises or consists of an amino acid sequence which is at least 70% identical to SEQ ID NO: 10 or a fragment thereof. Suitably, the NPHS2 comprises or consists of an amino acid sequence which is at least 75%, at least 80%, at least 85%, at least 90%, at least 95%, at least 96%, at least 97%, at least 98% or at least 99% identical to SEQ ID NO: 10 or a fragment thereof.

In some embodiments, the NPHS2 comprises or consists of SEQ ID NO: 10 or a fragment thereof.

### Example NPHS2 amino acid sequence (SEQ ID NO: 10)

In some embodiments, the NPHS2 transgene comprises or consists of a nucleotide sequence which is at least 70% identical to SEQ ID NO: 11 or a fragment thereof. Suitably, the NPHS2 transgene comprises or consists of a nucleotide sequence which is at least 75%, at least 80%, at least 85%, at least 90%, at least 95%, at least 96%, at least 97%, at least 98% or at least 99% identical to SEQ ID NO: 11 or a fragment thereof.

In some embodiments, the NPHS2 transgene, comprises or consists of the nucleotide sequence SEQ ID NO: 11 or a fragment thereof.

ATGGAGAGGAGGGCGCGGAGCTCCTCCAGGGAGTCCCGCGGCGAGGCGGCAGGACTCCGCACAAGGAG
AACAAGAGGGCAAAGGCCGAGAGGAGCGGCGGGGCCGCGGCGCCAGGAGGCTGGGCCCGAGCCGTCG
GGCTCCGGACGGGCGGGGACCCCGGGGGAGCCCCGAGCGCCCGCCGCCACGGTGGTGGACGTGGATGAG
GTCCGAGGCTCCGGCGAGGAGGGCACCGAGGTGGTGGCGCTGTTGGAGAGCGAGCGGCCCGAGGAAGGT
ACCAAATCCTCCGGCTTAGGGGCCTGTGAGTGGCTTCTTGTCCTCATTTCCCTGCTCTTCATCATCATG
ACCTTCCCTTTTTCCATCTGGTTCTGCGTAAAGGTTGTACAAGAGTATGAAAGAGTAATTATATTCCGA
CTGGGACATCTGCTTCCTGGAAGAGCCAAAGGCCCTGGTCTTTTCTTTTTTTGCCCTGCCTGGATACC
TACCACAAGGTTGACCTTCGTCTCCAAACTCTGGAGATACCTTTTCATGAGATCGTGACCAAAGACATG
TTTATAATGGAGATAGATGCCATTTGCTACTACCGAATGGAAAATGCCTCTCTTCTCCTAAGCAGTCTT
GCTCATGTATCTAAAGCTGTGCAATTCCTTGTGCAAACCACTATGAAGCGTCTCCTAGCACATCGATCC
CTCACTGAAATTCTTCTAGAGAGGAAGAGCATCGCCCAAGATGCAAAGGTTGCCTTGGATTCAGTGACC
TGTATTTGGGGAATCAAAGTGGAGAGAATAGAAATTAAAGATGTGAGGTTGCCAGCTGGGCTTCAGCAC
TCACTGGCTGTGGAGGCTGAAGCGCAAAGACAAGCCAAAGTGCGGATGATTGCTGCAGAAGCGGAAAAG
GCTGCTTCTGAGTCCCTGAGGATGGCAGCTGAGATTCTGTCAGGCACCCCTGCTGCTGTTCAGCTTCGA
TACCTCCACACCCTTCAGTCTCTGTCCACAGAGAAGCCTTCCACTGTGGTTTTACCTTTGCCATTTGAC
CTACTGAATTGCCTGTCTTCTCCCAGCAACAGAACTCAGGGAAGCCTCCCCTTCCCAAGTCCTTCCAAA
CCTGTTGAGCCACTAAATCCTAAAAAGAAAGACTCTCCCATGTTA

### Example NPHS2 transgene sequence (SEQ ID NO: 11)

In some embodiments, the NPHS2 transgene comprises or consists of a nucleotide sequence which is at least 70% identical to SEQ ID NO: 12 or a fragment thereof. Suitably, the NPHS2 transgene comprises or consists of a nucleotide sequence which is at least 75%, at least 80%, at least 85%, at least 90%, at least 95%, at least 96%, at least 97%, at least 98% or at least 99% identical to SEQ ID NO: 12 or a fragment thereof.

In some embodiments, the NPHS2 transgene, comprises or consists of the nucleotide sequence SEQ ID NO: 12 or a fragment thereof.

ATGGAGAGGAGGGCGCGGAGCTCCTCCAGGGAGTCCCGCGGCGAGGCGGCAGGACTCCGCACAAGGAG
AACAAGAGGGCAAAGGCCGAGAGGAGCGGCGGAGGCCGCGGGCGCCAGGAGGCTGGGCCCGAGCCGTCG
GGCTCCGGACGGGCGGGGACCCCGGGGGAGCCCCGAGCGCCCGCCGCCACGGTGGTGGACGTGGATGAG
GTCCGAGGCTCCGGCGAGGAGGGCACCGAGGTGGTGGCGCTGTTGGAGAGCGAGCGGCCCGAGGAAGGT
ACCAAATCCTCCGGCTTAGGGGCCTGTGAGTGGCTTCTTGTCCTCATTTCCCTGCTCTTCATCATCATG
ACCTTCCCTTTTTCCATCTGGTTCTGCGTAAAGGTTGTACAAGAGTATGAAAGAGTAATTATATTCCGA
CTGGGACATCTGCTTCCTGGAAGAGCCAAAGGCCCTGGTCTTTTCTTTTTTTGCCCTGCCTGGATACC
TACCACAAGGTTGACCTTCGTCTCCAAACTCTGGAGATACCTTTTCATGAGATCGTGACCAAAGACATG
TTTATAATGGAGATAGATGCCATTTGCTACTACCGAATGGAAAATGCCTCTCTTCTCCTAAGCAGTCTT
GCTCATGTATCTAAAGCTGTGCAATTCCTTGTGCAAACCACTATGAAGCGTCTCCTAGCACATCGATCC
CTCACTGAAATTCTTCTAGAGAGGAAGAGCATCGCCCAAGATGCAAAGGTTGCCTTGGATTCAGTGACC
TGTATTTGGGGAATCAAAGTGGAGAGAATAGAAATTAAAGATGTGAGGTTGCCAGCTGGGCTTCAGCAC
TCACTGGCTGTGGAGGCTGAAGCGCAAAGACAAGCCAAAGTGCGGATGATTGCTGCAGAAGCGGAAAAG
GCTGCTTCTGAGTCCCTGAGGATGGCAGCTGAGATTCTGTCAGGCACCCCTGCTGCCGTTCAGCTTCGA
TACCTCCACACCCTTCAGTCTCTGTCCACAGAGAAGCCTTCCACTGTGGTTTTACCTTTGCCATTTGAC
CTACTGAATTGCCTGTCTTCTCCCAGCAACAGAACTCAGGGAAGCCTCCCCTTCCCAAGTCCTTCCAAA
CCTGTTGAGCCACTAAATCCTAAAAAGAAAGACTCTCCCATGTTATAG

### Example NPHS2 transgene sequence (SEQ ID NO: 12)

### Vascular endothelial growth factor (VEGF)C transgene

The protein-coding sequence may comprise or consist of a vascular endothelial growth factor (VEGF)C transgene.

VEGFC is a lymphangiogenic growth factor, which is known to signal via two receptors, VEGFR-3 (Flt4) and VEGFR-2 (Flk4). VEGFC is produced by cells in a prepropeptide form, which dimerises before being cleaved into a tetramer.

The VEGFC transgene may comprises a polynucleotide encoding any form of VEGFC, such as the prepropeptide form, the tetramer form, the intermediate form, or fully processed mature VEGFC.

If desired, polynucleotides encoding different forms of VEGFC polypeptides may be used in any combination. Preferably the VEGFC transgene comprises a polynucleotide encoding one or more polypeptides having VEGFC biological activity, i.e., peptides that can bind to and activate VEGFR-2 and/or VEGRF-3. More preferably, the VEGFC transgene comprises a polynucleotide encoding a polypeptide comprising the VEGFC homology domain and having VEGFC biological activity, i.e., a polypeptide that can bind to and activate VEGFR-2 and/or VEGRF-3. Further details of suitable VEGFC polynucleotides and polypeptides include those described in WO 2015/022447 and US 2014/0087002.

The VEGFC polynucleotide may comprise the VEGFC open reading frame (ORF) sequence of SEQ ID NO: 13. The VEGFC polynucleotide may comprise a nucleic acid sequence which has at least 70%, at least 75%, at least 80%, at least 85%, at least 90%, at least 95%, at least 96%, at least 97%, at least 98%, or at least 99% sequence identity to the VEGFC ORF sequence of SEQ ID NO: 13. The variant sequence may encode a VEGFC polypeptide that has retained the capability to bind and activate VEGFR-2 and VEGFR-3.

ATGCACTTGCTGGGCTTCTTCTCTGTGGCGTGTTCTCTGCTCGCCGCTGCGCTGCTCCCGGGTCCTCG
CGAGGCGCCCGCCGCCGCCGCCTTCGAGTCCGGACTCGACCTCTCGGACGCGGAGCCCGACGCGG
GCGAGGCCACGGCTTATGCAAGCAAAGATCTGGAGGAGCAGTTACGGTCTGTGTCCAGTGTAGATGAA
CTCATGACTGTACTCTACCCAGAATATTGGAAAATGTACAAGTGTCAGCTAAGGAAAGGAGGCTGGCA
ACATAACAGAGAACAGGCCAACCTCAACTCAAGGACAGAAGAGACTATAAAATTTGCTGCAGCACATT
ATAATACAGAGATCTTGAAAAGTATTGATAATGAGTGGAGAAAGACTCAATGCATGCCACGGGAGGTG
TGTATAGATGTGGGGAAGGAGTTTGGAGTCGCGACAAACACCTTCTTTAAACCTCCATGTGTGTCCGT
CTACAGATGTGGGGGTTGCTGCAATAGTGAGGGGCTGCAGTGCATGAACACCAGCACGAGCTACCTCA
GCAAGACGTTATTTGAAATTACAGTGCCTCTCTCTCAAGGCCCCAAACCAGTAACAATCAGTTTTGCC
AATCACACTTCCTGCCGATGCATGTCTAAACTGGATGTTTACAGACAAGTTCATTCCATTATTAGACG
TTCCCTGCCAGCAACACTACCACAGTGTCAGGCAGCGAACAAGACCTGCCCCACCAATTACATGTGGA
ATAATCACATCTGCAGATGCCTGGCTCAGGAAGATTTTATGTTTTCCTCGGATGCTGGAGATGACTCA
ACAGATGGATTCCATGACATCTGTGGACCAAACAAGGAGCTGGATGAAGAGACCTGTCAGTGTGTCTG
CAGAGCGGGGCTTCGGCCTGCCAGCTGTGGACCCCACAAAGAACTAGACAGAAACTCATGCCAGTGTG
TCTGTAAAAACAAACTCTTCCCCAGCCAATGTGGGGCCAACCGAGAATTTGATGAAAACACATGCCAG
TGTGTATGTAAAAGAACCTGCCCCAGAAATCAACCCCTAAATCCTGGAAAATGTGCCTGTGAATGTAC
AGAAAGTCCACAGAAATGCTTGTTAAAAGGAAAGAAGTTCCACCACCAAACATGCAGCTGTTACAGAC
GGCCATGTACGAACCGCCAGAAGGCTTGTGAGCCAGGATTTTCATATAGTGAAGAAGTGTGTCGTTGT
GTCCCTTCATATTGGAAAAGACCACAAATGAGC

### Example VEGFC polynucleotide (SEQ ID NO: 13)

### Complement proteins and complement inhibitors

The protein-coding sequence may encode a complement protein, or a fragment and/or variant thereof.

As used herein, a "complement protein" is a protein which is part of the complement system. The complement system, also known as complement cascade, is a central part of the innate immunity that serves as a first line of defence against foreign and altered host cells. The complement system is composed of plasma proteins produced mainly by the liver or membrane proteins expressed on cell surface. Complement operates in plasma, in tissues, or within cells. Complement proteins collaborate as a cascade to opsonize pathogens and induce a series of inflammatory responses helping immune cells to fight infection and maintain homeostasis (Merle, N.S., et al., 2015. Frontiers in immunology, 6, 262).

There are three pathways of complement activation: the classical, the alternative, and the lectin pathways. The three complement pathways differ in their mechanisms of target recognition but converge in the activation of the central component C3. After this activation, C5 is cleaved, and the assembly of the membrane attack complex (MAC) is initiated. The enzymatic cleavage of C3 and C5 leads to the production and release of anaphylotoxins C3a and C5a.

Suitably, the complement protein is selected from the list consisting of CFI, CFH, FHL-1, C1INH, C4BP, MASP2, C3, C5aR1, C5, C5a, CD55, CD35, CD46, CD59, vitronectin, and clusterin, or fragments and/or variants thereof.

The protein-coding sequence may encode an inhibitor of the complement system, or a fragment and/or variant thereof.

As used herein, an "inhibitor of the complement system" or "complement inhibitor" is a protein which prevents activation of the complement system. Complement is tightly controlled by these inhibitors, which naturally protect self cells and tissues from unwanted complement activation. Complement inhibitors can regulate complement activation in different stages of the classical, lectin, and alternative pathways. Suitably, the complement inhibitor is a naturally-occurring complement inhibitor, or a fragment and/or variant thereof. Preferably, the inhibitor of the complement system is an inhibitor of the complement system in humans.

Complement inhibitors are grouped into two categories: soluble inhibitors and membrane-bound inhibitors. Preferably, the inhibitor of the complement system is a soluble complement inhibitor. Soluble complement inhibitors include C1 inhibitor (C1INH), complement factor I (CFI), complement factor H (CFH), complement factor H-like protein 1 (FHL-1), C4 binding protein (C4BP), clusterin and vitronectin. Membrane-bound regulators include CD46, CD55, CD59, CD35 and CUB and Sushi multiple domain 1 (CSMD1).

The inhibitor of the complement system may be selected from: CFI, CFH, FHL-1, C1INH, C4BP, CD46, CD55, CD59, CD35, vitronectin, clusterin, and CSMD1, or fragments and/or variants thereof.

Preferably, the inhibitor of the complement system is selected from: CFI, CFH, and FHL-1, or fragments and/or variants thereof.

### CFI

The protein-coding sequence may encode CFI, or a fragment and/or variant thereof.

Complement factor I (CFI) is a trypsin-like serine protease that inhibits the complement system by cleaving three peptide bonds in the alpha-chain of C3b and two bonds in the alpha-chain of C4b thereby inactivating these proteins.

CFI is a glycoprotein heterodimer consisting of a disulfide linked heavy chain and light chain. The heavy chain has four domains: an FI membrane attack complex (FIMAC) domain, CD5 domain, and low density lipoprotein receptor 1 and 2 (LDLr1 and LDLr2) domains. The heavy chain plays an inhibitory role in maintaining the enzyme inactive until it meets the complex formed by the substrate (either C3b or C4b) and a cofactor protein (Factor H, C4b-binding protein, complement receptor 1, and membrane cofactor protein). Upon binding of the enzyme to the substrate:cofactor complex, the heavy:light chain interface is disrupted, and the enzyme activated by allostery. The light chain contains only the serine protease domain. This domain contains the catalytic triad His-362, Asp-411, and Ser-507, which is responsible for specific cleavage of C3b and C4b.

The CFI or a fragment and/or variant thereof may be capable of cleaving C3b into iC3b and/or may be capable of cleaving iC3b into C3d,g.

The fragment and/or variant of CFI may retain at least 50%, 60%, 70%, 80%, 90%, 95% or 100% of the C3b-inactivating and iC3b-degradation activity of native CFI. The C3b-inactivating and iC3b-degradation activity of the fragment and/or variant of CFI and native CFI, may be determined using any suitable method known to those of skill in the art. For example, using a proteolytic assay.

Preferably, the CFI is a human CFI. An example human CFI is the CFI having the UniProtKB accession number P05156.

Suitably, the CFI may comprise or consist of the polypeptide sequence shown as SEQ ID NO: 14, or a variant which is at least 70% identical to SEQ ID NO: 14.

MKLLHVFLLFLCFHLRFCKVTYTSQEDLVEKKCLAKKYTHLSCDKVFCQPWQRCIEGTCVCKLPYQCP
KNGTAVCATNRRSFPTYCQQKSLECLHPGTKFLNNGTCTAEGKFSVSLKHGNTDSEGIVEVKLVDQDK
TMFICKSSWSMREANVACLDLGFQQGADTQRRFKLSDLSINSTECLHVHCRGLETSLAECTFTKRRTM
GYQDFADVVCYTQKADSPMDDFFQCVNGKYISQMKACDGINDCGDQSDELCCKACQGKGFHCKSGVCI
PSQYQCNGEVDCITGEDEVGCAGFASVTQEETEILTADMDAERRRIKSLLPKLSCGVKNRMHIRRKRI
VGGKRAQLGDLPWQVAIKDASGITCGGIYIGGCWILTAAHCLRASKTHRYQIWTTVVDWIHPDLKRIV
IEYVDRIIFHENYNAGTYQNDIALIEMKKDGNKKDCELPRSIPACVPWSPYLFQPNDTCIVSGWGREK
DNERVFSLQWGEVKLISNCSKFYGNRFYEKEMECAGTYDGSIDACKGDSGGPLVCMDANNVTYVWGVV
SWGENCGKPEFPGVYTKVANYFDWISYHVGRPFISQYNV

### Example CFI polypeptide sequence (SEQ ID NO: 14)

Suitably, the variant may be at least 75%, at least 80%, at least 85%, at least 90%, at least 95%, at least 96%, at least 97%, at least 98%, at least 99% identical to SEQ ID NO: 14.

An example nucleotide sequence encoding CFI is NM_000204.5. Suitably, a protein-coding sequence encoding CFI may comprise or consist of the polynucleotide sequence shown as SEQ ID NO: 15, or a variant which is at least 70% identical to SEQ ID NO: 15.

ATGAAGCTTCTTCATGTTTTCCTGTTATTTCTGTGCTTCCACTTAAGGTTTTGCAAGGTCACTTATAC
ATCTCAAGAGGATCTGGTGGAGAAAAAGTGCTTAGCAAAAAAATATACTCACCTCTCCTGCGATAAAG
TCTTCTGCCAGCCATGGCAGAGATGCATTGAGGGCACCTGTGTTTGTAAACTACCGTATCAGTGCCCA
AAGAATGGCACTGCAGTGTGTGCAACTAACAGGAGAAGCTTCCCAACATACTGTCAACAAAAGAGTTT
GGAATGTCTTCATCCAGGGACAAAGTTTTTAAATAACGGAACATGCACAGCCGAAGGAAAGTTTAGTG
TTTCCTTGAAGCATGGAAATACAGATTCAGAGGGAATAGTTGAAGTAAAACTTGTGGACCAAGATAAG
ACAATGTTCATATGCAAAAGCAGCTGGAGCATGAGGGAAGCCAACGTGGCCTGCCTTGACCTTGGGTT
TCAACAAGGTGCTGATACTCAAAGAAGGTTTAAGTTGTCTGATCTCTCTATAAATTCCACTGAATGTC
TACATGTGCATTGCCGAGGATTAGAGACCAGTTTGGCTGAATGTACTTTTACTAAGAGAAGAACTATG
GGTTACCAGGATTTCGCTGATGTGGTTTGTTATACACAGAAAGCAGATTCTCCAATGGATGACTTCTT
TCAGTGTGTGAATGGGAAATACATTTCTCAGATGAAAGCCTGTGATGGTATCAATGATTGTGGAGACC
AAAGTGATGAACTGTGTTGTAAAGCATGCCAAGGCAAAGGCTTCCATTGCAAATCGGGTGTTTGCATT
CCAAGCCAGTATCAATGCAATGGTGAGGTGGACTGCATTACAGGGGAAGATGAAGTTGGCTGTGCAGG
CTTTGCATCTGTGACTCAAGAAGAAACAGAAATTTTGACTGCTGACATGGATGCAGAAAGAAGACGGA
TAAAATCATTATTACCTAAACTATCTTGTGGAGTTAAAAACAGAATGCACATTCGAAGGAAACGAATT
GTGGGAGGAAAGCGAGCACAACTGGGAGACCTCCCATGGCAGGTGGCAATTAAGGATGCCAGTGGAAT
CACCTGTGGGGGAATTTATATTGGTGGCTGTTGGATTCTGACTGCTGCACATTGTCTCAGAGCCAGTA
AAACTCATCGTTACCAAATATGGACAACAGTAGTAGACTGGATACACCCCGACCTTAAACGTATAGTA
ATTGAATACGTGGATAGAATTATTTTCCATGAAAACTACAATGCAGGCACTTACCAAAATGACATCGC
TTTGATTGAAATGAAAAAAGACGGAAACAAAAAAGATTGTGAGCTGCCTCGTTCCATCCCTGCCTGTG
TCCCCTGGTCTCCTTACCTATTCCAACCTAATGATACATGCATCGTTTCTGGCTGGGGACGAGAAAAA
GATAACGAAAGAGTCTTTTCACTTCAGTGGGGTGAAGTTAAACTAATAAGCAACTGCTCTAAGTTTTA
CGGAAATCGTTTCTATGAAAAAGAAATGGAATGTGCAGGTACATATGATGGTTCCATCGATGCCTGTA
AAGGGGACTCTGGAGGCCCCTTAGTCTGTATGGATGCCAACAATGTGACTTATGTCTGGGGTGTTGTG
AGTTGGGGGGAAAACTGTGGAAAACCAGAGTTCCCAGGTGTTTACACCAAAGTGGCCAATTATTTTGA
CTGGATTAGCTACCATGTAGGAAGGCCTTTTATTTCTCAGTACAATGTATAA

### Example CFI polynucleotide sequence (SEQ ID NO: 15)

Suitably, the variant may be at least 75%, at least 80%, at least 85%, at least 90%, at least 95%, at least 96%, at least 97%, at least 98%, at least 99% identical to SEQ ID NO: 15.

### CFH

The protein-coding sequence may encode CFH, or a fragment and/or variant thereof.

Complement factor H (CFH) regulates complement activation on self cells and surfaces. CFH competes for binding of complement factor B (CFB) to C3b, acts as a cofactor for CFI-catalysed proteolytic cleavage of C3b, and accelerates the irreversible dissociation of C3bBb and C3b2Bb into their separate components. Thus, CFH not only inhibits formation of the convertases but it also shortens the lifespan of any convertase complex that forms.

CFH is a large (155 kDa) soluble glycoprotein. CFH is composed from a total of 20 domains, each containing approximately 60 amino acid residues and termed complement control protein modules (CCPs) or short consensus repeats that are joined by short linkers consisting of 3-8 residues. The CCP modules are numbered from 1-20 (from the N-terminus of the protein): CCPs 1-4 and CCPs 19-20 engage with C3b while CCPs 7 and CCPs 19-20 bind to GAGs and sialic acid.

The CFH or a fragment and/or variant thereof may be capable of binding C3b and/or C3d; and/or acting as a cofactor for the CFl-catalysed proteolytic cleavage of C3b; and/or increasing the irreversible dissociation of C3bBb and C3b2Bb into their separate components. The fragment and/or variant of CFH may retain at least 50%, 60%, 70%, 80%, 90%, 95% or 100% of the activity of native CFH. The activity of the fragment and/or variant of CFH and native CFH may be determined using any suitable method known to those of skill in the art.

Preferably, the CFH is a human CFH. An example human CFH is the CFH having the UniProtKB accession number P08603.

Suitably, the CFH may comprise or consist of the polypeptide sequence shown as SEQ ID NO: 16, or a variant which is at least 70% identical to SEQ ID NO: 16.

MRLLAKIICLMLWAICVAEDCNELPPRRNTEILTGSWSDQTYPEGTQAIYKCRPGYRSLGNVIMVCRK
GEWVALNPLRKCQKRPCGHPGDTPFGTFTLTGGNVFEYGVKAVYTCNEGYQLLGEINYRECDTDGWTN
DIPICEVVKCLPVTAPENGKIVSSAMEPDREYHFGQAVRFVCNSGYKIEGDEEMHCSDDGFWSKEKPK
CVEISCKSPDVINGSPISQKIIYKENERFQYKCNMGYEYSERGDAVCTESGWRPLPSCEEKSCDNPYI
PNGDYSPLRIKHRTGDEITYQCRNGFYPATRGNTAKCTSTGWIPAPRCTLKPCDYPDIKHGGLYHENM
RRPYFPVAVGKYYSYYCDEHFETPSGSYWDHIHCTQDGWSPAVPCLRKCYFPYLENGYNQNYGRKFVQ
GKSIDVACHPGYALPKAQTTVTCMENGWSPTPRCIRVKTCSKSSIDIENGFISESQYTYALKEKAKYQ
CKLGYVTADGETSGSITCGKDGWSAQPTCIKSCDIPVFMNARTKNDFTWFKLNDTLDYECHDGYESNT
GSTTGSIVCGYNGWSDLPICYERECELPKIDVHLVPDRKKDQYKVGEVLKFSCKPGFTIVGPNSVQCY
HFGLSPDLPICKEQVQSCGPPPELLNGNVKEKTKEEYGHSEVVEYYCNPRFLMKGPNKIQCVDGEWTT
LPVCIVEESTCGDIPELEHGWAQLSSPPYYYGDSVEFNCSESFTMIGHRSITCIHGVWTQLPQCVAID
KLKKCKSSNLIILEEHLKNKKEFDHNSNIRYRCRGKEGWIHTVCINGRWDPEVNCSMAQIQLCPPPPQ
IPNSHNMTTTLNYRDGEKVSVLCQENYLIQEGEEITCKDGRWQSIPLCVEKIPCSQPPQIEHGTINSS
RSSQESYAHGTKLSYTCEGGFRISEENETTCYMGKWSSPPQCEGLPCKSPPEISHGVVAHMSDSYQYG
EEVTYKCFEGFGIDGPAIAKCLGEKWSHPPSCIKTDCLSLPSFENAIPMGEKKDVYKAGEQVTYTCAT
YYKMDGASNVTCINSRWTGRPTCRDTSCVNPPTVQNAYIVSRQMSKYPSGERVRYQCRSPYEMFGDEE
VMCLNGNWTEPPQCKDSTGKCGPPPPIDNGDITSFPLSVYAPASSVEYQCQNLYQLEGNKRITCRNGQ
WSEPPKCLHPCVISREIMENYNIALRWTAKQKLYSRTGESVEFVCKRGYRLSSRSHTLRTTCWDGKLE
YPTCAKR

### Example CFH polypeptide sequence (SEQ ID NO: 16)

Suitably, the variant may be at least 75%, at least 80%, at least 85%, at least 90%, at least 95%, at least 96%, at least 97%, at least 98%, at least 99% identical to SEQ ID NO: 16.

An example nucleotide sequence encoding CFH is NM_000186.4. Suitably, a protein-coding sequence encoding CFH may comprise or consist of the polynucleotide sequence shown as SEQ ID NO: 17, or a variant which is at least 70% identical to SEQ ID NO: 17.

ATGAGACTTCTAGCAAAGATTATTTGCCTTATGTTATGGGCTATTTGTGTAGCAGAAGATTGCAATGA
ACTTCCTCCAAGAAGAAATACAGAAATTCTGACAGGTTCCTGGTCTGACCAAACATATCCAGAAGGCA
CCCAGGCTATCTATAAATGCCGCCCTGGATATAGATCTCTTGGAAATGTAATAATGGTATGCAGGAAG
GGAGAATGGGTTGCTCTTAATCCATTAAGGAAATGTCAGAAAAGGCCCTGTGGACATCCTGGAGATAC
TCCTTTTGGTACTTTTACCCTTACAGGAGGAAATGTGTTTGAATATGGTGTAAAAGCTGTGTATACAT
GTAATGAGGGGTATCAATTGCTAGGTGAGATTAATTACCGTGAATGTGACACAGATGGATGGACCAAT
GATATTCCTATATGTGAAGTTGTGAAGTGTTTACCAGTGACAGCACCAGAGAATGGAAAAATTGTCAG
TAGTGCAATGGAACCAGATCGGGAATACCATTTTGGACAAGCAGTACGGTTTGTATGTAACTCAGGCT
ACAAGATTGAAGGAGATGAAGAAATGCATTGTTCAGACGATGGTTTTTGGAGTAAAGAGAAACCAAAG
TGTGTGGAAATTTCATGCAAATCCCCAGATGTTATAAATGGATCTCCTATATCTCAGAAGATTATTTA
TAAGGAGAATGAACGATTTCAATATAAATGTAACATGGGTTATGAATACAGTGAAAGAGGAGATGCTG
TATGCACTGAATCTGGATGGCGTCCGTTGCCTTCATGTGAAGAAAAATCATGTGATAATCCTTATATT
CCAAATGGTGACTACTCACCTTTAAGGATTAAACACAGAACTGGAGATGAAATCACGTACCAGTGTAG
AAATGGTTTTTATCCTGCAACCCGGGGAAATACAGCAAAATGCACAAGTACTGGCTGGATACCTGCTC
CGAGATGTACCTTGAAACCTTGTGATTATCCAGACATTAAACATGGAGGTCTATATCATGAGAATATG
CGTAGACCATACTTTCCAGTAGCTGTAGGAAAATATTACTCCTATTACTGTGATGAACATTTTGAGAC
TCCGTCAGGAAGTTACTGGGATCACATTCATTGCACACAAGATGGATGGTCGCCAGCAGTACCATGCC
TCAGAAAATGTTATTTTCCTTATTTGGAAAATGGATATAATCAAAATCATGGAAGAAAGTTTGTACAG
GGTAAATCTATAGACGTTGCCTGCCATCCTGGCTACGCTCTTCCAAAAGCGCAGACCACAGTTACATG
TATGGAGAATGGCTGGTCTCCTACTCCCAGATGCATCCGTGTCAAAACATGTTCCAAATCAAGTATAG
ATATTGAGAATGGGTTTATTTCTGAATCTCAGTATACATATGCCTTAAAAGAAAAAGCGAAATATCAA
TGCAAACTAGGATATGTAACAGCAGATGGTGAAACATCAGGATCAATTACATGTGGGAAAGATGGATG
GTCAGCTCAACCCACGTGCATTAAATCTTGTGATATCCCAGTATTTATGAATGCCAGAACTAAAAATG
ACTTCACATGGTTTAAGCTGAATGACACATTGGACTATGAATGCCATGATGGTTATGAAAGCAATACT
GGAAGCACCACTGGTTCCATAGTGTGTGGTTACAATGGTTGGTCTGATTTACCCATATGTTATGAAAG
AGAATGCGAACTTCCTAAAATAGATGTACACTTAGTTCCTGATCGCAAGAAAGACCAGTATAAAGTTG
GAGAGGTGTTGAAATTCTCCTGCAAACCAGGATTTACAATAGTTGGACCTAATTCCGTTCAGTGCTAC
CACTTTGGATTGTCTCCTGACCTCCCAATATGTAAAGAGCAAGTACAATCATGTGGTCCACCTCCTGA
ACTCCTCAATGGGAATGTTAAGGAAAAAACGAAAGAAGAATATGGACACAGTGAAGTGGTGGAATATT
ATTGCAATCCTAGATTTCTAATGAAGGGACCTAATAAAATTCAATGTGTTGATGGAGAGTGGACAACT
TTACCAGTGTGTATTGTGGAGGAGAGTACCTGTGGAGATATACCTGAACTTGAACATGGCTGGGCCCA
GCTTTCTTCCCCTCCTTATTACTATGGAGATTCAGTGGAATTCAATTGCTCAGAATCATTTACAATGA
TTGGACACAGATCAATTACGTGTATTCATGGAGTATGGACCCAACTTCCCCAGTGTGTGGCAATAGAT
AAACTTAAGAAGTGCAAATCATCAAATTTAATTATACTTGAGGAACATTTAAAAAACAAGAAGGAATT
CGATCATAATTCTAACATAAGGTACAGATGTAGAGGAAAAGAAGGATGGATACACACAGTCTGCATAA
ATGGAAGATGGGATCCAGAAGTGAACTGCTCAATGGCACAAATACAATTATGCCCACCTCCACCTCAG
ATTCCCAATTCTCACAATATGACAACCACACTGAATTATCGGGATGGAGAAAAAGTATCTGTTCTTTG
CCAAGAAAATTATCTAATTCAGGAAGGAGAAGAAATTACATGCAAAGATGGAAGATGGCAGTCAATAC
CACTCTGTGTTGAAAAAATTCCATGTTCACAACCACCTCAGATAGAACACGGAACCATTAATTCATCC
AGGTCTTCACAAGAAAGTTATGCACATGGGACTAAATTGAGTTATACTTGTGAGGGTGGTTTCAGGAT
ATCTGAAGAAAATGAAACAACATGCTACATGGGAAAATGGAGTTCTCCACCTCAGTGTGAAGGCCTTC
CTTGTAAATCTCCACCTGAGATTTCTCATGGTGTTGTAGCTCACATGTCAGACAGTTATCAGTATGGA
GAAGAAGTTACGTACAAATGTTTTGAAGGTTTTGGAATTGATGGGCCTGCAATTGCAAAATGCTTAGG
AGAAAAATGGTCTCACCCTCCATCATGCATAAAAACAGATTGTCTCAGTTTACCTAGCTTTGAAAATG
CCATACCCATGGGAGAGAAGAAGGATGTGTATAAGGCGGGTGAGCAAGTGACTTACACTTGTGCAACA
TATTACAAAATGGATGGAGCCAGTAATGTAACATGCATTAATAGCAGATGGACAGGAAGGCCAACATG
CAGAGACACCTCCTGTGTGAATCCGCCCACAGTACAAAATGCTTATATAGTGTCGAGACAGATGAGTA
AATATCCATCTGGTGAGAGAGTACGTTATCAATGTAGGAGCCCTTATGAAATGTTTGGGGATGAAGAA
GTGATGTGTTTAAATGGAAACTGGACGGAACCACCTCAATGCAAAGATTCTACAGGAAAATGTGGGCC
CCCTCCACCTATTGACAATGGGGACATTACTTCATTCCCGTTGTCAGTATATGCTCCAGCTTCATCAG

TTGAGTACCAATGCCAGAACTTGTATCAACTTGAGGGTAACAAGCGAATAACATGTAGAAATGGACAA
TGGTCAGAACCACCAAAATGCTTACATCCGTGTGTAATATCCCGAGAAATTATGGAAAATTATAACAT
AGCATTAAGGTGGACAGCCAAACAGAAGCTTTATTCGAGAACAGGTGAATCAGTTGAATTTGTGTGTA
AACGGGGATATCGTCTTTCATCACGTTCTCACACATTGCGAACAACATGTTGGGATGGGAAACTGGAG
TATCCAACTTGTGCAAAAAGATAG

### Example CFH polynucleotide sequence (SEQ ID NO: 17)

Suitably, the variant may be at least 75%, at least 80%, at least 85%, at least 90%, at least 95%, at least 96%, at least 97%, at least 98%, at least 99% identical to SEQ ID NO: 17.

The CFH fragment may be a splice variant. For example, complement factor H-like protein 1 (FHL-1) is a CFH gene splice variant, which is almost identical to the N-terminal 7 domains of CFH (CCPs 1-7).

### FHL-1

The protein-coding sequence may encode FHL-1, or a fragment and/or variant thereof.

FHL-1 or a fragment and/or variant thereof may be capable of binding C3b and/or C3d. The fragment and/or variant of FHL-1 may retain at least 50%, 60%, 70%, 80%, 90%, 95% or 100% of the activity of native FHL-1. The activity of the fragment and/or variant of FHL-1 and native FHL-1 may be determined using any suitable method known to those of skill in the art.

Preferably, the FHL-1 is a human FHL-1. An example human FHL-1 is the FHL-1 having the NCBI Reference Sequence: NP_001014975.1.

Suitably, the FHL-1 may comprise or consist of the polypeptide sequence shown as SEQ ID NO: 18, or a variant which is at least 70% identical to SEQ ID NO: 18.

MRLLAKIICLMLWAICVAEDCNELPPRRNTEILTGSWSDQTYPEGTQAIYKCRPGYRSLGNVIMVCRK
GEWVALNPLRKCQKRPCGHPGDTPFGTFTLTGGNVFEYGVKAVYTCNEGYQLLGEINYRECDTDGWTN
DIPICEVVKCLPVTAPENGKIVSSAMEPDREYHFGQAVRFVCNSGYKIEGDEEMHCSDDGFWSKEKPK
CVEISCKSPDVINGSPISQKIIYKENERFQYKCNMGYEYSERGDAVCTESGWRPLPSCEEKSCDNPYI
PNGDYSPLRIKHRTGDEITYQCRNGFYPATRGNTAKCTSTGWIPAPRCTLKPCDYPDIKHGGLYHENM
RRPYFPVAVGKYYSYYCDEHFETPSGSYWDHIHCTQDGWSPAVPCLRKCYFPYLENGYNQNHGRKFVQ
GKSIDVACHPGYALPKAQTTVTCMENGWSPTPRCIRVSFTL

### Example FHL-1 polypeptide sequence (SEQ ID NO: 18)

Suitably, the variant may be at least 75%, at least 80%, at least 85%, at least 90%, at least 95%, at least 96%, at least 97%, at least 98%, at least 99% identical to SEQ ID NO: 18.

An example nucleotide sequence encoding FHL-1 is NM_001014975.2. Suitably, a protein-coding sequence encoding FHL-1 may comprise or consist of the polynucleotide sequence shown as SEQ ID NO: 19, or a variant which is at least 70% identical to SEQ ID NO: 19.

ATGAGACTTCTAGCAAAGATTATTTGCCTTATGTTATGGGCTATTTGTGTAGCAGAAGATTGCAATGA
ACTTCCTCCAAGAAGAAATACAGAAATTCTGACAGGTTCCTGGTCTGACCAAACATATCCAGAAGGCA
CCCAGGCTATCTATAAATGCCGCCCTGGATATAGATCTCTTGGAAATGTAATAATGGTATGCAGGAAG
GGAGAATGGGTTGCTCTTAATCCATTAAGGAAATGTCAGAAAAGGCCCTGTGGACATCCTGGAGATAC
TCCTTTTGGTACTTTTACCCTTACAGGAGGAAATGTGTTTGAATATGGTGTAAAAGCTGTGTATACAT
GTAATGAGGGGTATCAATTGCTAGGTGAGATTAATTACCGTGAATGTGACACAGATGGATGGACCAAT
GATATTCCTATATGTGAAGTTGTGAAGTGTTTACCAGTGACAGCACCAGAGAATGGAAAAATTGTCAG
TAGTGCAATGGAACCAGATCGGGAATACCATTTTGGACAAGCAGTACGGTTTGTATGTAACTCAGGCT
ACAAGATTGAAGGAGATGAAGAAATGCATTGTTCAGACGATGGTTTTTGGAGTAAAGAGAAACCAAAG
TGTGTGGAAATTTCATGCAAATCCCCAGATGTTATAAATGGATCTCCTATATCTCAGAAGATTATTTA
TAAGGAGAATGAACGATTTCAATATAAATGTAACATGGGTTATGAATACAGTGAAAGAGGAGATGCTG
TATGCACTGAATCTGGATGGCGTCCGTTGCCTTCATGTGAAGAAAAATCATGTGATAATCCTTATATT
CCAAATGGTGACTACTCACCTTTAAGGATTAAACACAGAACTGGAGATGAAATCACGTACCAGTGTAG
AAATGGTTTTTATCCTGCAACCCGGGGAAATACAGCAAAATGCACAAGTACTGGCTGGATACCTGCTC
CGAGATGTACCTTGAAACCTTGTGATTATCCAGACATTAAACATGGAGGTCTATATCATGAGAATATG
CGTAGACCATACTTTCCAGTAGCTGTAGGAAAATATTACTCCTATTACTGTGATGAACATTTTGAGAC
TCCGTCAGGAAGTTACTGGGATCACATTCATTGCACACAAGATGGATGGTCGCCAGCAGTACCATGCC
TCAGAAAATGTTATTTTCCTTATTTGGAAAATGGATATAATCAAAATCATGGAAGAAAGTTTGTACAG
GGTAAATCTATAGACGTTGCCTGCCATCCTGGCTACGCTCTTCCAAAAGCGCAGACCACAGTTACATG
TATGGAGAATGGCTGGTCTCCTACTCCCAGATGCATCCGTGTCAGCTTTACCCTCTGA

### Example FHL-1 polynucleotide sequence (SEQ ID NO: 19)

Suitably, the variant may be at least 75%, at least 80%, at least 85%, at least 90%, at least 95%, at least 96%, at least 97%, at least 98%, at least 99% identical to SEQ ID NO: 19.

### Gene-editing agents

In preferred embodiments, the protein-coding sequence does not encode a gene-editing agent.

As used herein, a "gene-editing agent" may refer to a protein that can edit the sequence of a genome (see e.g. Raguram, A., Banskota, S. and Liu, D.R., 2022. Therapeutic in vivo delivery of gene editing agents. Cell). The gene editing reagent can be a rare-cutting endonuclease, including a meganuclease, a zinc finger nuclease, a TAL effector endonuclease, or a CRISPR endonuclease.

In some embodiments, the protein-coding sequence does not encode a nuclease. A "nuclease" is an enzyme that can cleave the phosphodiester bond present within a polynucleotide chain. Suitably, the nuclease is an endonuclease. Endonucleases are capable of breaking the bond from the middle of a chain.

In some embodiments, the protein-coding sequence does not encode an RNA-guided nuclease. An "RNA-guided nuclease" is a nuclease which can be directed to a specific site by a guide RNA (see e.g. Murugan, K., et al., 2017. Molecular cell, 68(1), pp.15-25). RNA-guided nucleases include, but are not limited to, Type II CRISPR nucleases such as Cas9, and Type V CRISPR nucleases such as Cas12a and Cas12b, as well as other nucleases derived therefrom. RNA-guided nucleases can be defined, in broad terms, by their PAM specificity and cleavage activity.

In some embodiments, the protein-coding sequence does not encode a Type II CRISPR nuclease. In some embodiments, the protein-coding sequence does not encode a Cas9 nuclease. Cas9 is a dual RNA-guided endonuclease enzyme associated with the Clustered Regularly Interspaced Short Palindromic Repeats (CRISPR) adaptive immune system. Cas9 nucleases include the well-characterized ortholog from Streptococcus pyogenes (SpCas9). SpCas9 and other orthologs (including SaCas9, FnCa9, and AnaCas9) have been reviewed by Jiang, F. and Doudna, J.A., 2017. Annual review of biophysics, 46, pp.505-529.

The term "gene-editing agent" may also include guide RNAs. A "guide RNA" (gRNA) confers target sequence specificity to a RNA-guided nuclease. Guide RNAs are non-coding short RNA sequences which bind to the complementary target DNA sequences. For example, in the CRISPR/Cas9 system, guide RNA first binds to the Cas9 enzyme and the gRNA sequence guides the resulting complex via base-pairing to a specific location on the DNA, where Cas9 performs its nuclease activity by cutting the target DNA strand. The term "guide RNA" encompasses any suitable gRNA that can be used with any RNA-guided nuclease, and not only those gRNAs that are compatible with a particular nuclease such as Cas9.

### Regulatory elements

The viral vector of the invention may comprise one or more regulatory sequences which may act pre- or post-transcriptionally. Suitably, a protein-coding sequence may be operably linked to one or more regulatory sequences. The one or more regulatory sequences may facilitate expression of the protein in podocytes.

"Regulatory sequences" are any sequences which facilitate expression of the polypeptides, e.g. act to increase expression of a transcript or to enhance mRNA stability. Suitable regulatory sequences include for example promoters, enhancer elements, post-transcriptional regulatory elements and polyadenylation sites.

### Promoters

The viral vector of the invention may comprise a promoter. Suitably, the promoter may be operably linked to a protein-coding sequence. The promoter may facilitate expression of the protein in podocytes.

A "promoter" is a region of DNA that leads to initiation of transcription of a gene. Promoters are located near the transcription start sites of genes, upstream on the DNA (towards the 5' region of the sense strand). Any suitable promoter may be used, the selection of which may be readily made by the skilled person.

The promoter may be a constitutive promoter or a tissue-specific promoter.

Suitable constitutive promoters will be known to the skilled person. For example, in one embodiment the promoter is a CMV promoter.

Preferably, the viral vector of the invention comprises a podocyte-specific promoter. Suitably, a protein-coding sequence is operably linked to the podocyte-specific promoter.

As used herein, a "podocyte-specific promoter" is a promoter which preferentially facilitates expression of a gene in podocyte cells. Suitably, a podocyte-specific promoter may facilitate higher expression of a gene in podocytes as compared to other cell-types. Higher expression in podocytes may be measured for example by measuring the expression of a transgene, e.g. GFP, operably linked to the promoter, wherein expression of the transgene in podocytes correlates with the ability of the promoter to facilitate expression of a gene in podocytes. For example, a podocyte-specific promoter may be a promoter which facilitates gene expression levels at least 10% higher, at least 20% higher, at least 30% higher, at least 40% higher, at least 50% higher, at least 100% higher, at least 200% higher, at least 300% higher, at least 400% higher, at least 500% higher, or at least 1000% higher in podocytes compared to expression levels in other cell types.

Suitable podocyte-specific promoters will be well known to those of skill in the art.

Suitably, the podocyte-specific promoter may be or may be derived from a promoter associated with a gene with selective expression in human podocytes. Genes selectively expressed in podocytes will be known to those of skill in the art and selective gene expression in podocytes can be readily determined by methods know to those of skill in the art, for instance with microarrays. Genes selectively expressed in podocytes include NPHS1, NPHS2, WT1, FOXC2, ABCA9, ACPP, ACTN4, ADM, ANGPTL2, ANXA1, ASB15, ATP8B1, B3GALT2, BB014433, BMP7, C1QTNF1, CAR13, CD2AP, CD55, CD59A, CD59B, CDC14A, CDH3, CDKN1B, CDKN1C, CEP85L, CLIC3, CLIC5, COL4A1, COL4A2, COL4A3, COL4A4, COL4A5, COLEC12, CRIM1, CST12, DEGS1, DOCK4, DOCK5, EGF, ENPEP, EPHX1, FAM81A, FAT1, FGFBP1, FOXD1, FRYL, GABRB1, GALC, GM10554, H2-D1, H2-Q7, H2BC4, H3C15, HS3ST3A1, HTRA1, IFNGR1, IL18, ILDR2, ITGB5, ITGB8, KIRREL, LAMA1, LAMA5, LAMB1, LAMB2, LMX1B, MAFB, MAGI2, MELA, MERTK, MGAT4A, MYO1D, MYO1E, MYOM2, MYZAP, NEBL, NES, NOD1, NPR3, NR2F2, NUPR1, OPTN, P3H2, PAK1, PARD3B, PDPN, PLAT, PLCE1, PLSCR2, PODXL, PROS1, PTPRO, RAB3B, RDH1, RDH9, SDC4, SEMA3E, SERPINB6B, SH3BGRL2, SLC41A2, SLCO2A1, ST3GAL6, SYNPO, TDRD5, THSD7A, TIMP3, TJP1, TLR7, TM4SF1, TMEM108, TMEM54, TMTC1, TOP1MT, TRAV10, TRAV10N, TRAV5-4, TSHB, UACA, UBA1Y, UPRT, VEGFA, VTCN1, ZBTB20, and 5730407I07RIK.

Methods to identify the promoter regions associated with genes will be well known to those of skill in the art. The promoter is usually located just proximal to or overlapping the transcription initiation site and contains several sequence motifs with which transcription factors (TFs) interact in a sequence-specific manner.

Suitably, the podocyte-specific promoter is selected from a NPHS1 promoter, a NPHS2 promoter, a WT1 promoter, a FOXC2 promoter, a ABCA9 promoter, a ACPP promoter, a ACTN4 promoter, a ADM promoter, a ANGPTL2 promoter, a ANXA1 promoter, a ASB15 promoter, a ATP8B1 promoter, a B3GALT2 promoter, a BB014433 promoter, a BMP7 promoter, a C1QTNF1 promoter, a CAR13 promoter, a CD2AP promoter, a CD55 promoter, a CD59A promoter, a CD59B promoter, a CDC14A promoter, a CDH3 promoter, a CDKN1B promoter, a CDKN1C promoter, a CEP85L promoter, a CLIC3 promoter, a CLIC5 promoter, a COL4A1 promoter, a COL4A2 promoter, a COL4A3 promoter, a COL4A4 promoter, a COL4A5 promoter, a COLEC12 promoter, a CRIM1 promoter, a CST12 promoter, a DEGS1 promoter, a DOCK4 promoter, a DOCK5 promoter, a EGF promoter, a ENPEP promoter, a EPHX1 promoter, a FAM81A promoter, a FAT1 promoter, a FGFBP1 promoter, a FOXD1 promoter, a FRYL promoter, a GABRB1 promoter, a GALC promoter, a GM10554 promoter, a H2-D1 promoter, a H2-Q7 promoter, a H2BC4 promoter, a H3C15 promoter, a HS3ST3A1 promoter, a HTRA1 promoter, a IFNGR1 promoter, a IL18 promoter, a ILDR2 promoter, a ITGB5 promoter, a ITGB8 promoter, a KIRREL promoter, a LAMA1 promoter, a LAMA5 promoter, a LAMB1 promoter, a LAMB2 promoter, a LMX1B promoter, a MAFB promoter, a MAGI2 promoter, a MELA promoter, a MERTK promoter, a MGAT4A promoter, a MYO1D promoter, a MYO1E promoter, a MYOM2 promoter, a MYZAP promoter, a NEBL promoter, a NES promoter, a NOD1 promoter, a NPR3 promoter, a NR2F2 promoter, a NUPR1 promoter, a OPTN promoter, a P3H2 promoter, a PAK1 promoter, a PARD3B promoter, a PDPN promoter, a PLAT promoter, a PLCE1 promoter, a PLSCR2 promoter, a PODXL promoter, a PROS1 promoter, a PTPRO promoter, a RAB3B promoter, a RDH1 promoter, a RDH9 promoter, a SDC4 promoter, a SEMA3E promoter, a SERPINB6B promoter, a SH3BGRL2 promoter, a SLC41A2 promoter, a SLCO2A1 promoter, a ST3GAL6 promoter, a SYNPO promoter, a TDRD5 promoter, a THSD7A promoter, a TIMP3 promoter, a TJP1 promoter, a TLR7 promoter, a TM4SF1 promoter, a TMEM108 promoter, a TMEM54 promoter, a TMTC1 promoter, a TOP1MT promoter, a TRAV10 promoter, a TRAV10N promoter, a TRAV5-4 promoter, a TSHB promoter, a UACA promoter, a UBA1Y promoter, a UPRT promoter, a VEGFA promoter, a VTCN1 promoter, a ZBTB20 promoter, and a 5730407I07RIK promoter, or a fragment or derivative thereof.

Suitably, the podocyte-specific promoter is selected from a NPHS1 promoter, a NPHS2 promoter, a WT1 promoter, a FOXC2 promoter, a ACTN4 promoter, a BMP7 promoter, a CD2AP promoter, a CDH3 promoter, a CDKN1B promoter, a CDKN1C promoter, a COL4A1 promoter, a COL4A2 promoter, a COL4A3 promoter, a COL4A4 promoter, a COL4A5 promoter, a CRIM1 promoter, a FAT1 promoter, a FOXD1 promoter, a KIRREL promoter, a LAMA1 promoter, a LAMA5 promoter, a LAMB1 promoter, a LAMB2 promoter, a LMX1B promoter, a MAFB promoter, a NES promoter, a NR2F2 promoter, a PODXL promoter, a PTPRO promoter, a SYNPO promoter, a TJP1 promoter, and a VEGFA promoter, or a fragment of derivative thereof.

Suitably, the podocyte-specific promoter is a NPHS1 promoter, a NPHS2 promoter, a WT1 promoter, or a FOXC2 promoter, or a fragment or derivative thereof.

Preferably, the podocyte-specific promoter is a NPHS1 or a NPHS2 promoter, or a fragment or derivative thereof. More preferably, the podocyte-specific promoter is a NPHS1 promoter, or a fragment or derivative thereof.

The podocyte-specific promoter may be a minimal podocyte-specific promoter. As used, herein, a "minimal podocyte-specific promoter" means the minimal sequence that can act as a podocyte-specific promoter.

Preferably, the podocyte-specific promoter is a minimal NPHS1 or a minimal NPHS2 promoter, or a fragment or derivative thereof. More preferably, the podocyte-specific promoter is a minimal NPHS1 promoter, or a fragment or derivative thereof.

Preferably, the promoter is a human promoter, e.g. a minimal human NPHS1 promoter.

### NPHS1 promoter

The viral vector of the invention may comprise a NPHS1 promoter, or a fragment or derivative thereof. Suitably, the NPHS1 promoter, or fragment or derivative thereof, may be operably linked to a protein-coding sequence.

The NPHS1 gene encodes nephrin, which is selectively expressed in podocytes.

The NPHS1 promoter may be a minimal NPHS1 promoter. For example, the NPHS1 promoter may have a length of 1.2 kb or less.

A minimal human NPHS1 promoter has been described in Moeller et al. 2002 J Am Soc Nephrol, 13(6):1561-7 and Wong MA et al. 2000 Am J Physiol Renal Physiol, 279(6):F1027-32. This minimal NPHS1 is a 1.2kb fragment and appears to be podocyte-specific. The 1.2kb promoter region lacks a TATA box, but has recognition motifs for other transcription factors e.g. PAX-2 binding element, E-box and GATA consensus sequences.

Suitably, the NPHS1 promoter may comprise or consist of the nucleotide sequence shown as SEQ ID NO: 20, or a variant which is at least 70% identical to SEQ ID NO: 20.

cacctgaggtcaggagttcgagaccagcgtggccaacatgatgaaaccccgtctctagtaaaaataca
aaaattagccaggcatggtgctatatacctgtagcaccagctacttgggagacagaggtgggagaatt
acttgaacctgggaggttcaagccatgggaggtggaagttgcagtgagccgagatgccactgcactcc
agcctgagcaacagagcaagactatctcaagaaaagaaagaaagaaagaaagagacttgccaaggtca
tgtatcagggcaaggaagagctgggggcccagctggctgctcccctgctgagctgggagaccaccttg
atctgacttctcccatcttcccagcctaagccaggccctggggtcacggaggctggggaggcaccgag
gaacgcgcctggcatgtgctgacaggggatttatgctccagctggccagctgggaggagcctgctg
ggcagaggccagagctgggggctctggaaggtacctgggggaggttgcactgtgagaatgagctcaag
ctgggtcagagagcaggctgactctgccagtgcctgcatcagcctcatcgctctcctaggctcctgg
cctgctggactctgggctgcaggtccttcttgaaaggctgtgagtagtgagacaaggagcaggagtga
ggggtggcaggagagaagatagagattgagagagagagagagagagagacagagagagaggaagagac
agagacaaaaggagagagaacggcttagacaaggagagaaagatggaaagataaagagactggcgca
gtggctcacgcctgtaatcccaacacttgggaggccaaggtgggaggatggcttgaaggaaagagtc
tgagatcaacctggccaacatagtgagaccccgtctctaaaaaaaaaagaaaaaaaaaagaaaaaaga
aaaaaaagtttttttaaagagacagagaaagagactcagagattgagactgagagcaagacagagaga
gatactcacaggaagaggggaagaggaaaacgagaaaggaggagagtaacggaaagagataaaaaa
gaaaagcaggtggcagagacacacagagaggacccagagaaagccagacagacgcaggtggctggca
gcgggcgctgtgggggtcacagtaggggacctgtg

### Example minimal NPHS1 promoter (SEQ ID NO: 20)

Suitably, the variant may be at least 75%, at least 80%, at least 85%, at least 90%, at least 95%, at least 98% or at least 99% identical to SEQ ID NO: 20.

In some embodiments, the NPHS1 promoter may comprise or consist of the nucleotide sequence shown as SEQ ID NO: 21, or a variant which is at least 70% identical to SEQ ID NO: 21.

GGCCCTGGGGTCACGGAGGCTGGGAGGCACCGAGGAACGCGCCTGGCATGTGCTGACAGGGGATTTT
ATGCTCCAGGAGCAAGACAGAGAGAGATACTCACAGGGAAGAGGGAAGAGGAAAACGAGAAAGGGAG
GAGAGTAACGGAAAGAGATAAAAAAGAAAAGCAGGTGGCAGAGACACACAGAGAGGGACCCAGAGAAA
GCCAGACAGACGCAGGTGGCTGGCAGCGGGCGCTGTGGGGGTCACAGTAGGGGGACCTGTG

### Example minimal NPHS1 promoter - 265 bp (SEQ ID NO: 21)

Suitably, the variant may be at least 75%, at least 80%, at least 85%, at least 90%, at least 95%, at least 98% or at least 99% identical to SEQ ID NO: 21. The NPHS1 promoter may comprise or consist of a variant of SEQ ID NO: 21 shown as SEQ ID NO: 22 or SEQ ID NO: 23. 
GGCCCTGGGGTCACGGAGGCTGGGAGGCACCGAGGAACGCGCCTGGCATGTGCTGACAGGGAATTTT
ATGCTCCAGGAGCAAGACAGAGAGAGACACTCACAGGGAAGAGGGGAAGAGGAAAACGAGAAAGGGAG
GAGAGTAACGGAAAGAGATAAAAAAGAAAAGCAGGTGGCAGAGACACAGAGAGAGGGACCCAGAGAAA
GCCAGACAGACGCAGGTGGCTGGCAGCGGGCGCTGTGGGGGTCACAGTAGGGGGACCTGTC
Example minimal nephrin promoter - 265 bp (SEQ ID NO: 22) 
GGCCCTGGGGTCACGGAGGCTGGGAGGCACCGAGGAACGCGCCTGGCATGTGCTGACAGGGGATTTT
ATGCTCCAGGAGCAAGACAGAGAGAGATACTCACAGGGAAGAGGGAAGAGGAAAACGAGAAAGGGAG
GAGAGTAACGGAAAGAGATAAAAAAGAAAAGCAGGTGGCAGAGACACAGAGAGAGGGACCCAGAGAAA
GCCAGACAGACGCAGGTGGCTGGCAGCGGGCGCTGTGGGGGTCACAGTAGGGGGACCTGTC
Exemplary minimal nephrin promoter variant - 265 bp (SEQ ID NO: 23)

In some embodiments, the NPHS1 promoter may comprise or consist of the nucleotide sequence shown as SEQ ID NO: 22 or 23, or a variant which is at least 70% identical to SEQ ID NO: 22 or 23. Suitably, the variant may be at least 75%, at least 80%, at least 85%, at least 90%, at least 95%, at least 98% or at least 99% identical to SEQ ID NO: 22 or 23.

### NPHS2 promoter

The viral vector of the invention may comprise a NPHS2 promoter, or a fragment or derivative thereof. Suitably, the NPHS2 promoter, or fragment or derivative thereof, may be operably linked to a protein-coding sequence.

The NPHS2 gene encodes podocin, which is selectively expressed in podocytes.

The NPHS2 promoter may be a minimal NPHS2 promoter. For example, the NPHS1 promoter may have a length of 0.6 kb or less.

A minimal human NPHS2 promoter has been described in Oleggini R, et al., 2006. Gene Expr. 13(1):59-66. This minimal NPHS2 is a 630bp fragment which has shown expression in podocytes *in vitro.*

Suitably, the NPHS2 promoter may comprise or consist of the nucleotide sequence shown as SEQ ID NO: 24, or a variant which is at least 70% identical to SEQ ID NO: 24.

ggaaagttggggatgaggcgaaatttctgattttaccttaaagtgaccctaattcgatgaccttttgt
ggtttttttctttttttctttttttctttttttacttggccctgcccaagcaggacctaaaaacaaacaga
caaaaaaggttactaacaactgttcctctccacgaaaatctgcagtaaaaggtaaaagatgtattcgt
tttgaagagaaaccagagcttgcgatgagcttctgtatctccgtcagccctctagcatgacattagga
accctccaggagatgagtcttcacagcccgggttggcacctgcagacacgcacttttcaacgcccgca
ccctgcccggggccggctctcccacccaggcctctctctgcttcagcgccgccccggccgtgggagtc
ggcgggcgcagtccacagctccaccaagacacagctgtcggggttccggtgcgcccgcccgcggcc
ccggtgtcccgcccctcgccctcagcccccacccgacggtctttaggtcccccgggcacgccacgcg
gacccgcagcgactccacaggactgcgctcccgtgcccctagcgctcccgcgctgctgctccagccg
cccggcagctctgacc

### Example minimal NPHS2 promoter (SEQ ID NO: 24)

Suitably, the variant may be at least 75%, at least 80%, at least 85%, at least 90%, at least 95%, at least 98% or at least 99% identical to SEQ ID NO: 24.

### Enhancers

The viral vector of the invention may comprise an enhancer. Suitably, the enhancer may be operably linked to a protein-coding sequence. The enhancer may facilitate expression of the protein in podocytes.

An "enhancer" is a region of DNA that can be bound by proteins (activators) to increase the likelihood that transcription of a particular gene will occur. Enhancers are cis-acting. They can be located up to 1 Mbp (1,000,000 bp) away from the gene, upstream or downstream from the start site. Any suitable enhancer may be used, the selection of which may be readily made by the skilled person.

The viral vector of the invention may comprise a podocyte-specific enhancer. Suitably, the enhancer may be operably linked to a protein-coding sequence.

As used herein, a "podocyte-specific enhancer" is an enhancer which preferentially facilitates expression of a gene in podocyte cells. Suitably, a podocyte-specific enhancer may facilitate higher expression of a gene in podocytes as compared to other cell-types. Higher expression in podocytes may be measured for example by measuring the expression of a transgene, e.g. GFP, operably linked to the enhancer, wherein expression of the transgene in podocytes correlates with the ability of the enhancer to facilitate expression of a gene in podocytes. For example, a podocyte-specific enhancer may be an enhancer which facilitates gene expression levels at least 10% higher, at least 20% higher, at least 30% higher, at least 40% higher, at least 50% higher, at least 100% higher, at least 200% higher, at least 300% higher, at least 400% higher, at least 500% higher, or at least 1000% higher in podocytes compared to expression levels in other cell types.

Suitable podocyte-specific enhancer will be well known to those of skill in the art.

Suitably, the podocyte-specific enhancer may be or may be derived from an enhancer associated with a gene with selective expression in human podocytes. Methods to identify the enhancer regions associated with genes will be well known to those of skill in the art.

Preferably, the podocyte-specific enhancer is a NPHS1 or a NPHS2 enhancer, or a fragment or derivative thereof. More preferably, the podocyte-specific enhancer is a NPHS1 enhancer, or a fragment or derivative thereof.

Preferably, the enhancer is a human enhancer, e.g. a human NPHS1 enhancer.

The enhancer may be used with the corresponding promoter, for example the NPHS1 enhancer may be used with the NPHS1 promoter. Alternatively, the enhancer may be used with a different promoter, for example a promoter which is not podocyte-specific e.g. hsp promoter.

The viral vector of the invention may comprise a promoter-enhancer. Suitably, the promoter-enhancer may be operably linked to a protein-coding sequence. The promoter-enhancer may facilitate expression of the protein in podocytes. The promoter-enhancer may be a podocyte-specific promoter-enhancer. The promoter-enhancer may be a NPHS1 promoter-enhancer or a NPHS2 promoter-enhancer, or a fragment or derivative thereof.

### NPHS1 enhancer

A NPHS1 enhancer has been described in Guo, G., et al., 2004. Journal of the American Society of Nephrology, 15(11), pp.2851-2856. A 186-bp fragment from the human NPHS1 promoter was capable of directing podocyte-specific expression of a β-galactosidase transgene when placed in front of a heterologous minimal promoter in transgenic mice.

Suitably, the NPHS1 enhancer may comprise or consist of the nucleotide sequence shown as SEQ ID NO: 25, or a variant which is at least 70% identical to SEQ ID NO: 25.

ctgctgagctgggagaccaccttgatctgacttctcccatcttcccagcctaagccaggccctggggt
cacggaggctggggaggcaccgaggaacgcgcctggcatgtgctgacaggggattttatgctccagct
gggccagctgggaggagcctgctgggcagaggccagagctgggggctctg

### Example NPHS1 enhancer (SEQ ID NO: 25)

Suitably, the variant may be at least 75%, at least 80%, at least 85%, at least 90%, at least 95%, at least 98% or at least 99% identical to SEQ ID NO: 25.

### Kozak sequence

The viral vector of the invention may comprise a Kozak sequence. Suitably, the Kozak sequence may be operably linked to a protein-coding sequence. A Kozak sequence may be inserted before the start codon of the protein to improve the initiation of translation.

Suitable Kozak sequences will be well known to those of skill in the art.

Suitably, the Kozak sequence may comprise or consist of the nucleotide sequence shown as SEQ ID NO: 26, or a variant which is at least 65% identical to SEQ ID NO: 26.
GCCGCCACCAUGG

### Example Kozak sequence (SEQ ID NO: 26)

Suitably, the variant may be at least 75%, at least 85%, or at least 90% identical to SEQ ID NO: 26.

### Post-transcriptional regulatory elements

The viral vector of the invention may comprise a post-transcriptional regulatory element. Suitably, the post-transcriptional regulatory element may be operably linked to a protein-coding sequence. The post-transcriptional regulatory element may improve gene expression.

The viral vector may comprise a Woodchuck Hepatitis Virus Post-transcriptional Regulatory Element (WPRE). Suitably, the WPRE may be operably linked to a protein-coding sequence.

The WPRE sequence may have mutations within the X-antigen promoter and/or the initiation codon of the X-antigen. This may prevent the production of a functional X-antigen.

Suitably, the WPRE may comprise or consist of the nucleotide sequence shown as SEQ ID NO: 27, or a variant which is at least 70% identical to SEQ ID NO: 27.

aatcaacctctggattacaaaatttgtgaaagattgactggtattcttaactatgttgctccttttac
gctatgtggatacgctgctttaatgcctttgtatcatgctattgcttcccgtatggctttcattttct
cctccttgtataaatcctggttgctgtctctttatgaggagttgtggcccgttgtcaggcaacgtggc
gtggtgtgcactgtgtttgctgacgcaacccccactggttggggcattgccaccacctgtcagctcct
ttccgggactttcgctttccccctccctattgccacggcggaactcatcgccgcctgccttgcccgct
gctggacaggggctcggctgttgggcactgacaattccgtggtgttgtcggggaaatcatcgtccttt
ccttggctgctcgcctgtgttgccacctggattctgcgcgggacgtccttctgctacgtcccttcggc
cctcaatccagcggaccttccttcccgcggcctgctgccggctctgcggcctcttccgcgtcttcgcc
ttcgccctcagacgagtcggatctccctttgggccgcctccccgc

### Example WPRE (SEQ ID NO: 27)

Suitably, the variant may be at least 75%, at least 80%, at least 85%, at least 90%, at least 95%, at least 98%, at least 99% identical to SEQ ID NO: 27.

### Polyadenylation signal

The viral vector of the invention may comprise a polyadenylation signal. Suitably, the polyadenylation signal may be operably linked to a protein-coding sequence. The polyadenylation signal may improve gene expression.

Suitable polyadenylation signals include the early SV40 polyadenylation signal (SV40pA), a bovine growth hormone polyadenylation signal (bGH), or a soluble neuropilin-1 polyadenylation signal. Preferably, the polyadenylation signal is a bGH polyadenylation signal or a soluble neuropilin-1 polyadenylation signal.

Suitably, the polyadenylation signal may comprise or consist of the nucleotide sequence shown as SEQ ID NO: 28, or a variant which is at least 70% identical to SEQ ID NO: 28.

ctgtgccttctagttgccagccatctgttgtttgcccctcccccgtgccttccttgaccctggaaggt
gccactcccactgtcctttcctaataaaatgaggaaattgcatcgcattgtctgagtaggtgtcattc
tattctggggggtggggtggggcaggacagcaagggggaggattgggaagacaatagcaggcatgctg
gggatgcggtgggctctatgg

### Example bGH poly(A) signal sequence (SEQ ID NO: 28)

Suitably, the variant may be at least 75%, at least 80%, at least 85%, at least 90%, at least 95%, at least 98%, at least 99% identical to SEQ ID NO: 28.

Suitably, the polyadenylation signal may comprise or consist of the nucleotide sequence shown as SEQ ID NO: 29, or a variant which is at least 70% identical to SEQ ID NO: 29.
aaataaaatacgaaatg

Example soluble neuropilin-1 polyadenylation signal (SEQ ID NO: 29) Suitably, the variant may be at least 75%, at least 80%, at least 85%, at least 90%, at least 95%, at least 98%, at least 99% identical to SEQ ID NO: 29.

### Methods of treatment

The present invention provides a viral vector for use in a method of treating a human subject that has or is at risk of a kidney disease, wherein the viral vector is delivered by the method according to the present invention.

For example, the kidney disease may be a glomerular disease. For example, the kidney disease may be a podocyte-associated glomerular disease. For example, the kidney disease may be a GBM-associated glomerular disease.

The kidney disease may be a genetic kidney disease (see e.g. Hildebrandt, F., 2010. The Lancet, 375(9722), pp.1287-1295). The viral vector may correspond to the kidney disease which is intended to be treated and/or prevented. For example, when the kidney disease is Alport Syndrome, the viral vector may encode COL4A3, COL4A4 or COL4A5. For example, when the kidney disease is nephrotic syndrome, the viral vector may encode a NS-associated transgene. For example, when the kidney disease is complement-associated, the viral vector may encode a complement protein.

In some embodiments, the kidney disease is a genetic glomerular disease. Genetic glomerular diseases include podocyte-associated genetic glomerular diseases, such as nephrotic syndrome, and GBM-associated glomerular diseases, such as Alport Syndrome.

In some embodiments, the kidney disease is a podocyte-associated genetic glomerular disease. Podocyte-associated genetic glomerular diseases include Congenital nephrotic syndrome of the Finnish type, Congenital nephrotic syndrome type 2, Familial nephrotic syndrome type 3, Frasier syndrome and Denys-Drash syndrome, Schimke immuno-osseous dysplasia, Nephrotic syndrome caused by mutations in CD2AP, Nephrotic syndrome caused by mutations in actinin-4, Nephrotic syndrome caused by mutations in TRPC6, and Epstein and Fechtner syndrome. Suitably, the glomerular disease is nephrotic syndrome.

In some embodiments, the kidney disease is a GBM-associated genetic glomerular disease. GBM-associated genetic glomerular diseases include X-linked Alport syndrome, Autosomal recessive Alport syndrome, Autosomal dominant Alport syndrome, Thin basement membrane diseases, Pierson syndrome, and Nail-patella syndrome. Suitably, the glomerular disease is Alport syndrome (AS). AS is also known as familial nephritis, hereditary nephritis, thin basement membrane disease and thin basement membrane nephropathy.

In some embodiments, the kidney disease is a complement-mediated kidney disease. Exemplary complement-mediated kidney diseases include IgA nephropathy, C3 glomerulopathy, atypical hemolytic uremic syndrome (aHUS), stx-associated HUS, lupus nephritis, cryoglobulinemia, anti-GBM disease, ANCA-associated vasculitis, bacterial endocarditis, post-infectious glomerulonephritis, antibody-mediated rejection of renal transplant, membranous nephropathy, membranoproliferative glomerulonephritis I, or membranoproliferative glomerulonephritis III.

In some embodiments, the kidney disease is diabetic nephropathy.

In some embodiments, the kidney disease is Fabry disease.

### Variants, derivatives, analogues, homologues and fragments

In addition to the specific proteins and nucleotides mentioned herein, the invention also encompasses variants, derivatives, homologues and fragments thereof.

In the context of the invention, a "variant" of any given sequence is a sequence in which the specific sequence of residues (whether amino acid or nucleic acid residues) has been modified in such a manner that the polypeptide or polynucleotide in question retains at least one of its endogenous functions. A variant sequence can be obtained by addition, deletion, substitution, modification, replacement and/or variation of at least one residue present in the naturally occurring polypeptide or polynucleotide.

The term "derivative" as used herein in relation to proteins or polypeptides of the invention includes any substitution of, variation of, modification of, replacement of, deletion of and/or addition of one (or more) amino acid residues from or to the sequence, providing that the resultant protein or polypeptide retains at least one of its endogenous functions.

Typically, amino acid substitutions may be made, for example from 1, 2 or 3, to 10 or 20 substitutions, provided that the modified sequence retains the required activity or ability. Amino acid substitutions may include the use of non-naturally occurring analogues.

Proteins used in the invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

Conservative substitutions may be made, for example according to the table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R H |
| AROMATIC | | F W Y |

The term "homologue" as used herein means a variant having a certain homology with the wild type amino acid sequence or the wild type nucleotide sequence. The term "homology" can be equated with "identity".

In the present context, a homologous sequence is taken to include an amino acid sequence which may be at least 50%, 55%, 65%, 75%, 85% or 90% identical, preferably at least 95%, 96% or 97% or 98% or 99% identical to the subject sequence. Typically, the homologues will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

In the present context, a homologous sequence is taken to include a nucleotide sequence which may be at least 50%, 55%, 65%, 75%, 85% or 90% identical, preferably at least 95%, 96% or 97% or 98% or 99% identical to the subject sequence. Although homology can also be considered in terms of similarity, in the context of the present invention it is preferred to express homology in terms of sequence identity.

Preferably, reference to a sequence which has a percent identity to any one of the SEQ ID NOs detailed herein refers to a sequence which has the stated percent identity over the entire length of the SEQ ID NO referred to.

Homology comparisons can be conducted by eye, or more usually, with the aid of readily available sequence comparison programs. These commercially available computer programs can calculate percent homology or identity between two or more sequences.

Percent homology may be calculated over contiguous sequences, i.e. one sequence is aligned with the other sequence and each amino acid or nucleotide in one sequence is directly compared with the corresponding amino acid or nucleotide in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the amino acid or nucleotide sequence may cause the following residues or codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids or nucleotides, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example, when using the GCG Wisconsin Bestfit package the default gap penalty for amino acid sequences is -12 for a gap and -4 for each extension.

Calculation of maximum percent homology therefore firstly requires the production of an optimal alignment, taking into consideration gap penalties. A suitable computer program for carrying out such an alignment is the GCG Wisconsin Bestfit package (University of Wisconsin, USA; Devereux et al. (1984) Nucleic Acids Research 12: 387). Examples of other software that can perform sequence comparisons include, but are not limited to, the BLAST package (see Ausubel et al. (1999) ibid - Ch. 18), FASTA (Atschul et al. (1990) J. Mol. Biol. 403-410), EMBOSS Needle (Madeira, F., et al., 2019. Nucleic acids research, 47(W1), pp.W636-W641) and the GENEWORKS suite of comparison tools. Both BLAST and FASTA are available for offline and online searching (see Ausubel et al. (1999) ibid, pages 7-58 to 7-60). However, for some applications, it is preferred to use the GCG Bestfit program. Another tool, BLAST 2 Sequences, is also available for comparing protein and nucleotide sequences (FEMS Microbiol. Lett. (1999) 174(2):247-50; FEMS Microbiol. Lett. (1999) 177(1):187-8).

Although the final percent homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix (the default matrix for the BLAST suite of programs). GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62.

Once the software has produced an optimal alignment, it is possible to calculate percent homology, preferably percent sequence identity. The software typically does this as part of the sequence comparison and generates a numerical result. The percent sequence identity may be calculated as the number of identical residues as a percentage of the total residues in the SEQ ID NO referred to.

"Fragments" are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants, derivatives, homologues and fragments may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

The practice of the present invention will employ, unless otherwise indicated, conventional techniques of chemistry, biochemistry, molecular biology, microbiology and immunology, which are within the capabilities of a person of ordinary skill in the art. Such techniques are explained in the literature. See, for example: Skoog, D.A., et al. (2013) Fundamentals of Analytical Chemistry, 9th edition, Cengage learning; Walker J.M. (2009) The Protein Protocols Handbook, 3rd edition, Springer Nature; Green, M.R. and Sambrook, J. (2012) Molecular Cloning: A Laboratory Manual, 4th Edition, Cold Spring Harbor Laboratory Press; Ausubel, F.M., et al. (2003) Current Protocols in Molecular Biology, John Wiley & Sons; Hill, A. J. (2013) DNA Sequencing Protocols, Humana Press; Nielsen, B.S. and Jones, J. (2021) In Situ Hybridization Protocols, Springer US; Herdewijn, P. (2010) Oligonucleotide Synthesis: Methods and Applications, Humana Press; and Luo, Y. (2019) CRISPR Gene Editing: Methods and Protocols, Springer New York.

### EXAMPLES

The invention will now be further described by way of Examples, which are meant to serve to assist one of ordinary skill in the art in carrying out the invention and are not intended in any way to limit the scope of the invention.

### Example 1 - Direct renal artery injection of an AAV vector encoding podocin under control of a hNPHS1 promoter

This study was designed to explore kidney transduction efficiency using a rAAV product in healthy pigs when administered via direct renal artery injection (dRAi) with renal artery occlusion while also testing the biodistribution of injected rAAV in key organs and tissues.

### Materials and methods

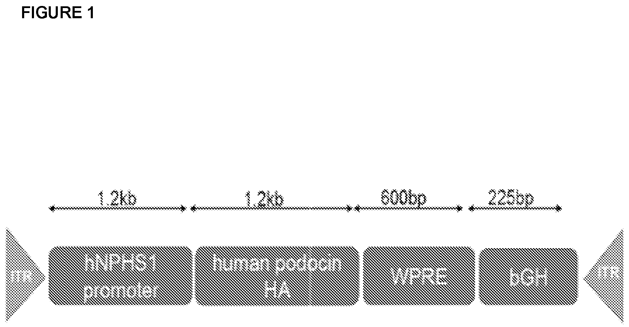

A schematic representation of an AAV vector expressing human HA-tagged podocin under the human full-length nephrin (hNPHS1) promoter is shown in **Figure 1****.** The cassette was encapsulated into AAV-LK03 serotype for study use.

The rAAV was made via a triple plasmid transfection in HEK293T cells. The virus underwent 72-hour incubation post transfection. Viral particles were purified using density gradient separation, meaning a large portion of empty capsids were eradicated/removed from the final formulation. The virus was sterile filtered through a 0.2 micron filter. The rAAV product was stored at -80°C until use. The vehicle was phosphate-buffered saline (PBS).

The dose of rAAV product administered to four male pigs is summarised in Table 1. The viral vector was dosed according to body weight (target weight at dosing was 10 kg).

**Table 1 Dosing Scheme and Treatment**

| **Animal No.** | **Treatment and Dose Route** | **Dose (adjusted for body weight)** | **Virus Formulation** | **Ischemia Time (Mins)** |
|---|---|---|---|---|
| 1 | Control | N/A | N/A | N/A |
| 2 | Control | N/A | N/A | N/A |
| 3 | dRAi gene therapy with occlusion | 7.2× 10¹³ vg/kg | 16.5 mL/kg in PBS | 15-22 |
| 4 | dRAi gene therapy with occlusion | 7.5×10¹³ vg/kg | 16.5 mL/kg in PBS | 15-22 |
| 5 | dRAi gene therapy with occlusion (pre- and post-treated with 10 mg prednisolone) | 9.6×10¹³ vg/kg | 16.5 mL/kg in PBS | 15-22 |
| 6 | dRAi gene therapy with occlusion (pre- and post-treated with 10 mg prednisolone) | 9.0×10¹³ vg/kg | 16.5 mL/kg in PBS | 15-22 |

Prior to Day 0, all animals were given prophylactic antibiotics twice daily in-feed, and 2 animals were also pretreated with 1 mg/kg prednisolone. On the day of the treatment, the test product was thawed and then made up to a maximum volume of 5 mL/kg in PBS at ambient room temperature. Standard procedures for surgery and anaesthesia, which included Hartmann's IV fluid therapy were followed.

The surgical procedure of dRAi with balloon occlusion involved accessing the left renal artery via the percutaneous route and inflating a balloon to occlude the renal artery prior to the injection of the rAAV test product. Following confirmation that the artery was occluded, the study treatment was administered for approximately 15-20 minutes distal to the balloon without using an infusion pump.

The steps involved in the final surgical procedure of rAAV test product via dRAi are summarised below:
1. Catheterisation of the carotid artery
2. Introduction of the sheath in the carotid artery
3. Introduction of the guidewire into the renal artery
4. Introduction of the occlusion balloon catheter into the renal artery
5. Balloon occlusion of the renal artery by inflating the balloon (15-20 minutes)
6. Check of occlusion with contrast injection and imaging (angiogram was from the tail vein and contrast added to show flow and recirculation)
7. Flush contrast out
8. Injection of rAAV test product directly into the renal artery
9. Flush with saline to ensure that no test product remained within the catheter
10. Deflate the balloon catheter
11. Withdraw the balloon catheter slowly to avoid damaging vessels
12. Subsequently withdraw the sheath and close

The animals were then recovered and returned to individual pens for 24 hours, then back to group pens following the removal of their carotid artery catheter. Post-anaesthesia analgesia IV buprenorphine (20 mg/kg) was given to animals numbered 3 to 6. All animals were given prophylactic antibiotics twice daily in-feed throughout the study period. Two animals were given prednisolone (10 mg) once daily until termination, and the 2 remaining animals did not receive this medication. Euthanasia was performed with sodium pentobarbital (10-15 mL) under a surgical plane of anaethesia, approximately 4 weeks post treatment.

Animals were observed daily for general health/mortality and moribundity. Clinical observations and body weights were recorded on arrival at the test facility, then Day -1, Day 0 and Weeks 1 to 4. Pain assessment scores were recorded on Days 1 through to Day 5 post treatment.

Kidney (cortex and medulla) and other tissue samples (liver, spleen, pancreas) were taken for the following assessments: Immunohistochemistry and histology; Protein western blotting; RNA; DNA; and Immunofluorescence.

### Results

This study showed that rAAV AAVLK03-hPodocin could successfully be delivered to the kidney glomerulus, and more specifically to the podocyte via dRAi+occlusion.

### Transcriptomic analysis

qPCR analysis was performed to look for the bGH expression in 2 control animals and 4 animals dRAi treated with AAV expressing HA-tagged podocin via the left renal artery to assess transduction efficiency in kidneys (by comparing injected left versus untreated right kidney) and biodistribution (pancreas, liver, spleen). Each bGH mRNA expression of the organ was normalized to the bGH expression of the same organ of the control animals.

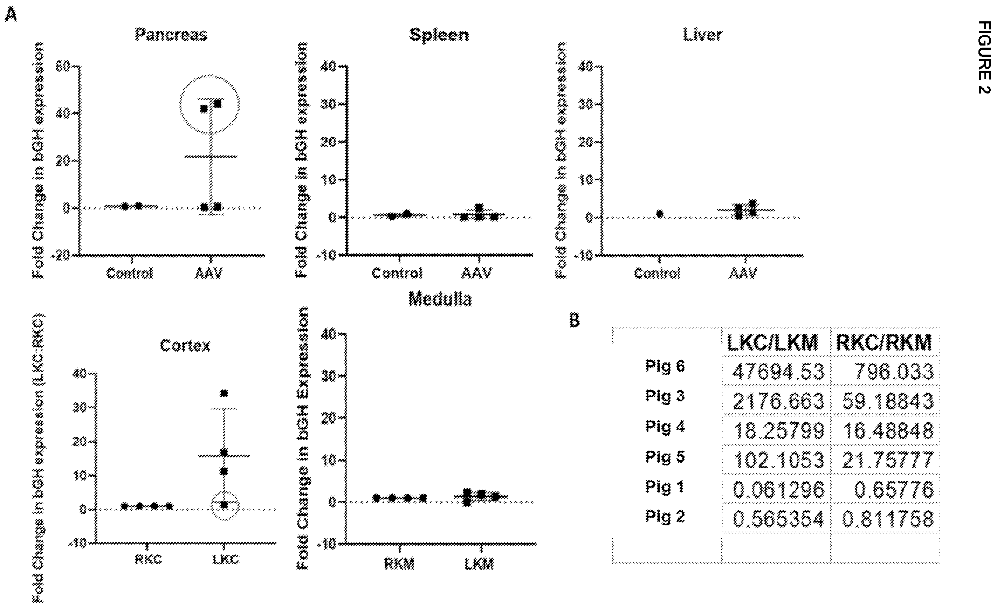

**Figure 2** shows the transcriptomic analysis, where there is an increased mRNA bGH detection in left kidney cortex (LKC) of the dRAi treated animals in comparison to right kidney cortex (RKC). There were no significant changes in detected expression levels in other organs, apart from the pancreas, where 2 of the dRAi treated animals demonstrated around 40-45-fold increase in bGH mRNA levels. When LKC was normalized to left kidney medulla (LKM) of the same animals, all 4 dRAi treated pigs shown relatively high levels of bGH expression when compared to their wild-type counterparts (2 control animals).

From data generated from the second round of the transcriptomic analysis, there is an increased mRNA bGH expression in the LKC of the injected animals compared to RKC or LKM/RKM, or the other organs. The kidney transcriptomic analysis demonstrated the following trend LKC>LKM>RKC>RKM, as anticipated.

### Immunofluorescence Studies

To visualise local penetration and persistence of the virus to the intended area glomeruli-immunofluorescence studies were performed to detect the level of HA-tagged podocin protein in glomeruli of 2 control and 4 rAAV treated animals at the point of sacrificing.

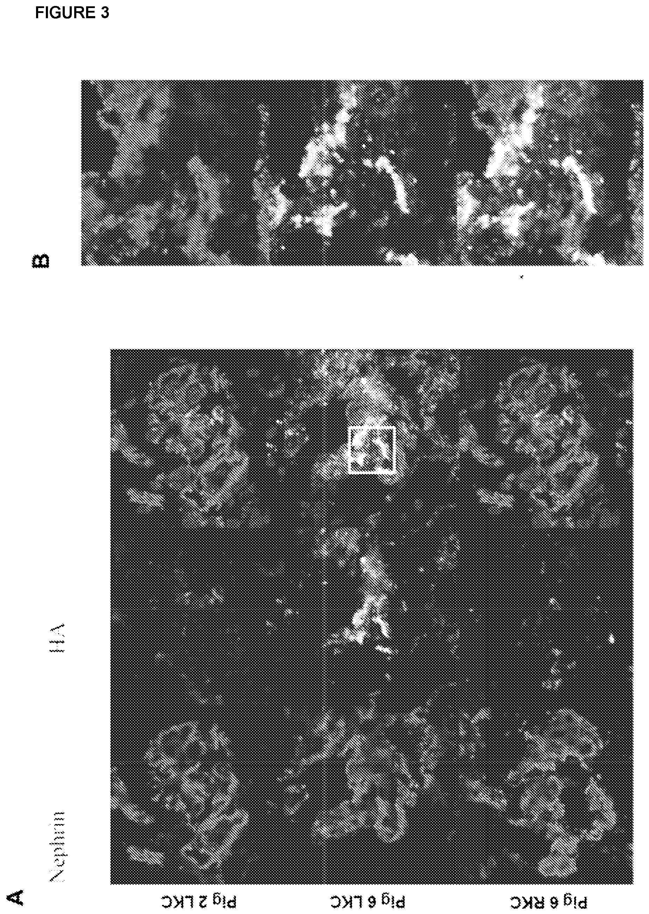

**Figure 3** demonstrates that the dRAi of the rAAV is successfully delivered, by detection of HA-tagged podocin being seen in the LKC and more specifically to the targeted cell, the podocyte, as seen by colocalization of nephrin (podocyte specific protein) and HA-tagged podocin in the middle panel (Animal number 6 LKC), when compared to the untreated (no dRAi) control animal number 2 or animal number 6 RKC. Other animals also underwent immunofluorescence analysis, however, animal number 2 LKC (control), rAAV treated animal number 6 (LKC and RKC) are merely highlighted as the representative images.

Immunofluorescence studies further confirmed the localization of HA-tagged podocin to the podocytes in the glomeruli of the injected animals compared to the uninjected or control pigs.

### In-life observations/measurements

There were no deaths during the study period. There was no AAVLK03-hPodocin related effect seen by clinical observations or monitoring in any of the treated animals over the study period. There was no AAVLK03-hPodocin related effect on body weight in any of the treated animals over the study period.

### Example 2 - Direct renal artery injection of an AAV vector encoding eGFP under control of CMV promoter

This study was designed to compare kidney transduction efficiency using a rAAV test product in healthy Göttingen pigs when administered via dRAi (with renal artery occlusion) versus IV systemic administration, while also testing the biodistribution of injected rAAV in key organs and tissues.

### Materials and methods

A schematic representation of an AAV vector expressing eGFP under the CMV promoter is shown in **Figure 4****.** eGFP is widely used as a reporter molecule for gene expression due to its ability to be detected after blue-light excitation. The expression of eGFP protein in this study was driven by human CMV major promoter, known for its ability to produce high level recombinant protein in mammalian cells. The cassette was encapsulated into AAV-LK03 serotype for study use.

The rAAV was made via a triple plasmid transfection in HEK293T cells. The virus underwent 72-hour incubation post transfection. Viral particles were purified using density gradient separation, meaning a large portion of empty capsids were eradicated/removed from the final formulation. The virus was sterile filtered through a 0.2 micron filter. The rAAV product was stored at -80°C until use. The vehicle was phosphate-buffered saline (PBS) buffer (pH7.4) supplemented with 200 mM NaCl and 0.001% Pluronic F-68 made up to a maximum volume of 15 mL.

The dose of rAAV test product administered into the 3 groups is summarised in **Table 2,** where there were 3 animals per group and 1 animal served as a control animal without receiving any rAAV test product. The viral vector was dosed according to weight and used the same fixed volume (15 mL) for all experimental animals. The total ischemia time includes the flush step as the flush is also performed under occlusion in the dRAi+O arm.

**Table 2 Dosing Scheme and Treatment**

| **Group No.** | **No. of animals/ sex** | **Treatment and Dose Route** | **Dose** | **Dose volume** | **Virus Formulation** | **Ischemia Time (Mins)** |
|---|---|---|---|---|---|---|
| 1 | 3/F | Systemic IV | High | 15 mL | PBS buffer (pH7.4) supplemented with 200 mM NaCl and 0.001% Pluronic F-68 | N/A |
| 2 | 3/F | High dose dRAi with occlusion | High | 15 mL | PBS buffer (pH7.4) supplemented with 200 mM NaCl and 0.001% Pluronic F-68 | 15 |
| 3 | 3/F | Low dose dRAi with occlusion | Low | 15 mL | PBS buffer (pH7.4) supplemented with 200 mM NaCl and 0.001% Pluronic F-68 | 15 |
| 4 | 1/F | Control, untreated | N/A | N/A | N/A | N/A |

The "High" dose was calculated as 5.7×10¹² vg per pig and the "Low" dose was calculated as 5.7×10¹¹ vg per pig.

It is expected that variability exists in renal artery anatomy and the size of the animal, thus varying the total amount of AAV required. Therefore, to add consistency Göttingen minipigs were chosen for this study, as the pigs were derived from a closed colony and interbred, while also known to show low levels of neutralising antibodies to AAV. All animals were single sex (female) 4.5 months of age and were fed a weight management diet to restrict growth to be 10-12 kg body weight prior to study treatment.

Prior to Day 0 animals were pretreated prophylactically (3 or 5 days) with amoxicillin (antibiotic). On Day 0 and prior to surgery animals were pre-anaethetised. On the day of the treatment, the test product was thawed and then made up to a maximum volume of 15 mL in PBS at ambient room temperature. Standard procedures for surgery and anaesthesia, which included Hartmann's IV fluid therapy, were followed.

The surgical procedure of dRAi with balloon occlusion involved accessing the renal artery of the first kidney via the percutaneous route and inflating an occlusion balloon catheter to occlude the renal artery for 15-20 minutes prior to the injection of the rAAV test product. Following confirmation that the artery was occluded, the study treatment was administered for approximately 15-20 minutes distal to the balloon via an infusion pump.

The steps involved in the surgical procedure of rAAV test product via dRAi+O are summarised below:
1. Catheterisation of the common femoral artery (CFA) per standard procedure
2. Introduction of the sheath in the CFA
3. Introduction of the guidewire into the renal artery
4. Introduction of the occlusion balloon catheter into the renal artery
5. Balloon occlusion of the renal artery by inflating the balloon (15-20 minutes)
6. Checked for full occlusion with contrast injection and imaging (angiogram was from the tail vein and contrast added to show flow and recirculation)
7. Flushed with Plasma-Lyte and heparin
8. Injection / infusion of rAAV test product directly into the real artery under no flow conditions by using an infusion pump
9. Flushed with saline / PBS to ensure that no rAAV test product remained within the catheter
10. Deflated the balloon catheter
11. Slow withdraw of the balloon catheter to avoid damaging vessels
12. Subsequent withdrawal of the sheath and close

Due to surgical complications (see Table 2), two animals in the low rAAV dose dRAi group (Pigs No. 2 and No. 5) were injected in the right kidney as opposed to the left kidney.

For IV of the rAAV test product a total dose was injected via jugular vein over 15 minutes. Control animals were not treated with rAAV test product. However, they were anaesthetised for a line to be added to allow for blood sample collection.

Post-anaesthesia analgesia IV buprenorphine (20 mg/kg) was given to all animals. Pig 2 received additional post-operative analgesia (Day 0) of paracetamol (10 mg/kg IV), fentanyl patch (50 mg for 3 days) and then on Day 1 paracetamol 10 mg/kg orally. Euthanasia was performed with sodium pentobarbital (10-15 mL) under a surgical plane of anaesthesia, approximately 4 weeks post treatment.

Animals were observed for general health/mortality and moribundity. Clinical observations and body weights were recorded on arrival at the test facility, then Day -1, Day 0 and Weeks 1 to 4. Pain assessment scores were recorded on Days 1 through to Day 5 post surgery. Blood samples were collected for haematology and biochemistry analysis at 3 timepoints: before intervention (0 hr), Day 1 (24 hrs) and at termination.

Kidney (cortex and medulla) and other tissue samples (liver, pancreas, colon) were taken for the following assessments: Immunohistochemistry and histology; RNA (ribonucleic acid); DNA (deoxyribonucleic acid); and Immunofluorescence. Brain, lung, heart and spleen samples were taken and stored for possible future analysis.

### Results

This study demonstrated that direct Renal Artery injection (dRAi) with occlusion results in rAAV-mediated transgene transduction and subsequent expression of GFP which is localised in the injected kidney at the glomeruli, detected by immunofluorescence.

No safety concerns related to the surgical procedure of dRAi with occlusion was observed in this study. In all animals administered using this route of administration, there was no observable damage, fibrosis, necrosis, or sclerosis after evaluation of the histology of all animal samples.

Immunofluorescence analysis showed consistent and clear GFP expression in the injected left kidney cortex (LKC) of animals, which underwent high rAAV dose dRAi. Furthermore, moderate GFP expression was seen on the injected side in the cortexes of low dose dRAi animals (Pig 2 and Pig 5 - injected into the right kidney, Pig 8 - left kidney). In comparison, no GFP expression has been seen following IV administration, in LKC or right kidney cortex (RKC) apart from Pig 6 in the high rAAV dose IV group.

The conclusions from the immunofluorescence images were further supported by review of the data from the qPCR results. These results demonstrated that there was elevated expression in (injected) LKC of the high rAAV dose dRAi group and a slight expression in the low rAAV dose dRAi group in the RKC. This analysis also demonstrated that there was no GFP mRNA expression in the pancreas and colon.

Importantly, there was no evidence of liver GFP expression either on protein or mRNA level in any animals in both the high and low rAAV dose dRAi groups. In contrast, expression on both protein and mRNA level was observed in the livers of high rAAV dose IV animals.

### Transcriptomic analysis

qPCR analysis was performed to analyse mRNA GFP expression in 1 control animal and 6 animals injected with rAAV (serotype LK03) expressing GFP driven by CMV promoter via the renal artery (dRAi - high dose and low dose) to assess transduction efficiency in animal kidneys (by comparing the rAAV injected kidney versus the contralateral uninjected kidney) and biodistribution (liver, colon, pancreas). There was ~80-fold average GFP relative change in high rAAV dose (dRAi vs IV) and a ~20-fold change in low rAAV dose (dRAi vs IV) in the injected kidney cortex, as shown in **Figure 5****.**

qPCR analysis was also conducted to analyse mRNA GFP expression in 3 animals that were administered rAAV test product via IV injection to compare the expression profile to the dRAi groups. Each GFP mRNA expression of the organ was normalized to the GFP expression from the same organ of the control animal **(****Figure 6****).** The results presented therefore compare the injected kidney with the contralateral non-injected kidney. For completeness, the transduction efficiency data via GFP from the left kidney and right kidney are presented.

### Immunofluorescence Studies

Following sectioning of the organs, 5-6 images were acquired per sample. Adrenal cortex and medulla was identified by visual examination of the bisected whole kidney and areas to section were chosen at random (1 slice per kidney). Sections shown here are a representative sample of all slices examined.

In the animals injected via the left renal artery, the majority of GFP protein expression was found in the glomeruli. In some images, GFP was seen to co-localise in focal linear distribution with nephrin, a podocyte specific marker (linear staining in focal areas suggesting co-localisation) (Pigs 1 and 8 used as an example) **(****Figure 7****).** In comparison, the high dose rAAV IV injected group demonstrated there was no kidney GFP protein expression in 2 animals, although one animal (Pig 6) showed some expression in the glomerulus, which appears to be much less than in other groups. Pigs 2 and 5 were injected via right kidney due to surgical complications. Therefore, no GFP expression seen in left cortex or medulla as expected as animals were injected in the right kidney.

To confirm the prevalent expression of the GFP in the left kidney cortex in animals injected with rAAV via the left renal artery, immunofluorescence analysis was performed in the contralateral kidney, more specifically in right kidney cortex and medulla. There was very little expression seen in the right cortexes of animals injected via the left renal artery (**Figure 8**), suggesting that the majority of GFP expression is localised to the injected left kidney cortex. In the 2 animals injected with a low dose of rAAV test product via the right artery, GFP signal is observed in podocytes in the injected kidney along with other cells within the glomerulus. In comparison, the IV injected group demonstrated no GFP protein expression in either kidney in 2 animals, with one animal showing some expression in the glomerulus, but much less than in the low dose group.

To further confirm kidney localisation, images from the high rAAV dose dRAi group were magnified further to show key areas within the glomerulus harbouring GFP expression (**Figure 9**). GFP signal shows localisation deep within the glomerulus where podocytes would reside.

Immunofluorescence analysis was performed on porcine liver after administration of intravenous injection (high dose) and renal artery injection (dRAi - high dose and low dose) rAAV test product expressing GFP protein under the CMV promoter. All porcine liver sections that were imaged following administration of high and low dose dRAi were negative for GFP (**Figure 10**). In contrast, GFP was observed in the porcine liver section following rAAV IV injection. Results demonstrate that GFP expression was only observed in liver of IV animals when compared to animals injected with rAAV via dRAi (both high dose and low dose).

### In-life observations/measurements

There were no deaths during the study period. There was no AAV-LK03-eGFP-related effect on clinical observations or monitoring in any of the treatment groups over the study period.

There was no AAV-LK03-eGFP-related effect on body weight in any of the treatment groups over the study period.

### Clinical pathology

All clinical pathology results obtained were within normal ranges expected for the same species of Göttingen minipigs. All controls were within range, the analysis was valid and correct results were obtained.

### Immunohistochemistry and Histology

The sections stained with Masson's Trichrome from the control animal and all 3 rAAV treatment groups (high dose IV, high dose dRAi and low dose dRAi) show a normal amount and distribution of collagen fibers in the renal corpuscle and between the renal tubules, with no aberrant accumulation of collagen or fibrin.

The section stained with PAS from the control animal and rAAV treated animals show normal glomeruli without any obvious GBM thickening or mesangial cell deposits. Bowman's space and proximal tubules are well-defined. All other conditions show normal histology, in both glomeruli and tubules. No rAAV treated animals developed focal segmental glomerulosclerosis and no glomeruli were affected by sclerosis.

### Example 3 - Direct renal artery injection of an AAV-LK03 vector encoding GFP under control of a hNPHS1 promoter

This study was designed to explore localisation of gene expression within the glomerulus of the kidney cortex using an rAAV vector in healthy pigs when administered via direct renal artery injection with (dRAi+O) or without (dRAi) renal artery occlusion, compared with an IV control.

### Materials and methods

A schematic representation of an AAV vector expressing GFP under the human full-length nephrin (hNPHS1) promoter is shown in **Figure 11A** (referred to herein as PS0528). The cassette was encapsulated into AAV-LK03 serotype for study use.

The rAAV was made via a triple plasmid transfection in HEK293T cells. The virus underwent 72-hour incubation post transfection. Viral particles were purified using density gradient separation. The virus was sterile filtered through a 0.2 micron filter. The rAAV vector was stored at -80°C until use.

Female Gottingen minipigs were acclimatised for 3 weeks prior to study start and were between 4.5 and 6.5 months old, and between 10 and 15kg in body weight at the time of dosing. Water was available ad libitum, while food was provided twice daily.

Female Gottingen pigs were dosed as shown below in Table 3, and the study design is also represented as a schematic in **Figure 11B****.**

**Table 3 Dosing Scheme and Treatment**

| **Group** | **N** | **Treatment** | **Dose (per pig)** | **Infusion vol** | **Occlusion Time** | **Infusion Time** |
|---|---|---|---|---|---|---|
| 1 | 3 | Saline (dRAi+O) | N/A | 15mL | 20-30 mins | 15 mins |
| 2 | 3 | PS0528 (dRAi+O) | 1x10¹³ vg | 15mL | 20-23 mins | 15 mins |
| 3 | 3 | PS0528 (dRAi) | 1x10¹² vg | 8mL | None | 4 mins |
| 4 | 3 | PS0528 (dRAi) | 5x10¹² vg | 8mL | None | 4 mins |
| 5 | 3 | PS0528 (dRAi) | 1x10¹³vg | 8mL | None | 4 mins |
| 6 | 3 | PS0528 (IV) | 1x10¹³vg | 8mL | None | 8 mins |

| | | | | | | |
|---|---|---|---|---|---|---|
| * Due to a dose administration error, one pig in group 2 was administered a double dose (2x10¹³ vg). This pig has been separated out in all analyses. | | | | | | |

A single kidney was dosed per pig. This was nominated as the left kidney, unless renal artery architecture presented a challenge based on angiographic assessment, in which case the right kidney was dosed. The interventional procedures of dRAi (renal artery delivery) and dRAi+O (renal artery delivery with occlusion) were carried out as detailed below, following the induction of general anaesthesia. Vector was diluted in saline (0.9% sodium chloride) to the volumes detailed above in Table 3, prior to administration.

### Local delivery (dRAi / dRAi+O):

- Catheterisation of the common femoral artery (CFA) per standard procedure
- Introduction of the short sheath in the CFA
- Introduction of the guidewire into the renal artery
- Introduction of the occlusion balloon catheter (for dRAi+O) or diagnostic catheter (for dRAi) into the renal artery
- For dRAi+O only: Balloon occlusion of the renal artery by inflating the balloon (aiming for <20 minutes total occlusion time)
- For dRAi+O only: Check for full occlusion with contrast injection and imaging
- Administration of heparinised saline flush
- Infusion of rAAV test product directly into the real artery using an infusion pump set to deliver at 1mL/minute for 15 minutes (dRAi+O) or 2mL/minute for 4 minutes (dRAi)
- Administration of heparinised saline flush
- For dRAi+O only: Deflation of the balloon catheter
- Withdrawal of the balloon (dRAi+O) or diagnostic (dRAi) catheter
- Withdrawal of the short sheath
- Application of pressure at access site until haemostasis is achieved

Animals were then recovered and observed daily until study termination. Pigs were culled 28-30 days post-dosing. At necropsy, sections of kidney (cortex and medulla) were resected and either fixed in formalin, frozen, or embedded in OCT blocks for the following assessments: histology, immunohistochemistry, immunofluorescence, RNAscope, ELISA, qPCR and RTqPCR.

### Results

### Biodistribution

Frozen kidney cortex from both treated and untreated kidney samples were thawed, DNA extracted and analysed by qPCR utilising primers and probes targeting WPRE.

**Figure 12A-B** shows AAV genomes detected per milligram (mg) of tissue. In the treated kidney (**Figure 12A**), comparing local delivery to IV at the 1x10¹³ dose level, the number of detected AAV genomes was >7-fold higher with dRAi and >23-fold higher with dRAi+O. The number of genomes detected in the untreated kidney increased with the administered dose **(****Figure 12B****).**

This data demonstrates that local delivery increases the retention of vector within the dosed kidney.

### RNAscope in-situ hybridisation (ISH)

Paraffin embedded formalin fixed tissues were assessed by RNAscope in-situ hybridisation for the localised presence of AAV mRNA in kidney cortex samples using a set of probes specific for WPRE. Stained sections were scanned and the images analysed by eye to determine the proportion of glomeruli with detectable WPRE mRNA. The number of mRNA positive regions were quantified and plotted as a frequency distribution.

**Figure 13A-D** shows the number of mRNA positive regions present in the glomerulus of the treated kidney from pigs dosed via either dRAi (direct renal artery injection without occlusion) or IV. Between 70 and 120 glomeruli were quantified per pig. dRAi delivery of both 5x10¹² vg and 1x10¹³ vg resulted in a significant increase in the number of mRNA regions per glomerulus as compared to 1x10¹³ vg delivered via IV administration.

This data demonstrates that local delivery of AAV to the kidney significantly increases glomerular transduction and gene expression.

**Figure 14A-C** shows the number of mRNA positive regions present in the glomerulus of the treated kidney from pigs dosed via either dRAi+O (direct renal artery injection with occlusion) or IV. Between 70 and 120 glomeruli were quantified per pig. dRAi+O delivery of both 1x10¹³ vg and 2x10¹³ vg resulted in a significant increase in the number of mRNA regions per glomerulus as compared to 1x10¹³ vg delivered via IV administration.

Collectively, **Figures 13** **and** **14** show that local delivery to the kidney, with or without disruption of blood flow, leads to significantly improved glomerular transduction and gene expression compared to systemic dosing. This result is consistent at multiple different delivery volumes, infusion rates and AAV vector concentrations.

### Example 4 - Direct renal artery injection of an AAV-LK03 vector encoding podocin under control of a hNPHS1 promoter

This study was designed to explore localisation of gene expression within the glomerulus of the kidney cortex, and selective transduction of podocytes within the glomerulus using an rAAV vector in healthy pigs when administered via direct renal artery injection with renal artery occlusion (dRAi+O).

### Materials and methods

A schematic representation of an AAV vector expressing human HA-tagged podocin under the human full-length nephrin (hNPHS1) promoter is shown in **Figure 15A** (referred to herein as PS0438). The cassette was encapsulated into AAV-LK03 serotype for study use.

The rAAV was made via a triple plasmid transfection in HEK293T cells. The virus underwent 72-hour incubation post transfection. Viral particles were purified using density gradient separation. The virus was sterile filtered through a 0.2 micron filter. The rAAV vector was stored at -80°C until use.

Female Gottingen minipigs were acclimatised for 2 weeks prior to study start and were between 23 and 25 weeks old, and between 11 and 15kg in body weight at the time of dosing. Water was available ad libitum, while food was provided twice daily.

Female Gottingen pigs were dosed as shown below in Table 4, and the study design is also represented as a schematic in **Figure 15B****.**

**Table 4 Dosing Scheme and Treatment**

| **Group** | **N** | **Treatment** | **Dose (per pig)** | **Infusion vol** | **Occlusion Time** | **Infusion Time** |
|---|---|---|---|---|---|---|
| 1 | 3 | None | N/A | N/A | N/A | N/A |
| 2 | 3 | PS0438 (dRAi+O) | 1x10¹³vg | 15mL | 20 mins | 15 mins |

In this study a single kidney was dosed per pig. This was nominated as the left kidney unless renal artery architecture presented a challenge, based on angiographic assessment, in which case the right kidney was dosed. The interventional procedure of dRAi+O was carried out as per the process below, following the induction of general anaesthesia. Vector was diluted in saline (0.9% sodium chloride) to the volumes detailed in Table 4 above, prior to administration.

### Local delivery (dRAi+O):

- Catheterisation of the common femoral artery (CFA) per standard procedure
- Introduction of the short sheath in the CFA
- Introduction of the guidewire into the renal artery
- Introduction of the occlusion balloon catheter into the renal artery
- Balloon occlusion of the renal artery by inflating the balloon (aiming for <20 minutes total occlusion time)
- Check for full occlusion with contrast injection and imaging
- Administration of heparinised saline flush
- Infusion of rAAV test product directly into the real artery using an infusion pump set to deliver at 1 mL/minute for 15 minutes
- Administration of heparinised saline flush
- Deflation of the balloon catheter
- Withdrawal of the balloon catheter
- Withdrawal of the short sheath
- Application of pressure at access site until haemostasis is achieved

Animals were then recovered and observed daily until study termination. Pigs were culled 27 days post-dosing. At necropsy, sections of kidney (cortex and medulla), liver and spleen were resected and either fixed in formalin, frozen, or embedded in OCT blocks for the following assessments: histology, immunohistochemistry, immunofluorescence, RNAscope, ELISA, qPCR and RTqPCR.

### Results

### Biodistribution

Frozen kidney cortex from both treated and untreated kidneys, spleen, and liver samples were thawed, DNA extracted and analysed by qPCR utilising primers and probes targeting WPRE.

**Figure 16A-D** shows AAV genomes detected per milligram (mg) of tissue. The number of genomes detected in the treated kidney of pigs dosed with PS0438 was 50 to 150-fold higher than untreated kidney, 75 to 430-fold higher than liver and >580 fold higher than spleen per mg of tissue.

This data demonstrates that local delivery to the kidney increases the retention of vector in the dosed kidney.

### RNAscope in-situ hybridisation (ISH)

Paraffin embedded formalin fixed tissues were assessed by RNAscope in-situ hybridisation for the localised presence of AAV mRNA in kidney cortex samples using a set of probes specific for WPRE. Stained sections were scanned and the images analysed by eye to determine the proportion of glomeruli with detectable WPRE mRNA. The number of mRNA positive regions were quantified and plotted as a frequency distribution.

**Figure 17A-B** shows the number of mRNA positive regions present in the glomerulus of the treated and untreated kidney from pigs dosed with PS0438 via dRAi+O. Between 77 and 120 glomeruli were quantified per pig. dRAi+O delivery of 1x10¹³ vg resulted in significantly greater expression of mRNA in the treated kidney than in the untreated kidney, localised within the glomerulus.

This data demonstrates that local delivery significantly increases glomerular transduction and gene expression in the treated kidney.

### Immunofluorescence

Immunofluorescent (IF) analysis was performed on sections obtained from OCT blocks of kidney cortex. Sections were cut from blocks obtained from the locally dosed kidney of pigs dosed with PS0438. To assess the localisation of expression of the HA-tagged podocin therapeutic gene encoded by the AAV vector, multiple markers were utilised. Sections were stained with DAPI and antibodies targeting WT-1, a podocyte-specific transcription factor, HA - the protein tag present on the AAV-encoded podocin protein and nephrin, a podocyte specific slit-diaphragm protein which localises with podocin in podocytes.

**Figure 18A** shows composite IF images of two glomeruli alongside single channel images showing HA-tagged podocin (**Figures 18B**) and nephrin (**Figure 18C**). **Figure 18D** shows composite IF images of one glomerulus alongside single channel images showing HA-tagged podocin (**Figures 18E**) and nephrin (**Figure 18F**). Co-localisation of podocin and nephrin is observed and, alongside the podocyte-specific WT-1 nuclear stain, demonstrates podocyte-specific expression of the podocin therapeutic gene.

Overall, this data demonstrates that local delivery of AAV to the kidney results in successful transduction of podocytes and the production of a therapeutic gene.

### Podocin ELISA

Cryopreserved sections of kidney cortex from the dosed kidney of pigs treated with PS0438 were thawed, homogenised and the lysate analysed by ELISA using a commercially available kit to quantify podocin protein. Total protein was also quantified by BCA assay.

**Figure 19** shows podocin protein detected in kidney cortex samples by ELISA in nanograms per milligram of total protein. Untreated control pigs (UTC) demonstrated endogenous levels of podocin, while between 1.7 and 3.8-fold higher levels of podocin protein were detectable in the cortex of the locally dosed kidney from pigs dosed with PS0438.

Collectively, **Figures 18 and 19** show that local delivery to the kidney with a podocin-expressing AAV (PS0438) results in podocyte-specific expression of a therapeutic transgene at greater levels than are expressed endogenously.

### Example 5 - Direct renal artery injection of an AAV9 vector encoding GFP under control of a CMV promoter

3 female nude mice were injected with AAV9-CMV-GFP via either IV injection at a dose of 1.5x10¹² vg or by direct injection into the renal artery (dRAi) at a dose of 7.5x10¹¹ vg, or with PBS as a negative control.

GFP expression in the kidney cortex was determined using immunofluorescence and the results are shown in **Figure 20****.** Direct renal artery injection (dRAi) results in higher transduction of glomerular cells than IV administration (**Figure 20A**), even though a larger dose was administered via IV, confirming what was observed in the previous pig studies. GFP co-localised with NPHS1 and PDGFb, which confirms localisation in podocytes and mesangial cells, respectively (**Figure 20B**).

GFP expression in the liver was determined by Western blot analysis and densitometry and the results are shown in **Figure 21A-B.** Quantification of band density from the Western blot (**Figure 21A**) demonstrates higher expression of GFP was observed in the liver with IV administration of the AAV vector in comparison to administration of the AAV vector by direct renal artery injection (dRAi) (**Figure 21B**).

This data shows that local delivery to the kidney with an AAV9 vector results in kidney-specific expression in podocytes and mesangial cells.

## Claims

1. A viral vector for use in a method of treating a human subject that has or is at risk of a kidney disease, the method comprising:
(a) inserting a catheter into the renal artery of the kidney;
(b) optionally inflating a balloon to occlude the renal artery; and
(c) injecting or infusing the viral vector into the renal artery via the catheter,
wherein the method is a minimally invasive procedure, wherein the method does not comprise a step of clamping the renal artery of the kidney, the renal vein of the kidney, or the aorta, and wherein the method does not comprise a step of inserting a catheter into the renal vein of the kidney.

2. The viral vector for use according to claim 1, wherein the use does not comprise a step of occluding the renal vein of the kidney.

3. The viral vector for use according to any preceding claim, wherein the use does not comprise a step of inserting a catheter directly into the aorta.

4. The viral vector for use according to any preceding claim, wherein the use does not comprise forming a closed circuit through the kidney.

5. The viral vector for use according to any preceding claim, wherein the catheter is an occlusion balloon catheter, and the method further comprises inflating the balloon to occlude the renal artery after inserting the catheter into the renal artery and before the injecting or infusing of the viral vector.

6. The viral vector for use according to any preceding claim, wherein the renal artery is occluded for from 1 minute to 25 minutes; from 2 minutes to 10 minutes, optionally 5 minutes; from 15 minutes to 25 minutes; or from 15 minutes to 20 minutes, optionally 20 minutes.

7. The viral vector for use according to claim1, wherein the use does not comprise a step of occluding the renal artery of the kidney.

8. The viral vector for use according to any preceding claim, wherein the catheter is inserted into the renal artery via a percutaneous route, optionally wherein the percutaneous route is via the carotid artery or via the femoral artery and/or wherein insertion of the catheter via the percutaneous route is facilitated with a sheath.

9. The viral vector for use according to any preceding claim, wherein the viral vector is infused into the renal artery under no flow conditions using an infusion pump.

10. The viral vector for use according to any preceding claim, wherein the viral vector is injected or infused into the renal artery over 1 minute to 30 minutes, preferably (a) wherein the viral vector is injected or infused into the renal artery over 1 minute to 5 minutes, optionally 2 minutes or 4 minutes; or (b) wherein the viral vector is injected or infused into the renal artery over 5 minutes to 30 minutes or 15 minutes to 20 minutes, optionally 17 minutes.

11. The viral vector for use according to any preceding claim, wherein the subject has or is at risk of a glomerular disease, optionally wherein the subject has or is at risk of a podocyte-associated glomerular disease.

12. The viral vector for use according to any preceding claim, wherein the viral vector is delivered in a dose of from 1x10⁸ vg to 1x10¹⁵ vg, or from 1x10⁸ vg to 5x10¹⁴ vg.

13. The viral vector for use according to any preceding claim, wherein the viral vector is capable of transducing kidney cells, optionally wherein the vector is capable of specifically transducing kidney cells; or wherein the viral vector is capable of transducing glomerular cells, optionally wherein the vector is capable of specifically transducing glomerular cells.

14. The viral vector for use according to any preceding claim, wherein the viral vector is capable of transducing podocytes, optionally wherein the vector is capable of specifically transducing glomerular podocytes.

15. The viral vector for use according to any preceding claim, wherein the viral vector is selected from an adeno-associated virus (AAV) vector, a lentiviral vector, a retroviral vector, an adenoviral vector, a herpes simplex viral vector, an alphaviral vector, a flaviviral vector, a rhabdoviral vector, a measles viral vector, a Newcastle disease viral vector, a poxviral vector, and a picornaviral vector.

16. The viral vector for use according to any preceding claim, wherein the viral vector is an adeno-associated virus (AAV) vector particle.

17. The viral vector for use according to any preceding claim, wherein the viral vector comprises a protein-coding sequence, optionally wherein the protein-coding sequence is operably linked to a kidney-specific promoter, preferably a podocyte-specific promoter.

18. The viral vector for use according to any preceding claim, wherein the viral vector is injected or infused into the renal artery in the form of a viral vector formulation, optionally wherein the viral vector formulation comprises the viral vector in an amount of from 1x10⁷ vg/ml to 1x10¹⁴ vg/ml, or from 1x10⁷ vg/ml to 5x10¹³ vg/ml.

## Patentansprüche

1. Viraler Vektor zur Verwendung in einem Verfahren zum Behandeln eines menschlichen Subjekts, das eine Nierenerkrankung aufweist oder bei dem ein Risiko für eine Nierenerkrankung besteht, wobei das Verfahren Folgendes umfasst:
(a) Einführen eines Katheters in die Nierenarterie der Niere;
(b) optional Aufblasen eines Ballons, um die Nierenarterie zu verschließen; und
(c) Injizieren oder Infundieren des viralen Vektors in die Nierenarterie über den Katheter,
wobei das Verfahren ein minimalinvasiver Eingriff ist, wobei das Verfahren keinen Schritt eines Abklemmens der Nierenarterie der Niere, der Nierenvene der Niere oder der Aorta umfasst und wobei das Verfahren keinen Schritt eines Einführens eines Katheters in die Nierenvene der Niere umfasst.

2. Viraler Vektor zur Verwendung nach Anspruch 1, wobei die Verwendung keinen Schritt eines Verschließens der Nierenvene der Niere umfasst.

3. Viraler Vektor zur Verwendung nach einem vorhergehenden Anspruch, wobei die Verwendung keinen Schritt eines Einführens eines Katheters direkt in die Aorta umfasst.

4. Viraler Vektor zur Verwendung nach einem vorhergehenden Anspruch, wobei die Verwendung kein Ausbilden eines geschlossenen Kreislaufs durch die Niere umfasst.

5. Viraler Vektor zur Verwendung nach einem vorhergehenden Anspruch, wobei der Katheter ein Okklusionsballonkatheter ist und das Verfahren ferner Aufblasen des Ballons umfasst, um die Nierenarterie nach Einführen des Katheters in die Nierenarterie und vor dem Injizieren oder Infundieren des viralen Vektors zu verschließen.

6. Viraler Vektor zur Verwendung nach einem vorhergehenden Anspruch, wobei die Nierenarterie für 1 Minute bis 25 Minuten; 2 Minuten bis 10 Minuten, optional 5 Minuten; 15 Minuten bis 25 Minuten; oder 15 Minuten bis 20 Minuten, optional 20 Minuten, verschlossen wird.

7. Viraler Vektor zur Verwendung nach Anspruch 1, wobei die Verwendung keinen Schritt eines Verschließens der Nierenarterie der Niere umfasst.

8. Viraler Vektor zur Verwendung nach einem vorhergehenden Anspruch, wobei der Katheter über einen perkutanen Weg in die Nierenarterie eingeführt wird, wobei der perkutane Weg optional über die Karotisarterie oder über die Femoralarterie verläuft und/oder wobei die Einführung des Katheters über den perkutanen Weg mit einer Schleuse erleichtert wird.

9. Viraler Vektor zur Verwendung nach einem vorhergehenden Anspruch, wobei der virale Vektor bei Bedingungen ohne Strömung unter Verwendung einer Infusionspumpe in die Nierenarterie infundiert wird.

10. Viraler Vektor zur Verwendung nach einem vorhergehenden Anspruch, wobei der virale Vektor über 1 Minute bis 30 Minuten in die Nierenarterie injiziert oder infundiert wird, bevorzugt (a) wobei der virale Vektor über 1 Minute bis 5 Minuten, optional 2 Minuten oder 4 Minuten, in die Nierenarterie injiziert oder infundiert wird; oder (b) wobei der virale Vektor über 5 Minuten bis 30 Minuten oder 15 Minuten bis 20 Minuten, optional 17 Minuten, in die Nierenarterie injiziert oder infundiert wird.

11. Viraler Vektor zur Verwendung nach einem vorhergehenden Anspruch, wobei das Subjekt eine glomeruläre Erkrankung aufweist oder ein Risiko für eine glomeruläre Erkrankung bei dem Subjekt besteht, wobei das Subjekt optional eine Podozyten-assoziierte glomeruläre Erkrankung aufweist oder ein Risiko für eine Podozyten-assoziierte glomeruläre Erkrankung bei dem Subjekt besteht.

12. Viraler Vektor zur Verwendung nach einem vorhergehenden Anspruch, wobei der virale Vektor in einer Dosis von 1x10⁸ vg bis 1x10¹⁵ vg oder von 1x10⁸ vg bis 5x10¹⁴ vg verabreicht wird.

13. Viraler Vektor zur Verwendung nach einem vorhergehenden Anspruch, wobei der virale Vektor in der Lage ist, Nierenzellen zu transduzieren, wobei optional der Vektor in der Lage ist, Nierenzellen spezifisch zu transduzieren; oder wobei der virale Vektor in der Lage ist, glomeruläre Zellen zu transduzieren, wobei optional der Vektor in der Lage ist, glomeruläre Zellen spezifisch zu transduzieren.

14. Viraler Vektor zur Verwendung nach einem vorhergehenden Anspruch, wobei der virale Vektor in der Lage ist, Podozyten zu transduzieren, wobei optional der Vektor in der Lage ist, glomeruläre Podozyten spezifisch zu transduzieren.

15. Viraler Vektor zur Verwendung nach einem vorhergehenden Anspruch, wobei der virale Vektor ausgewählt ist aus einem Adeno-assoziierten-Virus (AAV)-Vektor, einem lentiviralen Vektor, einem retroviralen Vektor, einem adenoviralen Vektor, einem Herpes-Simplex-Virus-Vektor, einem alphaviralen Vektor, einem flaviviralen Vektor, einem rhabdoviralen Vektor, einem Masern-Virus-Vektor, einem Newcastle-Disease-Virus-Vektor, einem Poxvirus-Vektor und einem Picornavirus-Vektor.

16. Viraler Vektor zur Verwendung nach einem vorhergehenden Anspruch, wobei der virale Vektor ein Adeno-assoziiertes-Virus(AAV)-Vektorpartikel ist.

17. Viraler Vektor zur Verwendung nach einem vorhergehenden Anspruch, wobei der virale Vektor eine Protein-kodierende Sequenz umfasst, wobei optional die Protein-kodierende Sequenz funktionsfähig mit einem nierenspezifischen Promotor, bevorzugt einem podozytenspezifischen Promotor, verknüpft ist.

18. Viraler Vektor zur Verwendung nach einem vorhergehenden Anspruch, wobei der virale Vektor in Form einer viralen Vektorformulierung in die Nierenarterie injiziert oder infundiert wird, wobei optional die virale Vektorformulierung den viralen Vektor in einer Menge von 1x10⁷ vg/ml bis 1x10¹⁴ vg/ml oder von 1x10⁷ vg/ml bis 5x10¹³ vg/ml umfasst.

## Revendications

1. Vecteur viral destiné à être utilisé dans un procédé de traitement d'un sujet humain qui présente ou est susceptible de présenter une maladie rénale, le procédé comprenant :
(a) l'insertion d'un cathéter dans l'artère rénale du rein ;
(b) éventuellement l'inflation d'un ballon pour occlure l'artère rénale ; et
(c) l'injection ou la perfusion du vecteur viral dans l'artère rénale par l'intermédiaire du cathéter,
ledit procédé étant une procédure peu invasive, ledit procédé ne comprenant pas d'étape de clampage de l'artère rénale du rein, de la veine rénale du rein ou de l'aorte, et ledit procédé ne comprenant pas d'étape d'insertion d'un cathéter dans la veine rénale du rein.

2. Vecteur viral destiné à être utilisé selon la revendication 1, ladite utilisation ne comprenant pas d'étape d'occlusion de la veine rénale du rein.

3. Vecteur viral destiné à être utilisé selon une quelconque revendication précédente, ladite utilisation ne comprenant pas d'étape d'insertion d'un cathéter directement dans l'aorte.

4. Vecteur viral destiné à être utilisé selon une quelconque revendication précédente, ladite utilisation ne comprenant pas la formation d'un circuit fermé à travers le rein.

5. Vecteur viral destiné à être utilisé selon une quelconque revendication précédente, ledit cathéter étant un cathéter à ballon d'occlusion, et le procédé comprenant en outre l'inflation du ballon pour occlure l'artère rénale après l'insertion du cathéter dans l'artère rénale et avant l'injection ou la perfusion du vecteur viral.

6. Vecteur viral destiné à être utilisé selon une quelconque revendication précédente, ladite artère rénale étant occluse pendant 1 minute à 25 minutes ; 2 minutes à 10 minutes, éventuellement 5 minutes ; 15 minutes à 25 minutes ; ou 15 minutes à 20 minutes, éventuellement 20 minutes.

7. Vecteur viral destiné à être utilisé selon la revendication 1, ladite utilisation ne comprenant pas d'étape d'occlusion de l'artère rénale du rein.

8. Vecteur viral destiné à être utilisé selon une quelconque revendication précédente, ledit cathéter étant inséré dans l'artère rénale par voie percutanée, éventuellement ladite voie percutanée passant par l'artère carotide ou par l'artère fémorale et/ou ladite insertion du cathéter par la voie percutanée étant facilitée par une gaine.

9. Vecteur viral destiné à être utilisé selon une quelconque revendication précédente, ledit vecteur viral étant perfusé dans l'artère rénale dans des conditions d'absence de flux à l'aide d'une pompe à perfusion.

10. Vecteur viral destiné à être utilisé selon une quelconque revendication précédente, ledit vecteur viral étant injecté ou perfusé dans l'artère rénale pendant 1 minute à 30 minutes, de préférence (a) ledit vecteur viral étant injecté ou perfusé dans l'artère rénale pendant 1 minute à 5 minutes, éventuellement 2 minutes ou 4 minutes ; ou (b) ledit vecteur viral étant injecté ou perfusé dans l'artère rénale pendant 5 minutes à 30 minutes ou 15 minutes à 20 minutes, éventuellement 17 minutes.

11. Vecteur viral destiné à être utilisé selon une quelconque revendication précédente, ledit sujet présentant ou étant susceptible de présenter une maladie glomérulaire, éventuellement ledit sujet présentant ou étant susceptible de présenter une maladie glomérulaire associée aux podocytes.

12. Vecteur viral destiné à être utilisé selon une quelconque revendication précédente, ledit vecteur viral étant administré à une dose de 1x10⁸ vg à 1x10¹⁵ vg, ou de 1x10⁸ vg à 5x10¹⁴ vg.

13. Vecteur viral destiné à être utilisé selon une quelconque revendication précédente, ledit vecteur viral étant capable de transduire des cellules rénales, éventuellement ledit vecteur étant capable de transduire spécifiquement des cellules rénales ; ou ledit vecteur viral étant capable de transduire des cellules glomérulaires, éventuellement ledit vecteur étant capable de transduire spécifiquement des cellules glomérulaires.

14. Vecteur viral destiné à être utilisé selon une quelconque revendication précédente, ledit vecteur viral étant capable de transduire des podocytes, éventuellement ledit vecteur étant capable de transduire spécifiquement des podocytes glomérulaires.

15. Vecteur viral destiné à être utilisé selon une quelconque revendication précédente, ledit vecteur viral étant choisi parmi un vecteur de virus adéno-associé (AAV), un vecteur lentiviral, un vecteur rétroviral, un vecteur adénoviral, un vecteur viral de l'herpès simplex, un vecteur alphaviral, un vecteur flaviviral, un vecteur rhabdoviral, un vecteur viral de la rougeole, un vecteur viral de la maladie de Newcastle, un vecteur poxviral et un vecteur picornaviral.

16. Vecteur viral destiné à être utilisé selon une quelconque revendication précédente, ledit vecteur viral étant une particule de vecteur de virus adéno-associé (AAV).

17. Vecteur viral destiné à être utilisé selon une quelconque revendication précédente, ledit vecteur viral comprenant une séquence codant pour une protéine, éventuellement ladite séquence codant pour une protéine étant liée de manière opérationnelle à un promoteur spécifique du rein, de préférence un promoteur spécifique des podocytes.

18. Vecteur viral destiné à être utilisé selon une quelconque revendication précédente, ledit vecteur viral étant injecté ou perfusé dans l'artère rénale sous la forme d'une formulation de vecteur viral, éventuellement ladite formulation de vecteur viral comprenant le vecteur viral en une quantité de 1x10⁷ vg/ml à 1x10¹⁴ vg/ml, ou de 1x10⁷ vg/ml à 5x10¹³ vg/ml.
